# FASCICULE DE BREVET EUROPEEN

(11) **EP 2 499 150 B1**
(45) Date de publication et mention de la délivrance du brevet: **12.03.2014**
(21) Numéro de dépôt: 10787512.2
(22) Date de dépôt: 10.11.2010
(51) Int. Cl.: C07H 15/26, A61K 31/7028

(54) **NOUVEAUX DERIVES DE MANNOPYRANOSIDE AYANT UNE ACTIVITE ANTICANCEREUSE**
NEUE MANNOPYRANOSIDDERIVATE MIT ANTIKREBSWIRKUNG
NOVEL MANNOPYRANOSIDE DERIVATIVES WITH ANTICANCER ACTIVITY

(30) Priorité: 10.11.2009 FR 0905394
(43) Date de publication de la demande: 19.09.2012
(73) Titulaire: Centre National de la Recherche Scientifique, 75016 Paris (FR)
(72) Inventeur: MONTERO, Jean-Louis, F-34270 Lauret (FR); MONTERO, Véronique, F-34270 Lauret (FR); MOLES, Jean-Pierre, F-34660 Cournonsec (FR); DE SANTA BARBARA, Pascal, F-34690 Fabrègues (FR); COMBEMALE, Stéphanie, 30000 Nimes (FR); AWWAD, Azzam, F-34096 Montpellier (FR); JOVER, Bernard, F-34080 Montpellier (FR)
(74) Mandataire: Mena, Sandra
(86) Numéro de dépôt international: PCT/FR2010/000749
(87) Numéro de publication internationale: WO 2011/058245

(56) Documents cités:
- EP-A1- 1 757 296
- US-A1- 2005 130 240
- JEANJEAN A ET AL: "Synthesis of new sulfonate and phosphonate derivatives for cation-independent mannose 6-phosphate receptor targeting", BIOORGANIC & MEDICINAL CHEMISTRY LETTERS, PERGAMON, ELSEVIER SCIENCE, GB, vol. 18, no. 23, 1 décembre 2008 (2008-12-01), pages 6240-6243, XP025646316, ISSN: 0960-894X, DOI: DOI:10.1016/J.BMCL.2008.09.101 [extrait le 2008-10-02]

## Description

La présente invention est relative à des composés dérivés de mannopyranoside, à un procédé de synthèse par chimie verte de tels composés, et à leur utilisation à titre de médicaments, notamment pour le traitement de maladies cancéreuses, ainsi qu'à des compositions pharmaceutiques comprenant de tels composés. Font également partis de l'invention des dispositifs médicaux implantables traités en surface par des composés dérivés de mannopyranoside selon l'invention.

De nombreuses pathologies ont été décrites dans l'état de l'art comme ayant une composante ou un stade lié au phénomène d'angiogénèse. On peut citer entre autres de très nombreux cancers, les rétinopathies liées au diabète, l'athérosclérose, l'arthrose, la polyarthrite rhumatoïde, le psoriasis, ainsi que les pathologies inflammatoires ou celles liées à une cicatrisation retardée.

L'angiogenèse est un mécanisme de néovascularisation prenant naissance à partir d'un réseau capillaire préexistant. Le bourgeonnement de petits vaisseaux, les capillaires, à partir de ceux préexistants, intervient pour le meilleur lors du développement embryonnaire et l'implantation du placenta, lorsqu'il s'agit de cicatriser une blessure, ou de pallier l'obstruction d'un vaisseau ; mais également pour le pire dans les cancers (croissance des tumeurs et développement des métastases), l'arthrite rhumatoïde, certaines maladies ophtalmologiques comme la rétinopathie diabétique ou la dégénérescence maculaire liée à l'âge. Pour l'ensemble de ces processus, le schéma général reste le même. L'activation des cellules endothéliales conduit à la dégradation de la membrane basale et de la matrice extracellulaire environnante. La migration orientée est suivie d'une phase proliférative. Les cellules se différencient ensuite en une structure de type capillaire pour former un réseau vasculaire nécessaire au développement des tissus. Toutefois, l'angiogenèse n'est pas contrôlée par un seul facteur, mais par une balance d'inducteurs et d'inhibiteurs produits par les cellules normales ou tumorales. Parmi ces facteurs, des polypeptides comme le facteur de croissance des fibroblastes-2 ("*Fibroblast Growth Factor-2*" : FGF-2) et le "facteur de croissance de l'endothélium vasculaire ("*Vascular Endothelial Growth Factor*" : VEGF) sont apparus comme étant des régulateurs clés de l'angiogenèse.

De nombreuses molécules ont été étudiées pour leur effet inhibiteur ou activateur de l'angiogénèse.

En ce qui concerne l'inhibition de l'angiogénèse, une révolution conceptuelle récente dans le traitement du cancer consiste à cibler le réseau vasculaire qui irrigue une tumeur. Il est maintenant bien établi que le développement d'une vascularisation intra ou péritumorale est un événement clé autant pour la croissance d'une tumeur que pour la dissémination métastatique par la voie sanguine. En décembre 2005, la revue scientifique anglaise Nature*,* qui consacrait son numéro à l'angiogénèse, dénombrait plus de 300 inhibiteurs, dont 80 en cours d'essais cliniques. Mais les premiers médicaments testés - angiostatine, endostatine, interférons, inhibiteurs de matrice métalloprotéinases, etc. - furent décevants. Parmi des molécules plus récentes, on peut citer le bevacizumab. Injecté au patient, il neutralise un type de VEGF circulant dans les capillaires ou diffus dans la tumeur, le VEGF-A. Sa première indication fut en 2004 pour le cancer colorectal métastatique, en association avec une chimiothérapie. Il est aujourd'hui en essais cliniques contre les cancers du rein métastatiques, du poumon et du sein. Le VEGF-A présente néanmoins l'inconvénient d'accroître le risque d'hypertension et d'hémorragie. On peut également citer le sunitinib et le sorafenib qui présentent l'avantage de pouvoir autoriser une formulation sous forme de comprimés à absorber par voie orale et qui conduisent à des résultats thérapeutiques encourageants. Ils présentent également l'inconvénient d'engendrer quelques effets secondaires comme l'hypertension, la fatigue ou des problèmes de peau.

De plus, les molécules actuellement utilisées pour leurs propriétés inhibitrices de l'angiogénèse présentent des toxicités élevées souvent rédhibitoires pour la poursuite des traitements, cette toxicité limitant la durée et l'efficacité des médications actuelles.

Il existe donc un besoin constant et extrêmement important en composés inhibiteurs de l'angiogénèse ayant une très faible toxicité et une meilleure affinité avec les récepteurs, dans le but de proposer de nouveaux traitements anticancéreux présentant une excellente activité anti-tumorale.

C'est donc afin de remédier à l'ensemble de ces inconvénients et de pourvoir à des composés ayant une activité d'inhibition de l'angiogénèse de très faible toxicité alliée à une excellente activité, ces composés pouvant notamment être utilisés pour la préparation de médicaments anticancéreux, que les inventeurs ont mis au point ce qui fait l'objet de la présente invention.

Les inventeurs ont en effet découvert que certains dérivés de mannopyranoside présentent une excellente activité anticancéreuse et une très faible toxicité, ces composés pouvant être utilisés pour la préparation de compositions pharmaceutiques destinées au traitement de pathologies cancéreuses.

La présente invention a donc pour objet un composé dérivé de mannopyranoside ou ses sels pharmaceutiquement acceptables, répondant à l'une des formules suivantes :
➢ Formule (I) : 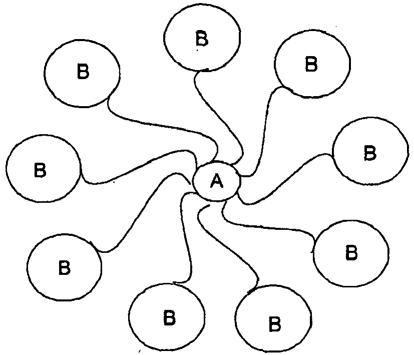 dans laquelle :
   ■ A est une nanoparticule de silice ou une nanoparticule métallique choisie parmi les éléments des colonnes (IB), (IIB), (IIIB), (IVB), (VB), (VIB), (VIIB) ou (VIIIB) de la classification de Mendeleïev, et
   ■ B est un groupement porteur d'une fonction mannopyranoside, également appelé « tête polaire », répondant à la structure suivante : 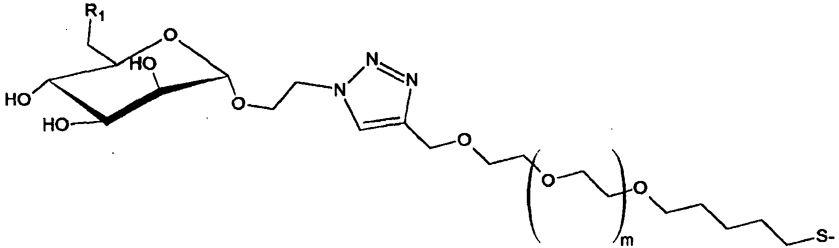 dans lequel :
      - le radical R₁ représente un radical sélectionné parmi -O-PO₃H₂, -N₃, -CH₂-PO₃H₂,-CH₂-COOH, -SO₃H, -OPHO₂H, -CH₂-B(OH)₂, -X-PHO₂H, X'-PO₂H-X-PO₃H₂, et de préférence -CH₂-COOH et -N₃, et
      - m est un entier compris entre 0 et 10, et de préférence m = 3, 4, 5 ou 6,
   les groupements B étant liés à la nanoparticule A via l'atome de soufre, et le nombre de groupements B liés à la nanoparticule A étant compris entre 100 et 1000, et de préférence entre 400 et 600,
➢ Formule (II) : 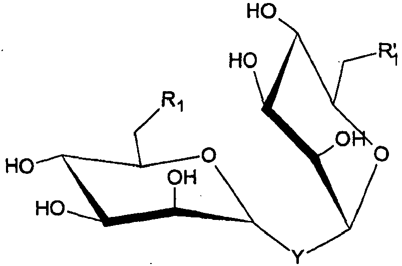 dans laquelle :
   - les radicaux R₁ et R₁', identiques ou différents, représentent des radicaux sélectionnés parmi -O-PO₃H₂, -N₃, -CH₂-PO₃H₂, -CH₂-COOH, -SO₃H, -OPHO₂H, -CH₂-B(OH)₂, -X-PHO₂H, X'-PO₂H-X-PO₃H₂, et de préférence -CH₂-COOH et -N₃, et
   - Y représente l'un des groupements suivants : 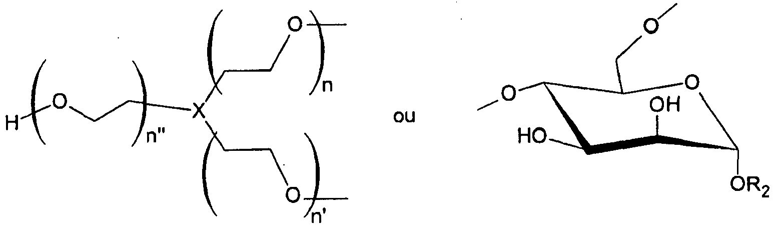 avec :
      ○ n, n' et n" étant des entiers compris entre 1 et 12, et de préférence entre 1 et 6, et
      ○ n" étant égal à 0 lorsque X représente un atome d'oxygène, le groupement X étant choisi parmi : N, O, S, une chaîne alkyle en C₁-C₄, le groupement X étant de préférence un atome d'oxygène, et
      ○ le radical R₂ représente une chaîne alkyle linéaire ou ramifiée en C₁-C₁₂, et de préférence en C₁-C₄ ; une chaîne alkyle linéaire ou ramifiée en C₁-C₁₂, et de préférence en C₁-C₄, porteur d'au moins un groupement -OH, -NH₂, -SH, -COOH, -N₃, -NO₂ ; un cycle hydrocarboné, saturé ou insaturé, en C₃-C₆ ; un cycle hydrocarboné en C₃-C₁₀, saturé ou insaturé, porteur d'au moins un groupement -OH, -NH₂, -SH, -COOH, -N₃, -NO₂, alkyle en C₁-C₄ ; un hétérocycle saturé ou insaturé comportant au moins un hétéroatome choisi parmi les atomes d'oxygène, d'azote ou de soufre ; un radical -(CH₂-CH₂-O)_{y}-H, dans lequel y est compris entre 1 et 12, et de préférence entre 1 et 6,
➢ Formule (III) : 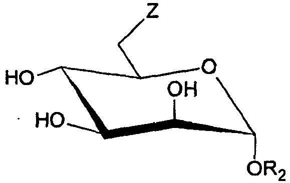 dans laquelle Z représente l'un des groupements suivants : 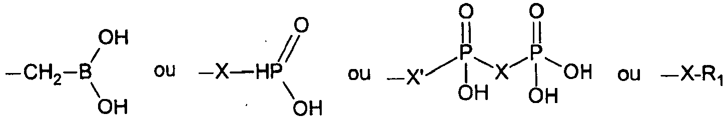 dans lesquelles :
   - le radical R₁ est sélectionné parmi -O-PO₃H₂, -N₃, -CH₂-PO₃H₂, -CH₂-COOH, -OPHO₂H, - CH₂-B(OH)₂, -X-PHO₂H, -X'-PO₂H-X-PO₃H₂, et de préférence -CH₂-COOH et -N₃,
   - le radical R₂ représente une chaîne alkyle linéaire ou ramifiée en C₁-C₁₂, et de préférence en C₁-C₄ ; une chaîne alkyle linéaire ou ramifiée en C₁-C₁₂, et de préférence en C₁-C₄, porteur d'au moins un groupement -OH, -NH₂, -SH, -COOH, -N₃, -NO₂ ; un cycle hydrocarboné, saturé ou insaturé, en C₃-C₆ ; un cycle hydrocarboné en C₃-C₁₀, saturé ou insaturé, porteur d'au moins un groupement -OH, -NH₂, -SH, -COOH, -N₃, -NO₂, alkyle en C₁-C₄ ; un hétérocycle saturé ou insaturé comportant au moins un hétéroatome choisi parmi les atomes d'oxygène, d'azote ou de soufre ; un radical -(CH₂-CH2-O)_{y}-H, dans lequel y est compris entre 1 et 12, et de préférence entre 1 et 6, et
   - les groupements X et X', identiques ou différents, sont choisis parmi : N, O, S, une chaîne alkyle en C₁-C₄, les groupements X et X' étant de préférence des atomes d'oxygène.

Parmi les chaînes alkyles en C₁-C₄ mentionnées pour R₂, on peut en particulier citer les radicaux méthyle, éthyle, *n*-propyle, isopropyle, *n*-butyle, *tert*-butyle, isobutyle et *n*-hexyle, le radical méthyle étant le plus préféré.

Parmi les cycles hydrocarbonés saturés mentionnés pour R₂, on peut en particulier citer le cyclopropane, le cyclobutane, le cyclopentane et le cyclohexane.

Parmi les cycles hydrocarbonés insaturés et les hétérocycles saturés mentionnés pour R₂, on peut en particulier citer les cycles phényle, oxadiazole, triazole, oxazole, isoxazole, imidazole, thiadiazole, pyrrole, tétrazole, furane, thiophène, pyrazole, pyrazoline, pyrazolidine, thiazole, isothiazole, pyridine, pyrimidine, pipéridine, pyranne, pyrazine, pyridazine, indole, indazole, benzoxazole, naphtalène, quinoline, quinoxaline, quinazoline, anthracène et acridine, les cycles phényles étant les plus préférés.

Selon un mode de réalisation avantageux, lorsque le composé de l'invention est un composé de formule (I), la nanoparticule A est choisie parmi les nanoparticules d'or, de fer et de cobalt, et plus particulièrement les nanoparticules d'or. Les nanoparticules A peuvent avoir un diamètre compris entre 2 et 10 nm, et de préférence entre 4 et 8 nm.

Du fait de la présence de têtes multipolaires (groupements B), les composés de formule (I) présentent une meilleure affinité vis-à-vis des récepteurs, et par conséquent des propriétés anti-angiogéniques améliorées par rapport aux composés connus de l'art antérieur.

Selon un autre mode de réalisation avantageux, lorsque le composé de l'invention est un composé de formule (II) dans lequel : 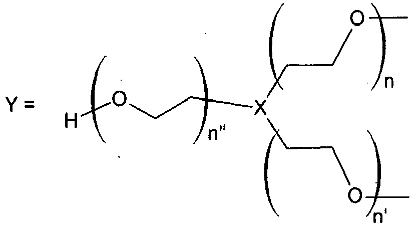 alors n = 1, n' = 2 et n" = 0, et X représente un atome d'oxygène.

Les composés conformes à l'invention peuvent être utilisés à titre de principe actif pour la fabrication de médicaments, en particulier pour la prévention et/ou le traitement de maladies dépendantes d'une inhibition de l'angiogénèse, parmi lesquelles on peut tout particulièrement citer les maladies cancéreuses, la cécité diabétique, la dégénérescence maculaire, la polyarthrite rhumatoïde et le psoriasis.

Un autre objet de l'invention est une composition pharmaceutique comprenant, à titre de principe actif, au moins un composé selon l'invention tel que défini ci-dessus, et au moins un excipient pharmaceutiquement acceptable, ladite composition pouvant elle aussi être utilisée pour la prévention et/ou le traitement de maladies dépendantes d'une inhibition de l'angiogénèse, telles que celles mentionnées précédemment.

La forme du médicament ou de la composition pharmaceutique peut être une solution, une suspension, une émulsion, des comprimés, des gélules, des suppositoires, et dépendra de la voie d'administration choisie.

Ainsi, le médicament ou la composition pharmaceutique de l'invention peut être administrée selon n'importe quelle voie appropriée, par exemple par voir orale, locale, systémique, intraveineuse, intramusculaire ou mucosale, ou bien en utilisant un patch.

En fonction de la voie d'administration du médicament ou de la composition pharmaceutique de l'invention, l'homme du métier choisira un ou plusieurs excipients pharmaceutiquement appropriés. On peut notamment citer, à titre d'exemples non limitatifs d'excipients appropriés pour une administration par voie orale : le talc, le lactose, l'amidon et ses dérivés, la cellulose et ses dérivés, les polyéthylèneglycols, les polymères d'acide acrylique, la gélatine, le stéarate de magnésium, les matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc.

Lorsqu'elle est destinée au traitement de maladies cancéreuses, la composition pharmaceutique peut en outre comprendre un ou plusieurs principes actifs antitumoraux parmi lesquels on peut citer des antimitotiques, des inducteurs de la différenciation, des anticorps, etc. Plus particulièrement, ces principes actifs peuvent être la doxorubicine, l'étoposide, le fluorouracile, le melphalan, la cyclophosphamide, la béomycine, la vinblastine, la mitomycine, la lomustine (CCNU), le taxotère, le taxol, le métotrexate et le cisplatinum.

Les composés de l'invention peuvent être préparés selon des procédés bien connus de l'homme de l'art, ces synthèses ayant déjà été décrites dans les documents suivants :
- B. G. Davis et al., J. Org. Chem., 1998, 63, 9614-9615,
- M. E. Evans et al., Carb. Res., 1977, 54,105-114,
- P. A. M. Van der Klein et al., Carb. Res., 1992, 224,193-200,
- H. H. Baer et al., Carb. Res., 1990, 200, 377-389,
- C. Grondal, Synlett, 2003, 10, 1568-1569,
- E. A. Hauser et al., Experiments in Colloid Chemistry, McGraw Hill, 1940, p.18,
- J. Turkevich et al., Discuss. Faraday. Soc., 1951, 11, 55-75,
- J. Kimling et al., J. Phys. Chem. B, 2006, 110, 15700-15707,
- Formation de boronates : R. Soundararajan et al., J. Org. Chem., 1990, 55, 2274-2275,
- Formation de borates :
   J. Meulenhoff et al., Allg. Chem., 1925, 373,
   S. D. Ross et al., J. Org. Chem., 1965, 30, 2852,
- C. J. Salomon et al., Tetrahedron Lett., 1995, 36, 6759-6760,
- Formation depyrophosphonates : A. M. Michelson, Biochem. Acta., 1964, 91, 1-13,
- Synthèse d'hydrogénophosphonates :
   Clavel et al., Tetrahedron Letters, 2004, 45(40), 7465-7467,
   Z. Ge et al., Journal of Applied Polymer Science, 2007, 104 (2), 1138-1142,
- K. Jarowicki et al., Journal of the Chemical Society-Perkin Transactions, 2001, (18), 2109-2135,
- F. Onyemauwa et al., Organic Letters, 2006, 8, 5255-5258.

Les composés de l'invention peuvent également être préparés selon un procédé écologique, aussi appelé « procédé par chimie verte ». Ce procédé présente l'avantage de ne pas nécessiter de solvant, ni d'étape supplémentaire de purification par chromatographie, tout en permettant l'obtention de rendements élevés. En effet, les procédés décrits dans l'art antérieur nécessitent très souvent des étapes de protection/déprotection qui sont consommatrices de réactifs et de solvants chers et polluants. Ainsi, les inventeurs ont mis au point un procédé permettant de surmonter ces inconvénients, ledit procédé comprenant au moins les étapes suivantes :
(i) une étape d'halogénation entre un composé de formule (I'), (II') ou (III') porteur d'au moins une fonction alcool primaire, par réaction avec un mélange dihalogène/phosphine ou N-halogénosuccinimide/phosphine, lesdits composés (I'), (II') ou (III') répondant aux formules ci-dessus :
   ➢ Formule (I') : 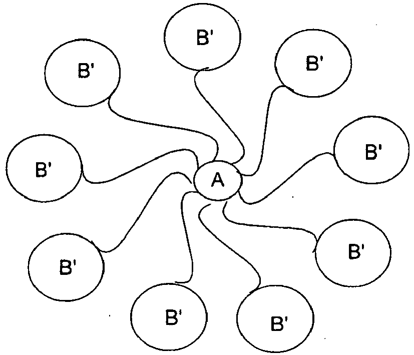 dans laquelle A a la même signification que précédemment, et B' est un groupement répondant à la structure suivante : 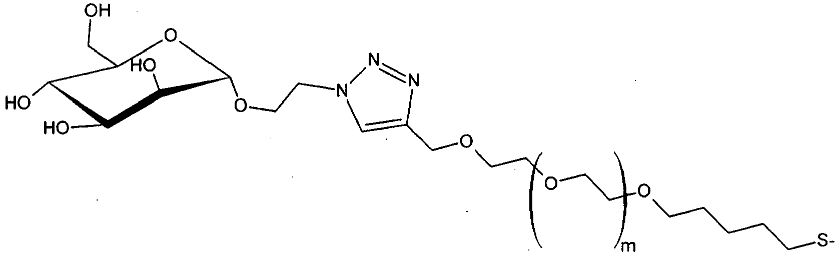 dans lequel m a la même signification que précédemment,
      les groupements B' étant liés à la nanoparticule A via l'atome de soufre, et le nombre de groupements B' liés à la nanoparticule A étant compris entre 100 et 1000, et de préférence entre 400 et 600,
   ➢ Formule (II') : 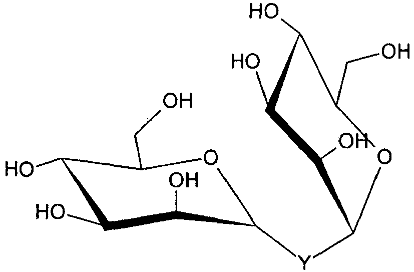 dans laquelle Y, n, n' et n" ont la même signification que précédemment,
   ➢ Formule (III') : 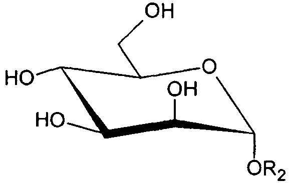 le radical R₂ et les groupements X et X' étant tels que définis précédemment,
(ii) une étape de substitution nucléophile des composés halogénés obtenus lors de l'étape (i) par réaction avec un réactif nucléophile porteur d'un radical R₁ et/ou R'₁, ce dernier étant de préférence un lithien, le réactif nucléophile pouvant notamment être choisi parmi LiO-PO₃H₂, NaN₃, LiCH₂-PO₃H₂, LiCH₂-COOH, LiSO₃H₂, LiOPHO₂H, LiCN₂-B(OH)₂, LiX-PHO₂H, LiX'-PO₂H-X-PO₃H₂, pour obtenir les composés de formule (I), (II) ou (III) de l'invention.

Lors des étapes (i) et (ii), les réactifs sont de préférence utilisés dans des proportions stoechiométriques.

Selon un mode de réalisation avantageux, la phosphine utilisée lors de l'étape (i) est choisie parmi une trialkylphosphine dont la chaîne alkyle est en C₁-C₆, la triphénylphosphine P_{ϕ3} ou la 2,2'-bis(diphénylphosphino)-1,1'-binaphthyle, et de manière encore plus préférée l'étape (i) est réalisée avec un mélange I₂/P_{ϕ3}, en présence d'imidazole. Les réactifs utilisés lors de l'étape (i) sont de préférence préalablement broyés, puis chauffés dans un bain d'huile à une température pouvant varier de 60 à 100°C, sous agitation, pendant une durée comprise entre 15 et 30 minutes. A la fin de l'étape (i), le mélange réactionnel est dissous dans un solvant tel que le méthanol, concentré, puis filtré sur gel de silice.

L'étape (ii) peut, quant à elle, avantageusement être réalisée en mélangeant le composé halogéné obtenu lors de l'étape (i) avec un réactif nucléophile porteur d'un radical R₁ et/ou R'₁. Les réactifs utilisés lors de l'étape (ii) sont de préférence préalablement broyés, puis chauffés dans un bain d'huile à une température pouvant varier de 60 à 100°C, sous agitation, pendant une durée comprise entre 15 et 30 minutes. A la fin de l'étape (ii), le mélange réactionnel est purifié, de préférence par filtration flash sur gel de silice.

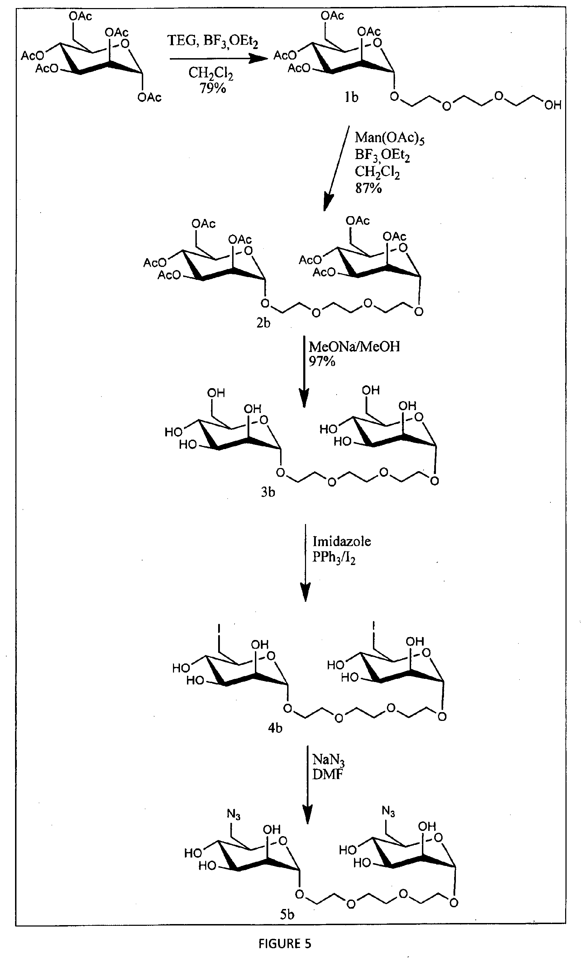

Un exemple de synthèse selon le « procédé par chimie verte » est schématisé à la Figure 5 annexée, le procédé mis en oeuvre comprenant une étape d'iodisation de l'α-D-mannopyranoside, l'iodo-α-D-mannopyranoside obtenu étant ensuite mis à réagir en proportions stoechiométriques avec de l'azide de sodium (NaN₃), ce dernier permettant la fonctionnalisation du mannopyranoside en position 6.

Enfin, un dernier objet selon l'invention concerne un dispositif médical implantable dans le corps humain, ledit dispositif étant traité en surface par au moins un composé dérivé de mannopyranoside selon l'invention. Parmi les dispositifs mentionnés ci-dessus, on peut citer les prothèses, et plus particulièrement les stents vasculaires, urétrals et biliaires. Le besoin de tels dispositifs existe car, actuellement, de nombreux dispositifs médicaux ne permettent qu'une implantation limitée dans le corps humain, du fait d'une angiogénèse excessive.

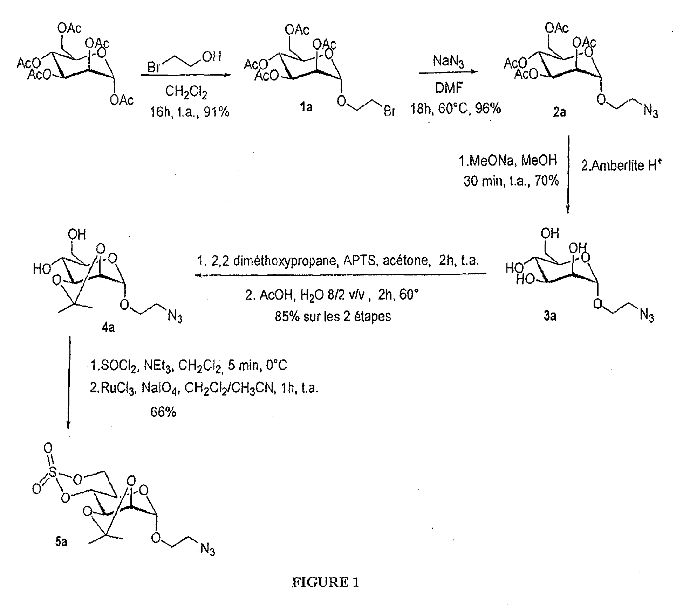

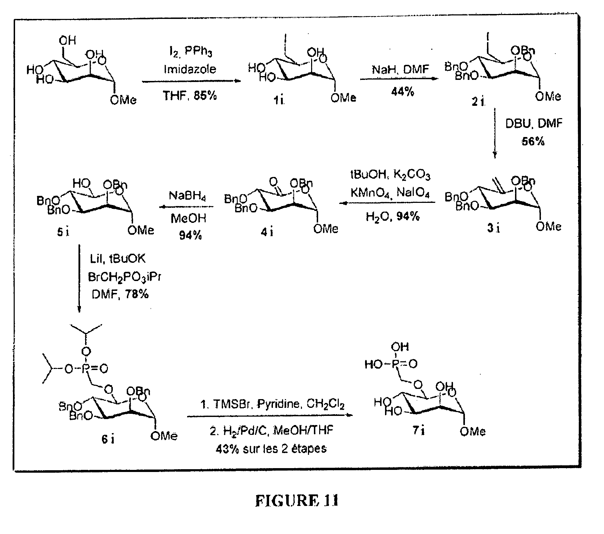

Outre les dispositions qui précèdent, l'invention comprend encore d'autres dispositions qui ressortiront de la description qui va suivre, qui se réfère à des exemples de préparation des composés conformes à l'invention, ainsi qu'à des exemples de mise en évidence de l'activité anti-tumorale des composés de l'invention par rapport à d'autres composés représentatifs de l'état de l'art ne faisant pas partie de l'invention, ainsi qu'aux figures 1 à 15 annexées, suivantes :
- la Figure 1 représente le schéma de synthèse des composés **1a** à **5a,**
- la Figure 2 représente le schéma de synthèse des composés **6a** à **9a,**
- la Figure 3 représente le schéma de synthèse des composés **10a** à **13a** (fonctionnalisation du mannopyranoside en position 6 par un groupement carboxy),
- la Figure 4 représente le schéma de synthèse des composés **14a** à **16a** (fonctionnalisation du mannopyranoside en position 6 par un groupement azido),
- la Figure 5 représente le schéma de synthèse d'un composé de formule (II) selon l'invention,
- la Figure 6 représente le schéma de synthèse d'un composé de formule (III) selon l'invention,
- la Figure 7 représente le schéma de synthèse d'un composé de formule (III) selon l'invention,
- la Figure 8 résume la synthèse d'un dérivé pyrophosphonate (composés **1g** à **6g**) répondant à la formule (III) de l'invention.
- la Figure 9 résume la synthèse d'un dérivé pyrophosphate (composés **1h** à **6h**) répondant à la formule (III) de l'invention,
- la Figure 10 représente le schéma de synthèse d'un composé répondant à la formule (II) de l'invention,
- la Figure 11 représente le schéma de synthèse d'un composé répondant à la formule (III) de l'invention (composés **1i** à **7i**),
- les Figures 12a et 12b sont des histogrammes représentant l'effet néo-angiogénique *in vitro* de différents composés de l'invention (modèle de Nicosia),
- la Figure 13 est un histogramme montrant l'effet cytotoxique de différents composés de l'invention,
- la Figure 14 est un graphe représentant le taux de survie de souris en fonction du nombre de jours de traitement, pour différents composés de l'invention,
- la Figure 15 est un graphe représentant la croissance tumorale en fonction du nombre de jours de traitement, pour différents composés de l'invention, et
- la Figure 16 représente des photographies de la vascularisation d'embryons de poulets lors d'un test classique d'étude de l'angiogénèse *in vivo* réalisé sur la membrane chorioallantoïdienne (CAM).

Il doit être entendu toutefois que ces exemples ne sont donnés qu'à titre purement illustratif de l'invention, dont ils ne constituent en aucune manière une quelconque limitation.

### EXEMPLE 1 : PREPARATION D'UN COMPOSE DE FORMULE (I)

### 1) SYNTHESE DU 2-BROMOETHYL-2,3,4,6-TETRA-O-ACETYL-α-D-MANNOPYRANOSE (Composé 1a)

8 g d'acétyl-2,3,4,6-tétra-*O*-α-D-mannopyranose (20,5 mmol, 1 éq.) dissous dans 70 mL de dichlorométhane sont mis à réagir avec 4,34 mL de 2-bromoéthanol (61,5 mmol, 2 éq.) et 15,5 mL de BF₃-Et₂O (123 mmol, 5 éq.). Après 8 heures d'agitation à température ambiante, le mélange réactionnel est dilué dans du CH₂Cl₂, lavé avec de l'eau, une solution saturée en NaHCO₃, puis à nouveau avec de l'eau. La phase aqueuse est ensuite séchée sur du Na₂SO₄, filtrée et évaporée. Le produit est purifié par chromatographie sur colonne de gel de silice (acétate d'éthyle (AcOEt)/éther de pétrole (EP) 5/5 v/v). On obtient le 2-bromoéthyl-2,3,4,6-tétra-*O*-acétyl-α-D-mannopyranoside sous forme de poudre blanche (8,5 g, 91 %).
Rf : 0,86 (AcOEt/EP 5/5 v/v).
SM (ESI⁺/MeOH) : m/z 477,01, 478,95 [M+Na]⁺.
RMN ¹H (400,13 MHz, CDCl₃) δ (ppm) : 2,00, 2,05, 2,11, 2,16 (4s, 12H, H_{b}) ; 3,52 (t, 2H, J₈₋₇ = 6,0 Hz, H₈) ; 3,93 (m, 2H, H₇) ; 4,13 (m, 2H, H₅ et H₆ₐ) ; 4,27 (dd, 1N, J_{6b-5} = 5,8 Hz, J_{6b-6a} = 12,6 Hz; H_{6b}) ; 4,88 (d, 1H, J₁₋₂ = 1,6 Hz, H₁) ; 5,27 (dd, 1H, J₂₋₁ = 2,0 Hz, J₂₋₃ = 3,2 Hz; H₂) ; 5,29 (t, 1H, J₄₋₅ = J₄₋₃ = 1,6 Hz, H₄) ; 5,35 (dd, 1H, J₃₋₂ = 3,6 Hz, J₃₋₄ = 10,0 Hz, H₃).
RMN ¹³C (100,62 MHz, CDCl₃) δ (ppm) : 20,67, 20,70, 20,75, 20,87 (4 C_{b}) ; 29,60 (C₈) ; 62,41 (C₆) ; 66,00 (C₄) ; 68,48 (C₇) ; 68,93 (C₅) ; 69,02 (C₃) ; 69,42 (C₂) ; 97,75 (C₁) ; 169,76, 169,86, 170,03, 170,62 (4 Cₐ).

### 2) SYNTHESE DU 2'-AZIDOETHYL-2,3,4,6-TETRA-O-ACETYL-α-D-MANNOPYRANOSE (Composé 2a)

5,7 g de 2-bromoéthyl-2,3,4,6-tétra-*O*-acétyl-α-D-mannopyranoside (composé **1**) (12,6 mmol, 1 éq.) et 1,64 g d'azide de sodium (25,16 mmol, 2 éq.) sont dissous dans 50 mL de diméthylformamide (DMF). Après 4 heures d'agitation à une température de 65°C, le mélange réactionnel est dilué dans 50 mL d'AcOEt et extrait avec une solution de NaCl saturée, puis lavé avec de l'eau distillée pour enlever le DMF. La phase organique est ensuite séchée sur du Na₂SO₄, filtrée et concentrée pour donner un solide blanc, le 2'-azidoéthyl-2,3,4,6-tétra-O-acétyl-α-D-mannopyranoside (5,25 g, 96%).
**Rf :** 0,86 (AcOEt/EP 5/5 v/v).
**SM (ESI⁺/MeOH)** m/z : 440,12 [M+Na]⁺.
**RMN ¹H (400,13 MHz, CDCl₃)** δ (ppm) : 2,00, 2,05, 2,11, 2,16 (4s, 12H, H_{b}) ; 3,47 (m, 2H, H₈) ; 3,67 (m, 1H, H₇ₐ) ; 3,87 (m, 1H, H_{7b}) ; 4,05 (ddd, 1H, J₅₋₆ₐ = 2,4 Hz, J_{5-6b} = 5,2 Hz, J₅₋₄ = 9,7 Hz, H₅) ; 4,13 (dd, 1 H, J₆ₐ₋₅ = 2,6 Hz, J_{6a-6b} = 12,2 Hz, H₆ₐ) ; 4,29 (dd, 1H, J_{6b-5} = 5,2 Hz, J_{6b-6a} = 12,4 Hz, H_{6b}) ; 4,87 (d, 1H, J₁₋₂ = 1,6 Hz, H₁) ; 5,30 (t, 1H, J₄₋₃ = J₄₋₅ = 10,0 Hz, H₄) ; 5,28 (dd, 1H, J₂₋₁ = 2,0 Hz, J₂₋₃ = 3,2 Hz, H₂) ; 5,36 (dd, 1H, J₃₋₂ = 3,2 Hz, J₃₋₄ = 10,0 Hz, H₃).
**RMN ¹³C (100,62 MHz, CDCl₃)** δ (ppm) : 20,63, 20,68, 20,71, 20,84 (4C, C_{b}) ; 50,32 (C₈) ; 62,42 (C₆) ; 65,96 (C₄) ; 67,02 (C₇) ; 68,82 (C₅ et C₃) ; 69,36 (C₂) ; 97,71 (C₁) ; 169,73, 169,78, 169,98, 170,59 (4C, Cₐ).

### 3) SYNTHESE DU 2'-AZIDOETHYL-α-D-MANNOPYRANOSE (Composé 3a)

6,8 g de 2'-azidoéthyl-2,3,4,6-tétra-*O*-acétyl-α-D-mannopyranoside (16,3 mmol, 1 éq.) et 880 mg de méthanolate de sodium (16,3 mmol, 1 éq.) sont dissous dans 60 mL de méthanol. Après 30 minutes d'agitation à température ambiante, la solution est neutralisée avec des billes de résines Amberlyst IRC-50-H⁺, filtrée et concentrée. L'huile obtenue est ensuite purifiée par chromatographie sur colonne de gel de silice avec un gradient d'élution (CH₂Cl₂/MeOH 9/1 v/v jusqu'à CH₂Cl₂/MeOH 6/4 v/v) pour donner des cristaux blancs (2,44 g, 65%).
**Rf :** 0,4 (CH₂Cl₂/MeOH 8/2 v/v).
**SM (ESI⁺/MeOH)** m/z : 272,11 [M+Na]⁺ ; 288,02 [M+K]⁺ ; 521,19 [2M+Na]⁺.
**RMN ¹H (400,13 MHz, CD₃OD)** δ (ppm) : 3,41 (t, 2H, J₈₋₇ = 5,0 Hz, C₈) ; 3,60 (m, 3H, H₃, H₅ et H₇ₐ) ; 3,71 (m, 2H, H₄ et H₆ₐ) ; 3,85 (m, 2H, H₂ et H_{6b}) ; 3,92 (m, 1H, H_{7b}) ; 4,81 (d, 1 H, J₁₋₂ = 1,2 Hz, H₁).
**RMN ¹³C (100,62 MHz, CD₃OD)** δ (ppm) : 51,76 (C₈) ; 62,94 (C₆) ; 67,74 (C₇) ; 72,08 (C₂) ; 72,49 (C₄) ; 68,54, 74,93 (C₃ et C₅) ; 101,82 (C₁).

### 4) SYNTHESE DU 2'-AZIDOETHYL-2,3-O-ISOPROPYLIDENE-α-D-MANNOPYRANOSE (Composé 4a)

### Formation du di-O-isopropylidène :

9,5 g de 2'-azidoéthyl-α-D-mannopyranose (composé 3a) (38,10 mmol, 1 éq.) sont mis en suspension dans 40 mL d'acétone, 23,5 mL de 2,2-diméthoxypropane (190 mmol, 5 éq.) sont ensuite ajoutés, puis 362 mg d'acide paratoluènesulfonique (APTS) (1,9 mmol, 0,05 éq.) sont également ajoutés. Le mélange est laissé sous agitation magnétique à température ambiante pendant 4 heures. La réaction est suivie par CCM (AcOEt/éther de pétrole 6/4) qui indique alors qu'il ne reste plus de produit de départ (Rf = 0), quelques traces de monoisopropylidène (Rf = 0,5) sont observés, le produit majoritairement présent étant le diisopropylidène (Rf = 0,8). L'APTS est ensuite neutralisé par une solution de NaHCO₃ à 5%, et la solution est ensuite concentrée pour enlever toute trace d'acétone. Le di-*O*-isopropylidène obtenu est extrait à l'éther de pétrole, puis séché sur du Na₂SO₄, filtré et concentré. Le produit obtenu sous forme d'une huile jaune est assez pur pour être remis directement en réaction. La phase aqueuse contenant le mono-isopropylidène est ensuite lyophilisée.

### Ouverture sélective :

7,5 g de 2'-azidoéthyl-2,3,4,6-di-*O*-isopropylidène-α-D=mannopyranose (22,9 mmol, 1 éq.) sont dissous dans 60 mL d'un mélange acide acétique/eau 80/20 v/v. Après 2 heures d'agitation à une température de 35°C, le produit de départ (2-azidoéthyl-α-D-mannopyranose) réapparait. Les solvants sont alors évaporés puis co-évaporés avec du toluène. L'huile transparente obtenue est purifiée par chromatographie sur colonne de gel de silice (AcOEt/EP 6/4 v/v) pour donner une huile légèrement jaune (5,65 g, 85%).

### Caractérisation du diisopropylidène :

**Rf :** 0,63 (AcOEt/EP 5/5 v/v).
**SM (ESI⁺/MeOH)** m/z : 352,20 [M+Na]⁺ ; 368,02 [M+K]⁺.
**RMN ¹H (400,13 MHz, acétone-d₆)** δ (ppm) : 1,31, 1,32 (2s, 6H, H_{b} et H_{d}) ; 1,47, 1,48 (2s, 6H, H_{c} et Hₑ) ; 3,50 (t, 2H, J = 4,8 Hz, H₈) ; 3,53 (m, 1 H, H₅) ; 3,72 (m, 3H, H₆ₐ, H₄ et H₇ₐ) ; 3,82 (dd, 1H, J_{6b-5} = 5,8 Hz, J_{6b-6a} = 10,6 Hz, H_{6b}) ; 3,93 (qt, 1H, J = 5,2 Hz, H_{7b}) ; 4,03 (dd, 1H, J₃₋₂ = 5,6 Hz, J₃₋₄ = 8,0 Hz, H₃) ; 4,18 (d, 1H, J₂₋₃ = J₂₋₁ = 5,6 Hz, H₂) ; 5,09 (s, 1H, H₁).
**RMN ¹³C (100,62 MHz, acétone-d₆)** δ (ppm) : 20,11, 29,38 (C_{b} et C_{d}) ; 27,45, 30,50 (C_{c} et Ce) ; 52,18 (C₈) ; 63,48, 63,53 (C₅ et C₆) ; 68,17 (C₇) ; 74,47 (C₄) ; 76,78 (C₃) ; 77,83 (C₂) ; 99,68 (C₁) ; 109,76 (Cₐ).

### Caractérisation du monoisopropylidène :

**Rf :** 0,26 (AcOEt/EP 6/4 v/v).
**SM (ESI⁺/MeOH)** m/z : 312,12 [M+Na]⁺ ; 328,15 [M+K]⁺,
**(ESI-/MeOH)** m/z : 324,12 [M+Cl]⁻.
**RMN ¹H (400,13 MHz, acétone-d₆ + D₂O)** δ (ppm) : 1,27, 1,41 (2s, 6H, H_{b} et H_{c}) ; 3,45 (t, 2H, J = 5,0 Hz, H₈) ; 3,52 (m, 2H, H₄ et H₅) ; 3,62 (dd, 1H, J₆ₐ₋₅ = 5,2Hz, J_{6a-6b} = 11,6Hz, H₆ₐ) ; 3,67 (m, 1H, H₇ₐ) ; 3,80 (m, 1H, H_{6b}) ; 3,93 (m, 1H, H_{7b}) ; 4,02 (m, 1H, H₃) ; 4,09 (d, 1H, J₂₋₃ = J₂₋₁ = 5,6 Hz, H₂) ; 5,03 (s, 1H, H₁).
**RMN ¹³C (100,62 MHz, acétone-d₆** + **D₂O)** δ (ppm) : 2,34, 29,11 (C_{b} et C_{c}) ; 51,95 (C₈) ; 62,97 (C₆) ; 67,74 (C₇) ; 70,41, 72,62 (C₄ et C₅) ; 77,30 (C₂) ; 80,42 (C₃) ; 98,60 (C₁) ; 110,60 (Cₐ).

### 5) SYNTHESE DU 2'-AZIDOETHYL-2,3-O-ISOPROPYLIDENE-4,6-O-(CYCLOSULFATE)-α-D-MANNOPYRANOSE (Composé 5a)

### Formation du sulfite :

100 mg de 2'-azidoéthyl-2,3-*O*-isopropylidène-α-D-mannopyranose (composé **4a**) (0,345 mmol, 1 éq.) et 169 µL de triéthylamine (0,001 mmol, 3 éq.) sont dissous dans 2 mL de CH₂Cl₂. Le ballon est placé dans un bain de glace et 27 µL de chlorure de thionyle (0,38 mmol, 1,1 éq.) sont ajoutés lentement. Un précipité blanc de chlorure de triéthylammonium apparaît rapidement et le mélange réactionnel devient progressivement jaune, puis marron. Après 5 minutes d'agitation à une température de 0°C, il ne reste plus de produit de départ, le sulfite désiré est obtenu sous forme de 2 diastéréoisomères (Rf = 0,53 et 0,62 (AcOEt/EP 5/5)). Le mélange est filtré, la phase organique est lavée avec de l'eau distillée, une solution d'acide chlorhydrique (HCl) à 1N, puis à nouveau avec de l'eau. La phase organique est ensuite séchée sur du Na₂SO₄, filtrée et concentrée pour donner un solide marron directement remis en réaction.

### Formation du sulfate :

Le sulfite brut (0,345 mmol, 1 éq.) est dissous dans 2 mL d'un mélange CH₂Cl₂/CH₃CN (1/1 v/v). 81 mg de métapériodate de sodium (0,38 mmol, 1,1 éq.), 0,5 mL d'eau et un grain de chlorure de ruthénium (1,38.10⁻³ mmol, 0,004 éq.) sont ajoutés successivement. La réaction est exothermique, un précipité de NaIO₃ se forme très rapidement. Après 1 heure d'agitation à température ambiante, le sulfite a été consommé, le mélange réactionnel est filtré et dilué dans 20 mL de CH₂Cl₂. La phase organique est lavée avec une solution de NaHCO₃ à 5%, de l'eau distillée, puis séchée, filtrée et concentrée. Le solide marron obtenu est dissous dans un minimum de CH₂Cl₂ et filtré sur silice. La silice est rincée plusieurs fois avec du CH₂Cl₂. On obtient alors un solide blanc (80 mg, 66%).
**Rf :** 0,58 (AcOEt/EP 5/5 v/v).
**SM (ESI⁺/MeOH)** m/z : 374,13 [M+Na]⁺,
**(ESI⁺/MeOH)** m/z : 386,08 [M+Cl]⁻.
**RMN ¹H (400,13 MHz, acétone-d₆)** δ (ppm) : 1,37, 1,52 (2s, 6H, C_{b} et C_{c}) ; 3,55 (m, 2H, H₈) ; 3,80 (m, 1H, H₇ₐ) ; 4,29 (m, 1H, H_{7b}) ; 4,26 (td, 1H, J₅₋₄ = J₅₋₆ₐ = 10,7 Hz, J_{5-6b} = 5,5 Hz, H₅) ; 4,36 (d, 1H, J₂₋₁ = J₂₋₃ = 6,0 Hz, H₂) ; 4,43 (dd, 1H, J₃₋₂ = 5,6 Hz, J₃₋₄ = 8,0 Hz, H₃) ; 4,6 (dd, 1H, J₄₋₃ = 7,6 Hz, J₄₋₅ = 10,8 Hz, H₄) ; 4,63 (t, 1H, J₆ₐ₋₅ = J_{6a-6b} = 10,8 Hz, H₆ₐ) ; 4,84 (dd, 1H, J_{6b-5} = 5,6 Hz, J_{6b-6a} = 10,4 Hz, H_{6b}) ; 5,28 (s, 1H, H₁).
**RMN ¹³C (100,62 MHz, acétone-d₆)** δ (ppm) : 27,16, 29,13 (C_{b} et C_{c}) ; 52,06 (C₈) ; 60,35 (C₅) ; 68,75 (C₇) ; 74,34 (C₆) ; 74,95 (C₃) ; 77,88 (C₂) ; 86,65 (C₄) ; 99,70 (C₁) ; 112,07 (Cₐ).

Les synthèses des composés 1a à 5a décrites ci-dessus sont résumées à la Figure 1 annexée.

### 6) SYNTHESE DU PENT-1-EN-5-YLHEXA(ETHYLENEGLYCOL) (Composé 6a)

25 g de hexa(éthylèneglycol) (88,5 mmol, 4,12 éq.) sont dissous dans une solution de NaOH à 50%. Après 30 minutes d'agitation à une température de 100°C, 2,58 mL de 5-bromopent-1-ène (21,85 mmol, 1 éq.) sont ajoutés. On maintient le mélange sous agitation à une température de 100°C pendant 15 minutes. Le mélange est ensuite dilué dans du CH₂Cl₂ et le produit est extrait avec de l'éther de pétrole. De l'eau est ajoutée à la phase CH₂Cl₂ qui est ré-extraite plusieurs fois par de l'éther de pétrole. Les phases organiques sont rassemblées, lavées avec un minimum d'eau, séchées sur du Na₂SO₄, filtrées et concentrées. L'huile jaune obtenue est purifiée sur colonne de gel de silice (AcOEt/EP 9/1 v/v puis AcOEt/MeOH 9/1 v/v) pour donner une huile liquide jaune (3,19 g, 99%).
**Rf :** 0,14 (AcOEt/EP 5/5 v/v).
**SM (ESI⁺/MeOH)** m/z : 373,27 [M+Na]⁺ ; 389,20 [M+K]⁺.
**RMN ¹H (400,13 MHz, CDCl₃)** δ (ppm) : 1,68 (m, 2H, H₁₄) ; 2,09 (m, 2H, H₁₅) ; 3,46 (t, 2H, J₁₃₋₁₄ = 6,6 Hz, H₁₃) ; 3,56-3,73 (m, 24H, H₁₋₁₂) ; 4,99 (m, 2H, H₁₇) ; 5,81 (m, 2H, H₁₆).
**RMN ¹³C (100,62 MHz, CDCl₃)** δ (ppm) : 28,66 (C₁₄) ; 30,12 (C₁₅) ; 61,51, 61,58, 69,98, 70,11, 70,21, 70,35, 70,47, 70,58, 72,46 et 72,58 (13C, C₁₋₁₃) ; 114,59 (C₁₇) ; 138,18 (C₁₆).

### 7) SYNTHESE DU (1-THIOACETYLPENT-5-YL)HEXA(ETHYLENEGLYCOL) (Composé 7a)

3,1 g de pent-1-èn-5-ylhexa(éthylèneglycol) (8,85 mmol, 1 éq.), 3,1 mL d'acide thioacétique (44,3 mmol, 5 éq.) et une spatule d'azobisisobutyronitrile (AIBN) (100 mg) sont dissous dans 12 mL de tétrahydrofurane fraîchement distillé sur du sodium. Après 1 heure d'agitation à reflux (90-100°C), le mélange est dilué dans de l'AcOEt puis lavé avec une solution de NaHCO₃ saturée. La phase organique est séchée sur du Na₂SO₄, filtrée et concentrée pour donner une huile liquide jaune qui est purifiée sur colonne de gel de silice (AcOEt/EP 9/1 v/v puis AcOEt/MeOH 9/1 v/v). On obtient une huile jaune liquide (2,68 g, 71 %).
**Rf :** 0,27 (AcOEt/MeOH 9/1 v/v).
**SM (ESI⁺/MeOH)** m/z : 449,26 [M+Na]⁺,
**(ESI⁻/MeOH)** m/z : 461,17 [M+Cl]⁻.
**RMN ¹H (400,13 MHz, CDCl₃)** δ (ppm) : 1,40 (m, 2H, H₁₅) ; 1,58 (m, 4H, H₁₄ et H₁₆) ; 1,83 (s, 1H, OH) ; 2,32 (s, 3H, H₁₉) ; 2,86 (t, 2H, J₁₇₋₁₆ = 7,2 Hz, H₁₇) ; 3,44 (t, 2H, J₁₃₋₁₄ = 6,6 Hz, H₁₃) ; 3,56-3,73 (m, 24H, H₁₋₁₂).
**RMN ¹³C (100,62 MHz, CDCl₃)** δ (ppm) : 25,25 (C₁₅) ; 28,90 (C₁₇) ; 28,99, 29,24 (C₁₄ et C₁₆) ; 30,52 (C₁₉) ; 61,55, 69,96, 70,17, 70,42, 70,98 et 72,43 (13C, C₁₋₁₃) ; 195,84 (C₁₈).

### 8) SYNTHESE DU (1-THIOMETHOXYTRITYLPENT-5-YL)HEXA(ETHYLENEGLYCOL) (Composé 8a)

### Déprotection de l'acétate :

2,6 g de (1-thioacétylpent-5-yl)hexa(éthylèneglycol) (60,9 mmol, 1 éq.) sont mis à réagir avec 3 mL de HCl concentré dans 65 mL d'éthanol absolu. Après 20 heures d'agitation à une température de 60°C, le mélange est neutralisé avec de l'ammoniaque, puis concentré. La solution obtenue est ensuite diluée dans de l'AcOEt, la phase organique est lavée à l'eau, séchée sur du Na₂SO₄, filtrée et concentrée. L'huile marron-noir obtenue est assez pure pour être remise directement en réaction.

### Protection avec le trityl :

Le thiol déprotégé est mis en présence de 2,8 g de chlorure de méthoxytrityl (91,3 mmol, 1,5 éq.) dans 60 mL de THF anhydre. Après 24 heures d'agitation à température ambiante, la solution est concentrée et purifiée par chromatographie sur colonne de gel de silice (AcOEt/MeOH 9/1 v/v) pour donner une huile jaune (3,65 g, 91%).
**Rf :** 0,4 (AcOEt/MeOH 7/3 v/v).
**SM (ESI⁺/MeOH)** mz : 679,34 [M+Na]⁺.
**RMN ¹H (400,13 MHz, acétone-d₆)** δ (ppm) : 1,31 (m, 2H, H₁₅) ; 1,40 (m, 4H, H₁₄ et H₁₆) ; 2,17 (t, 2H, J₁₇₋₁₈ = 7,4 Hz, H₁₇) ; 2,87 (s, 1H, OH) ; 3,35 (t, 2H, J₁₃₋₁₄ = 6,4 Hz, H₁₃) ; 3,47-3,63 (m, 24H, H₁₋₁₂) ; 3,79 (s, 3H, H₂₁) ; 6,86-7,42 (m, 14H, H_{Ar}).
**RMN ¹³C (100,62 MHz, acétone-d₆)** δ (ppm) : 27,37, 30,16 (3C, C₁₄, C₁₅ et C₁₆), 33,54 (C₁₇) ; 56,50 (C₂₁) ; 62,94, 62,94, 67,64, 71,82, 72,19 et 72,33 (13C, C₁₋₁₃) ; 74,48 (C₁₈) ; 114,86, 128,32, 129,61, 131,26 et 132,54 (14C, CH_{Ar}) ; 138,81, 147,37 (3C, C₁₉); 160,12 (C₂₀).

### 9) SYNTHESE DU O-(1-THIOMETHOXYTRITYLPENT-5-YL)-O-PROPARGYLHEXA(ETHYLENEGLYCOL) (Composé 9a)

100 mg de (1-thiométhoxytritylpent-5-yl)hexa(éthylèneglycol) (0,152 mmol, 1 éq.) sont dissous dans 3 mL de THF fraîchement distillé. 7,3 mg d'hydrure de sodium (0,183 mmol, 1,2 éq.) puis 16 µL de 2-bromopropyne (0,213 mmol, 1,4 éq.) sont ajoutés au mélange à une température de 0°C. Après 18 heures d'agitation à température ambiante, le mélange est concentré puis purifié sur colonne de gel de silice (AcOEt/EP 8/2 v/v). Le *O*-(1-thiométhoxytritylpent-5-yl)-*O*-propargylhexa(éthylèneglycol) est obtenu sous la forme d'une huile blanchâtre (103 mg, 97%).
**Rf :** 0,34 (AcOEt).
**SM (ESI⁺/MeOH)** m/z : 717,39 [M+Na]⁺.
**RMN ¹H (400,13 MHz, CDCl₃)** δ (ppm) : 1,28 (m, 2H, H₁₅) ; 1,42 (m, 4H, H₁₄ et H₁₆) ; 2,14 (t, 2H, J₁₇₋₁₆ = 7,4 Hz, H₁₇) ; 2,43 (t, 1H, J = 2,4 Hz, H_{2'}) ; 3,36 (t, 2H, J₁₃₋₁₄ = 6,8 Hz, H₁₃); 3,52-3,71 (m, 24H, H₁₋₁₂) ; 3,79 (s, 3H, H₂₁) ; 4,20 (d, 2H, J = 2,4 Hz, H_{1'}) ; 6,79-7,40 (m, 14H, H_{Ar}).
**RMN ¹³C (100,62 MHz, CDCl₃)** δ (ppm) : 25,59 (C₁₅) ; 28,46, 29,19 (C₁₄ et C₁₆) ; 31,96 (C₁₇) ; 55,20 (C₂₁) ; 58,39 (C_{1'}) ; 65,85 (C_{2'}) ; 69,10, 70,05, 70,40, 70,56 et 71,17 (13C, C₁₋₁₃) ; 74,51 (C₁₈) ; 113,03, 126,44, 127,77, 129,47 et 130,73 (14C, CH_{Ar}) ; 137,12, 145,32 (3C, C₁₉) ; 157,94 (C₂₀).

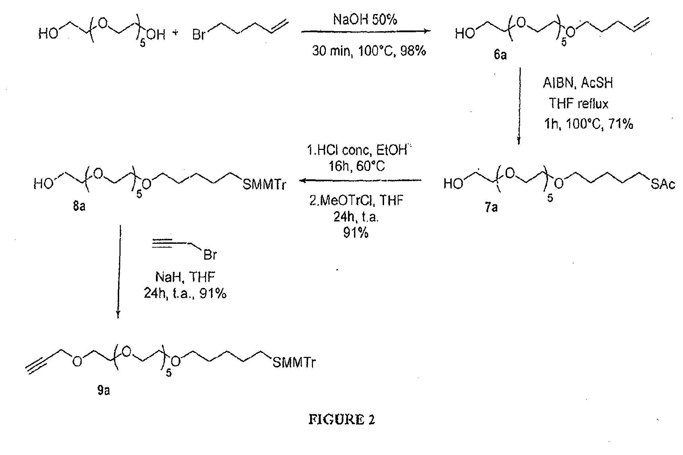

Les synthèses des composés **6a** à **9a** décrites ci-dessus sont représentées à la Figure 2 annexée.

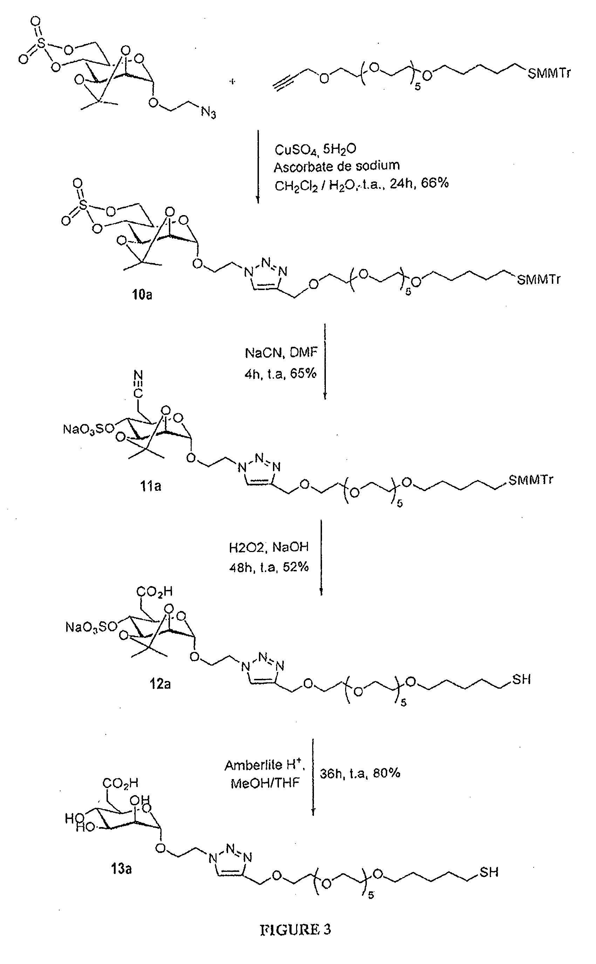

### « CLICK CHEMISTRY » DES COMPOSES 5α ET 9α - FONCTIONNALISATION DU MANNOPYRANOSIDE EN POSITION 6 PAR UN GROUPEMENT CARBOXY (cf: Figure 3) :

### 10) SYNTHESE DU {1-[2,3-O-ISOPROPYLIDENE-4,6-O-CYCLOSULFATE-α-D-MANNOPYRANOSYL]ETHYL-1H-1,2,3-TRIAZOL-4-YL}METHYL-[O-(1-THIOMETHOXYTRITYLPENT-5-YL)-O-HEXA(ETHYLENEGLYCOL)] (Composé 10a)

40 mg de 2'-azidoéthyl-2,3-*O*-isopropylidéne-4,6-*O*-cyclosulfate-α-D-mannopyranose (0,11 mmol, 1 éq.) et 88 mg de *O*-(1-thiométhoxytritylpent-5-yl)-*O-*propargylhexa(éthylèneglycol) (0,13 mmol, 1,1 éq.) sont dissous dans 4 mL d'un mélange CH₂Cl₂/H₂O (1/1 v/v). 7 mg de CuSO₄, 5H₂O (0,03 mmol, 0,25 éq.) et 11,3 mg d'ascorbate de sodium (0,06 mmol, 0,5 éq.) sont ajoutés. Après 24 heures d'agitation à température ambiante, le mélange réactionnel est dilué dans du CH₂Cl₂, puis lavé avec de l'eau. La phase organique est ensuite séchée, filtrée, concentrée et purifiée par chromatographie sur colonne de gel de silice avec un gradient d'éluant (CH₂C)₂/MeOH 99/1 v/v à 98/2 v/v) pour donner une huile incolore (80 mg, 64%).
**Rf :** 0,4 (CH₂Cl₂/MeOH 9/1 v/v).
**SM (ESI⁺/MeOH)** m/z : 1068,62 [M+Na]⁺,
**(ESI⁺/MeOH)** m/z : 1080,77 [M+Cl]⁻.
**RMN ¹H (400,13 MHz, CD₃OD)** δ (ppm) : 1,36 (m, 6H, H₂₅, H₂₆ et H₂₇) ; 1,34, 1,49 (2s, 6H, H_{b} et H_{C}) ; 2,14 (t, 2H, J₂₈₋₂₇ = 7,2 Hz, H₂₈) ; 3,38 (t, 2H, J₂₄₋₂₅ = 6,4 Hz, H₂₄) ; 3,44-3,66 (m, 25H, H₅ et H₁₂₋₂₃) ; 3,78 (s, 3H, H₃₂); 3,97 (m, 1H, H₇ₐ) ; 4,15 (m, 1H, H_{7b}); 4,27 (m, 3H, H₂, H₃ et H₆ₐ) ; 4,50 (m, 2H, H_{6b} et H₄) ; 4,64 (m, 4H, H₈ et H₁₁) ; 5,12 (s, 1H, H₁); 6,81-7,39 (m, 14H, H_{Ar}) ; 8,07 (s, 1H, H₉).
**RMN ¹³C (100,62 MHz, CD₃OD)** δ (ppm) : 26,34, 28,20 (C_{b} et C_{c}) ; 26,71 (C₂₆) ; 29,62 (C₂₇) 30,23 (C₂₅) ; 33,03 (C₂₈) ; 51,19 (C₈) ; 55,79 (C₃₂) ; 59,79 (C₅) ; 65,14 (C₁₁) ; 67,42 (C₇) ; 70,95, 71,19, 71,49, 71,58, 72,03 (13C, C₁₂₋₂₄) 73,53 (C₆) ; 74,46, 77,23 (C₂ et C₃) ; 85,66 (C₄) ; 98,96 (C₁); 108,26, 111,66 (C₂₉ et Cₐ) ; 114,11, 127,66, 128,86, 130,73 et 132,02 (14C, CH_{Ar}) ; 126,04 (C₉) ; 138,40, 146,86 (4C_{IV}, C₃₀ et C₁₀) ; 159,71 (C₃₁).

### 11) SYNTHESE DU {1-[6-CYANO-6-DEOXY-4-O-SODIUMSULFATE-2,3-O-ISOPROPYLIDENE-α-D-MANNOPYRANOSYL]ETHYL-1H-1,2,3-TRIAZOL-4-YL}METHYL-[O-(1-THIOMETHOXYTRITYLPENT-5-YL)-O-HEXA(ETHYLENEGLYCOL)] (Composé 11a)

160 mg de {1-[2,3-*O*-isopropylidène-4,6-*O*-cyclosulfate)-α-D-mannopyranosyl]éthyl-1*H-*1,2,3-triazol-4-yl}méthyl-[*O*-(1-thiométhoxytritylpent-5-yl)-*O*-hexa(éthylèneglycol)] (0,15 mmol, 1 éq.) et 15 mg de cyanure de sodium (0,31 mmol, 2 éq.) sont dissous dans 1,5 mL de DMF. Après 4 heures d'agitation à température ambiante, le mélange réactionnel est dilué dans 10 mL d'une solution de NaHCO₃ à 5% (pour éviter un éventuel risque de dégagement d'acide cyanhydrique HCN) et lavé avec du CH₂Cl₂. Le produit est extrait avec de l'eau, puis la phase aqueuse est lyophilisée. La poudre jaune obtenue est purifiée par chromatographie sur colonne de gel de silice (CH₂Cl₂/MeOH 9/1 v/v) pour donner une huile incolore (106 mg, 65%).
**Rf :** 0,24 (CH₂Cl₂/MeOH 9/1 v/v).
**SM (ESI⁺/MeOH)** m/z : 1117,77 [M+Na]⁺,
**(ESI⁺/MeOH)** m/z : 1071,63 [M-Na]⁻.
**RMN ¹H (400,13 MHz, CD₃OD)** δ (ppm) : 1,31 et 1,50 (2s, 6H, H_{b} et H_{C}) ; 1,38 (m; 6H, H₂₆, H₂₇ et H₂₈) ; 2,15 (t, 2H, J₂₉₋₂₈ = 7,2 Hz, H₂₉) ; 2,70 (dd, 1H, J₆ₐ₋₅ = 8,8 Hz, J_{6a-6b} = 17,2 Hz, H₆ₐ) ; 3,02 (dd, 1H, J_{6b-5} = 3,0 Hz, J_{6b-6a} = 17,0 Hz, H_{6b}) ; 3,38 (t, 2H, J₂₅₋₂₆ = 6,4 Hz, H₂₅), 3,48-3,66 (m, 26H, H_{4,5} et H₁₃₋₂₄) ; 3,78 (s, 3H, H₃₃) ; 3,93 (m, 1H, H₈ₐ) ; 4,10 (d, 1H, J₂₋₃ = J₂₋₁ = 4,8 Hz, H₂) ; 4,19 (m, 2H, H_{8b} et H₃) ; 4,65 (m, 4H, H₉ et H₁₂) ; 5,00 (s, 1H, H₁) ; 6,82-7,39 (m, 14H, H_{Ar}) ; 8,04 (s, 1H, H₁₀).
**RMN ¹³C (100,62 MHz, CD₃OD)** δ (ppm) : 21,83 (C₆) ; 26,60, 28,05 (C_{b} et C_{c}) ; 26,69 (C₂₇) ; 29,62 (C₂₈) ; 30,17 (C₂₆) ; 33,04 (C₂₉) ; 51,17 (C₉) ; 55,81 (C₃₃) ; 64,87 (C₁₂) ; 66,66, 76,70 (C₄ et C₅) ; 67,02 (C₈) ; 70,47, 70,99, 71,20, 71,26, 71,35 et 72,02 (13C, C₁₃₋₂₅) ; 77,54 (C₂) ; 77,92 (C₃) ; 98,52 (C₁) ; 110,98 (C₃₀ et Cₐ) ; 118,96 (C₇) ; 114,12, 127,67, 128,87, 130,72 et 132,01 (14C, CH_{Ar}); 125,87 (C₁₀) ; 138,38, 146,00 et 146,84 (4C_{IV}, C₃₁ et C₁₁) ; 159,71 (C₃₂).

### 12) SYNTHESE DU {1-[ACIDE(6,7-DIDESOXY-4-O-SODIUMSULFATE-2,3-O-ISOPROPYLIDENE-α-D-MANNO-HEPTOPYRANOSYL)URONIQUE]ETHYL-1H-1,2,3-TRIAZOL-4-YL}METHYL-[O-(1-PENT-5-YL)-O-HEXA(ETHYLENEGLYCOL)] (Composé 12a)

200 mg de {1-[6-cyano-6-déoxy-4-*O*-sodiumsulfate-2,3-*O*-isopropylidène-α-D-mannopyranosyl]éthyl-1*H-*1,2,3-triazol-4-yl}méthyl-[*O*-(1-thiométhoxytritylpent-5-yl)-*O-*hexa(éthylèneglycol)] (0,18 mmol, 1 éq.) et 60 mg de soude (NaOH) (1,46 mmol, 8 éq.) sont dissous dans 1,5 mL d'une solution aqueuse de peroxyde d'hydrogène à 30%. La solution est agitée à température ambiante. A 12 heures et 24 heures de réaction, 60 mg de NaOH et 1,5 mL de H₂O₂ sont ajoutés au milieu réactionnel. Après 48 heures, la solution est neutralisée avec des résines Amberlyst H⁺, avant d'être filtrée et lyophilisée. Le produit obtenu est ensuite purifié par chromatographie sur colonne de gel de silice avec un gradient d'élution (CH₂Cl₂/MeOH 9/1 v/v à NH₄OH/iPrOH 5/5 v/v) pour donner une huile jaune (80 mg, 52%).
**Rf :** 0 (CH₂Cl₂/MeOH 8/2 v/v).
**SM (ESI⁺/MeOH)** m/z : 864,34 [M+Na]⁺.
**RMN ¹H (400,13 MHz, D₂O)** δ (ppm) : 1,35 et 1,52 (2s, 6H, H_{b} et H_{C}) ; 1,44 (m, 2H, H₂₇) ; 1,60 (m, 2H, H₂₆) ; 1,73 (m, 2H, H₂₈) ; 2,29 (dd, 1H, J₆ₐ₋₅ = 10,6 Hz, J_{6a-6b} = 15,0Hz, H₆ₐ) ; 2,80 (dd, 1H, J_{6b-5} = 2,0 Hz, J_{6b-6a} = 15,2 Hz, H_{6b}) ; 2,89 (t, 2H, J₂₉₋₂₈ = 8,0 Hz, H₂₉) ; 3,53 (t, 2H, J₂₅₋₂₆ = 6,6 Hz, H₂₅) ; 3,50-3,69 (m, 26H, H₅ et H₁₃₋₂₄) ; 3,88 (m, 1H, H₈ₐ) ; 4,17 (m, 2H, H_{8b} et H₄) ; 4,19 (d, 1H, J₂₋₃ = J₂₋₁ = 5,6 Hz, H₂) ; 4,30 (m, 1H, H₃) ; 4,68 (m, 4H, H₉ et H₁₂) ; 4,95 (s, 1H, H₁) ; 8,12 (s, 1H, H₁₀).
**RMN ¹³C (100,62 MHz, D₂O)** δ (ppm) : 23,80 (C₂₈) ; 24,24 (C₂₇) ; 25,45 et 26,80 (C_{b} et C_{c}) ; 28,06 (C₂₆) ; 38,92 (C₆) ; 49,99 (C₉) ; 50,89 (C₂₉) ; 62,95 (C₁₂) ; 65,47 (C₈) ; 66,41 (C₅) ; 66,54, 68,69, 69,07, 69,40, 69,53 et 70,70 (13C, C₁₃₋₂₅) ; 75,21 (C₂) ; 76,00 (C₃) ; 78,30 (C₄) ; 95,99 (C₁) ; 110,39 (Cₐ) ; 125,55 (C₁₀) ; 143,85 (C₁₁) ; 177,75 (C₇).

### 13) SYNTHESE DU {1-[ACIDE(6,7-DIDESOXY-α-D-MANNOHEPTOPYRANOSYL)URONIQUE]ETHYL-1H-1,2,3-TRIAZOL-4-YL}METHYL-[O-(1-PENT-5-YL)-O-HEXA(ETHYLENEGLYCOL)] (Composé 13a)

60 mg de {1-[acide(6,7-didésoxy-4-*O*-sodiumsulfate-2,3-*O*-isopropylidëne-α-D-mannoheptopyranosyl)uronique]éthyl-1*H*-1,2,3-triazol-4-yl}méthyl-[*O*-(1-pent-5-yl)-*O-*hexa(éthylèneglycol)] (0,07 mmol, 1 éq.) sont dissous dans 2 mL d'un mélange MeOH/THF (1/1 v/v), puis mis à réagir avec des résines Amberlyst 15-H⁺ pendant 36 heures. Les résines sont ensuite filtrées et la solution est neutralisée avec une solution de NaHCO₃ à 5%. Les solvants organiques sont évaporés et l'eau restante est lyophilisée. Le mélange est repris au méthanol et le NaHCO₃ insoluble est filtré. L'huile obtenue est assez pure pour être remise directement en réaction (40 mg, 80%).
**Rf :** 0,18 (AcOEt/MeOH 5/5 v/v).
**SM (ESI⁺/MeOH)** m/z : 765,86 [M-3H+3Na]⁺.
**RMN ¹H (400,13 MHz, CD₃OD)** δ (ppm) : 1,49 (m, 2H, H₂₇) ; 1,61 (m, 2H, H₂₆) ; 1,80 (m, 2H, H₂₈) ; 2,41 (dd, 1H, J₆ₐ₋₅ = 10,2 Hz, J_{6a-6b} = 16,2 Hz, H₆ₐ) ; 2,84 (m, 3H, H₂₉ et H_{6b}) ; 3,49 (t, 2H, J₂₅₋₂₆ = 6,4 Hz, H₂₅) ; 3,40-3,79 (m, 28H, H₂₋₅ et H₁₃₋₂₄) ; 3,92 (m, 1H, H₈ₐ) ; 4,22 (m, 1H, H_{8b}) ; 4,71 (d, 1H, J₁₋₂ = 1,2 Hz, H₁) ; 4,87 (m, 2H, H₉) ; 4,92 (m, 2H, H₁₂) ; 8,65 (s, 1H, H₁₀).
**RMN ¹³C (100,62 MHz, D₂O)** δ (ppm) : 23,79 (C₂₈) ; 24,23 (C₂₇) ; 28,06 (C₂₆) ; 36,51 (C₆) ; 50,67 (C₉) ; 50,90 (C₂₉) ; 62,55 (C₁₂) ; 65,29 (C₈) ; 67,88, 69,08, 69,54 et 70,70 (13C, C₁₃₋₂₅) ; 52,32, 69,36, 69,82, 70,16 (4C, C₂₋₅) ; 99,48 (C₁) ; 109,39 (C₁₁) ; 146,74 (C₁₀) ; 175,27 (C₇).

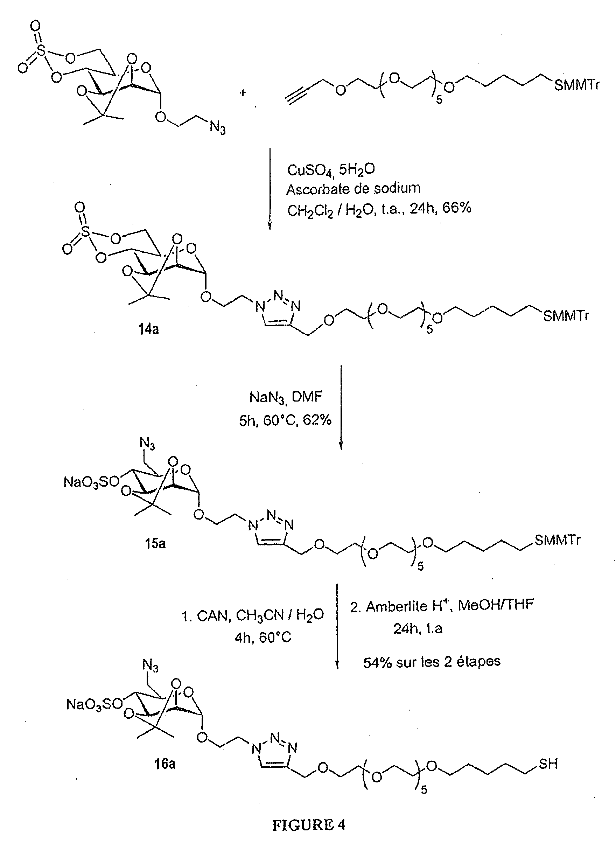

### « CLICK CHEMISTRY » DES COMPOSES 5α ET 9α - FONCTIONNALISATION DU MANNOPYRANOSIDE EN POSITION 6 PAR UN GROUPEMENT AZIDO (cf. Figure 4) :

### 14) SYNTHESE DU {1-(6-AZIDO-6-DEOXY-2,3-O-ISOPROPYLIDENE-4-O-SODIUMSULFATE-α-D-MANNOPYRANOSYL)ETHYL-1H-1,2,3-TRIAZOL-4-YL}METHYL-[O-(1-THIOPENT-5-YL)-O-HEXA(ETHYLENEGLYCOL)] (Composé 14a)

530 mg de {1-[2,3-*O*-isopropylidène-4,6-*O*-(cyclosulfate)-α-D-mannopyranosyl]éthyl-1*H-*1,2,3-triazol-4-yl}méthyl-[*O*-(1-thiométhoxytritylpent-5-yl)-*O*-hexa(éthylèneglycol)] (0,51 mmol, 1 éq.) et 65 mg d'azide de sodium (1,00 mmol, 2 éq.) sont mis à réagir dans 10 mL de DMF. Le même protocole que pour le 2'-azidoéthyl-2,3,4,6-tétra-*O*-acétyl-α-D-mannopyranose (composé 2a) est ensuite suivi (Exemple 1). On obtient alors une huile jaune (350 mg, 62%).
**Rf :** 0,15 (CH₂Cl₂/MeOH 8,5/1,5 v/v).

### 15) SYNTHESE DU {1-(6-AZIDO-6-DEOXY-2,3-α-D-MANNOPYRANOSYL)ETHYL-1H-1,2,3-TRIAZOL-4-YL}METHYL-[O-(1-THIOPENT-5-YL)-O-HEXA(ETHYLENEGLYCOL)] (Composé 15a)

200 mg de {1-(6-azido-6-déoxy-2,3-*O*-isopropylidène-4-*O*-sodiumsulfate-α-D-mannopyranosyl)éthyl-1*H-*1,2,3-triazol-4-yl}méthyl-[*O*-(1-thiopent-5-yl)-*O-*hexa(éthylèneglycol)] (0,18 mmol, 1 éq.) sont mis à réagir avec 50 mg de nitrate d'ammonium cérique (CAN) (0,09 mmol, 0,5 éq.) dans 4 mL d'un mélange CH₃CN/H₂O (95/5 v/v). Après 4 heures d'agitation à 60°C, le mélange réactionnel est dilué dans du CH₂Cl₂, lavé plusieurs fois à l'eau, et la phase aqueuse lyophilisée. L'huile jaune obtenue est purifiée par chromatographie sur colonne de gel de silice avec un gradient d'éluant (CH₂Cl₂/MeOH 90/10 v/v à CH₂Cl₂/MeOH 80/20 v/v) pour donner le {1-(6-azido-6-déoxy-4-*O*-sodiumsulfate-α-D-mannopyranosyl)éthyl-1*H*-1,2,3-triazol-4-yl}méthyl-[*O*-(1-thiopent-5-yl)-*O*-hexa(éthylèneglycol)] sous forme d'une huile incolore (100 mg, 72%).

Ce composé est ensuite mis à réagir avec des résines Amberlyst H⁺ dans 6 mL d'un mélange MeOH/THF (1/1 v/v) pendant 24 heures. Le même protocole que pour le {1-[acide(6,7-didésoxy-α-D-manno-heptopyranosyl)uronique]éthyl-1*H*-1,2,3-triazol-4-yl}méthyl-[*O*-(1-pent-5-yl)-O-hexa(éthylèneglycol)] (composé **13a)** est ensuite suivi (Exemple 1). On obtient alors une huile jaune (150 mg, 53%).
**Rf :** 0,25 (CH₂Cl₂/MeOH 9/1 v/v).
**RMN ¹H (400,13 MHz, CD₃OD)** δ (ppm) : 1,47 (m, 2H, H₂₆) ; 1,60 (m, 2H, H₂₅) ; 1,71 (m, 2H, H₂₇) ; 2,70 (t, 2H, J₂₈₋₂₇ = 7,2 Hz, H₂₈) ; 3,48 (t, 2H, J₂₄₋₂₅ = 6,2 Hz, H₂₄) ; 3,19-3,78 (m, 30H, H₂₋₆ et H₁₂₋₂₃) ; 3,88 (m, 1H, H₇ₐ) ; 4,13 (m, 1H, H_{7b}) ; 4,63 (m, 4H, H₈ et H₁₁) ; 4,72 (s, 1H, H₁) ; 8,03 (s, 1H, H₉).
**RMN ¹³C (100,62 MHz, CD₃OD)** δ (ppm) : 26,13 (C₂₆) ; 30,07 (C₂₇) ; 30,36 (C₂₅) ; 39,66 (C₂₈) ; 51,34 (C₈) ; 62,85 (C₆) ; 65,05 (C₁₁) ; 66,79 (C₇) ; 68,38, 70,81, 71,24, 71,59, 71,93, 72,15, 72,51 et 75,01 (17C, C₂₋₅ et C₁₂₋₂₄) ; 101,70 (C₁) ; 132,57 (C₉) ; 161,04 (C₁₀).

### SYNTHESE DU COMPOSE DE FORMULE (I), DANS LEQUEL A EST UNE NANOPARTICULE D'OR :

Deux solutions, l'une contenant 60 mg d'acide tétrachloroaurique (HAuCl₄) (0,18 mmol, 1 éq.) et 250 mL d'eau, et l'autre contenant 150 mg de citrate de sodium (0,5 mmol, 2,78 éq.) dissous dans 10 mL d'eau sont chauffées en parallèle à une température de 60°C pendant 10 minutes. La solution de citrate de sodium chaude est ensuite ajoutée à la solution d'HAuCl₄, puis le mélange est porté à une température de 120°C pendant 2,5 heures. La solution passe progressivement du gris au rouge-bordeaux, ce qui indique que les nanoparticules d'or se sont formées. Une fois la solution revenue à température ambiante, 50 mg de glycoconjugué solubilisé dans 1mL de MeOH sont ajoutés et laissés sous agitation à température ambiante pendant 48 heures. Les nanoparticules d'or sont ensuite précipitées par ajout d'une solution de NaCl saturée. Après une nuit au repos, le surnageant est enlevé et les nanoparticules d'or sont centrifugées pendant 30 minutes à une vitesse de 14 000 rpm. Elles sont ensuite lavées plusieurs fois à l'eau, puis au méthanol et séchées à l'air libre.
Taille des nanoparticules d'or A : 6-7 nm,
Taille des composés de formule (I) : 7-8 nm.

### EXEMPLE 2 : PREPARATION D'UN COMPOSE DE FORMULE (II)

### 1) SYNTHESE DU 2,3,4,6-TETRA-O-ACETYL-α-D-MANNOPYRANOSIDE DE 3,6,8-DIOXAOCTYLE (Composé 1b)

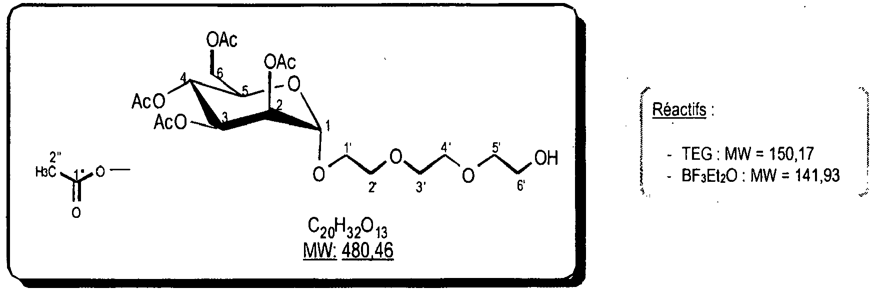

A une solution contenant 25,7 g (0,07 mol - 1 éq.) de α-D-mannose penta-acétate et 26 mL (0,21 mol - 3 éq.) de triéthylène glycol dans 150 mL de dichlorométhane anhydre refroidi à une température de 0°C, on ajoute sous atmosphère d'argon 60 mL (0,56 mol - 8 éq.) de BF₃Et₂O goutte à goutte. On laisse ensuite le mélange réactionnel revenir à température ambiante. Le mélange est maintenu sous agitation pendant toute la nuit, et la réaction est suivie par CCM (Et₂O/MeOH 90/10 v/v). Une fois la réaction terminée, le mélange réactionnel est lavé successivement avec 2 fois 100 mL d'une solution aqueuse saturée de NaHCO₃, avec 2 fois 100 mL d'eau distillée et enfin avec 100 mL d'une solution de saumure. La phase organique est séchée sur du Na₂SO₄, filtrée et évaporée sous pression réduite. Le brut réactionnel est ensuite purifiée par chromatographie sur colonne de gel de silice avec comme éluant (EP/Et₂O 60/40 v/v).
Aspect physique : Huile jaune.
Rendement : 79%.
**Rf :** 0,43 (Et₂O/MeOH 90/10 v/v).
**SM : (ESI⁺/MeOH)** m/z : 503,1 [M+Na]⁺,
**(ESI⁻/MeOH)** m/z : 479,3 [M-H]⁻.
**RMN ¹H (400,13 MHz, CDCl₃)** δ **(ppm)** : 2,05, 2,09, 2,12, 2,13 (4s, 12H, H_{2''}) ; 3,55-3,68 (m, 12H, H_{1'}, H_{2'}, H_{3'}, H_{4'}, H_{5'}, H_{6'}); 4,05-4,11 (m, 1H, H₆ₐ) ; 4,17-4,24 (m, 2H, H₅ et H_{6b}) ; 5,18 (d, 1H, ³J_{H1-H2} = 1,8 Hz, H₁) ; 5,20 (dd, 1H, ³J_{H2-H1} = 1,9 Hz, ³J_{H2-H3} = 3,2 Hz, H₂) ; 5,25 (t, 1H, ³J_{H4-H3} = ³J_{H4-H5} = 9,9 Hz, H₄) ; 5,36 (dd, 1H, ³J_{H3-H2} = 3,3 Hz, ³J_{H3-H4} = 10,1 Hz, H₃).
**RMN ¹³C (100,62 MHz, CDCl₃)** δ **(ppm)** : 20,5 (2C), 20,6 , 20,7 (4C, C_{2''}) ; 61,03 (1C, C_{6'}) ; 62,5 (1C, C₆) ; 66,0 (1C, C₄) ; 66,53 (1C, C_{1'}) ; 68,1 (1C, C₅) ; 68,8 (1C, C₃) ; 69,94 (2C, C_{3'} et C_{4'}) ; 70,1 (1C, C₂) ; 70,44 (1C, C_{2'}) ; 72,60 (1C, C_{5'}) ; 91,8 (1C, C₁) ; 169,8, 170,1, 170,2 et 170,9 (4C, C_{1''}).

### 2) SYNTHESE DU BI(2,3,4,6-TETRA-O-ACETYL-α-D-MANNOPYRANOSIDE) DE 3,6,8-DIOXAOCTYLE (Composé 2b)

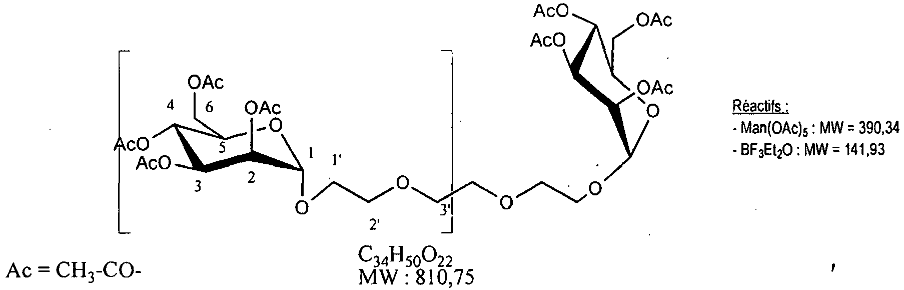

A une solution contenant 13,4 g (27,9 mmol - 1 éq.) du composé **1b** et 10 g (27,9 mmol - 1 éq.) de α-D-mannose penta-acétate dans 120 mL de dichlorométhane anhydre refroidi à une température de 0°C, on ajoute sous atmosphère d'argon 35,5 mL (0,28 mol - 10 éq.) de BF₃Et₂O goutte à goutte. On laisse ensuite le mélange réactionnel revenir à température ambiante. Le mélange est maintenu sous agitation pendant toute la nuit, et la réaction est suivie par CCM (Et₂O/MeOH 90/10 v/v). Une fois la réaction terminée, le mélange réactionnel est lavé successivement avec 2 fois 80 mL d'une solution aqueuse saturée de NaHCO₃, avec 2 fois 80 mL d'eau distillée et enfin avec 80 mL d'une solution de saumure. La phase organique est séchée sur du Na₂SO₄, filtrée et évaporée sous pression réduite. Le brut réactionnel est par la suite purifié par chromatographie sur colonne de gel de silice avec comme éluant (EP/Et₂O 60/40 v/v).
Aspect physique : Mousse blanche.
Rendement : 87%.
**Rf :** 0,61 (Et₂O).
**SM : (ESI⁺/MeOH)** m/z : 833,4 [M+Na]⁺ ; 811,2 [M+H]⁺,
**(ESI⁻/MeOH)** m/z : 809,3 [M-H]⁻ ; 845,4 [M+Cl]⁻.
**RMN ¹H (400,13 MHz, CDCl₃) δ (ppm)** : 1,98, 2,00, 2,11, 2,13 (4s, 24H, H_{2''}) ; 3,42-3,46 (m, 6H, H₃, H₅, H_{6b}) ; 3,51 (dd, 2H, ³J_{H6a-H5} = 7,1 Hz, ²J_{H6a-H6b} = 2,0 Hz, H₆ₐ) ; 3,53 (dd, 2H, ³J_{H4-H3} = 8,5 Hz, ³J_{H4-H5} = 2,7 Hz, H₄) ; 3,58 (s, 4H, H_{3'}) ; 3,60 (t, 4H, ³J_{H2'-H1'} = 3,4 Hz, H_{2'}) ; 3,63 (dd, 2H, ³J_{H2-H3} = 9,2 Hz, ³J_{H2-H1} = 1,8 Hz, H₂) ; 3,67 (t, 4H, ³J_{H1'-H2'} = 3.3 Hz, H_{1'}) ; 5,40 (dd, 2H, ³J_{H1-H2} = 1,5 Hz, H₁).
**RMN ¹³C (100,62 MHz, CDCl₃) δ (ppm)** : 20,6, 20,7, 20,75, 20,9 (8C, C_{2''}) ; 62,26 (2C, C₆) ; 66,53 (2C, C_{1'}) ; 69,94 (2C, C_{3'}) ; 70,44 (2C, C_{2'}) ; 70,46 (2C, C₄) ; 73,34 (2C, C₂) ; 74,80 (2C, C₃) ; 76,66 (2C, C₅) ; 103,79 (2C, C₁).

### 3) SYNTHESE DU BI(α-D-MANNOPYRANOSIDE) DE 3,6,8-DIOXAOCTYLE (Composé 3b)

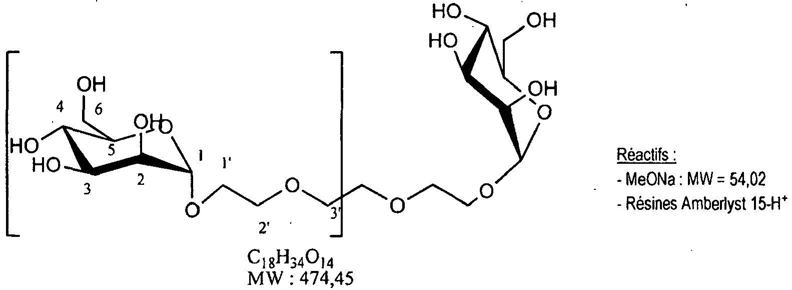

2,20 g (4 mmol - 0,3 éq.) de MeONa sont ajoutés à une solution de 100 mL de méthanol contenant 11 g (13 mmol - 1 éq.) du composé 2b. La réaction est suivie par CCM (iPrOH/NH₄OH 60/40 v/v). Après 30 minutes, le milieu réactionnel est neutralisé avec des résines acides Amberlyst 15-H⁺. Ensuite, les résines sont filtrées, rincées avec du méthanol. Le brut obtenu après concentration est purifié par chromatographie sur colonne de gel de silice avec un gradient d'élution (*i*PrOH/NH₄OH 80/20 v/v jusqu'à *i*PrOH/NH₄OH 60/40 v/v).
Aspect physique : Mousse beige clair.
Rendement : 97%.
Rf : 0,35 (*i*PrOH/NH₄OH 60/40 v/v).
**SM (ESI⁺/MeOH) m/z :** 497,3 [M+Na]⁺ ; 475,4 [M+H]⁺,
**(ESI⁻/MeOH) m/z :** 473,3 [M-H]⁻.
**RMN ¹H (400,13 MHz, MeOD) δ (ppm) :** 3,41-3,46 (m, 6H, H₃, H₅, H_{6b}) ; 3,53 (dd, 2H, ³J_{H6a-H5} = 7,5 Hz, ²J_{H6a-H6b} = 2,1 Hz, H₆ₐ) ; 3,54 (dd, 2H, ³J_{H4-H3} = 8,5 Hz, ³J_{H4-H5} = 3,1 Hz, H₄) ; 3,57 (m, 4H, H_{3'}) ; 3,60 (t, 4H, ³J_{H2'-H1'} = 3,3 Hz, H_{2'}) ; 3,64 (dd, 2H, ³J_{H2-H3} = 8,7 Hz, ³J_{H2-H1} = 1,5 Hz, H₂) ; 3,66 (t, 4H, ³J_{H1'-H2'} = 3,3 Hz, H_{1'}) ; 5,38 (dd, 2H, ³J*_{H1-H2}* = 1,9 Hz, H₁),
**RMN ¹³C (100,62 MHz, MeOD) δ (ppm) :** 63,08 (2C, C₆) ; 66,64 (2C, C_{1'}) ; 70,11 (2C, C_{3'}) ; 70,44 (2C, C_{2'}) ; 70,51 (2C, C₄) ; 73,35 (2C, C₂) ; 75,20 (2C, C₃) ; 76,83 (2C, C₅) ; 104,19 (2C, C₁).

### 4) SYNTHESE DU BI(6-DESOXY-6-IODO-α-D-MANNOPYRANOSIDE) DE 3,6,8-DIOXAOCTYLE (Composé 4b)

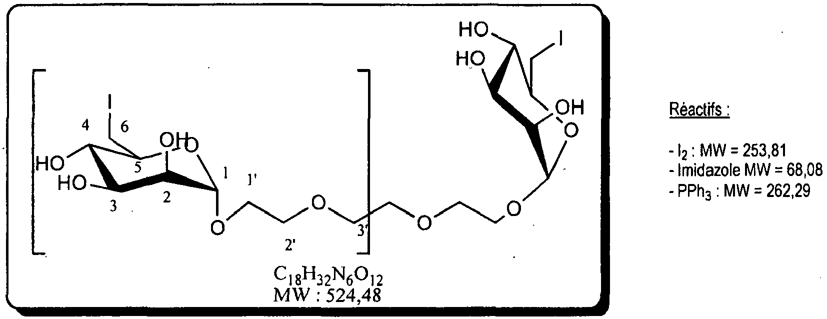

0,4 g (1,58 mmol - 1,5 éq.) d'iode, 0,5 g (1,05 mmol - 1 éq.) du composé 3b, 0,42 g (1,58 mmol - 1,5 éq.) de triphénylphosphine (PPh₃) et 145 mg (2,1 mmol - 2 éq.) d'imidazole sont broyés ensemble dans un petit ballon à l'aide d'une baguette en verre. Le mélange réactionnel est chauffé à une température de 100°C sous agitation pendant 10 minutes. La réaction d'iodation est suivie par CCM (CH₂Cl₂/MeOH 90/10 v/v). Le mélange est ensuite refroidi à température ambiante puis solubilisé dans du méthanol. Le brut réactionnel est ensuite concentré sous pression réduite, et purifié par chromatographie sur colonne de gel de silice (CH₂Cl₂/MeOH 90/10 v/v).

### 5) SYNTHESE DU BI(6-DESOXY-6-AZIDO-α-D-MANNOPYRANOSIDE) DE 3,6,8-DIOXAOCTYLE (Composé 5b)

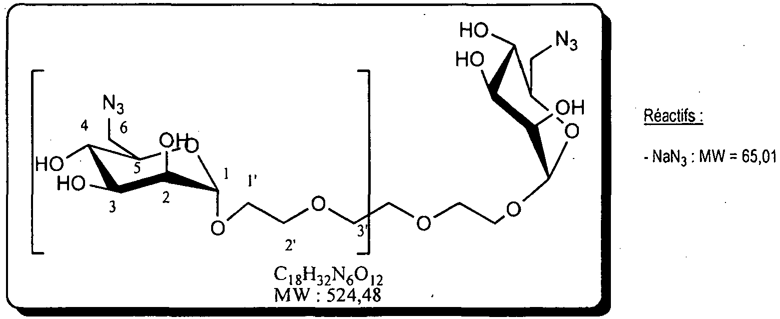

140 mg du composé 4b (0,14 mmol, 1 éq.) et 38 mg d'azide de sodium (0,57 mmol, 4 éq.) sont mélangés et broyés au mortier pendant 5 minutes, le mélange est ensuite plongé dans un bain d'huile à une température de 80°C. Après 20 minutes d'agitation manuelle, le mélange réactionnel est directement purifié par chromatographie sur colonne de gel de silice (CH₂Cl₂/MeOH 9/1 v/v) pour donner une huile jaune (53 mg, 70 %).

Le schéma de synthèse du composé de formule (II) est représenté à la Figure 5 annexée.

### EXEMPLE 3 : PREPARATION D'UN COMPOSE DE FORMULE (III)

### 1) SYNTHESE DU 6-MONOMETHOXYTRITYL-α-D-MANNOPYRANOSIDE DE METHYLE (Composé 1e)

10 g d'α-D-mannopyranoside de méthyle (51,5 mmol, 1 éq.) et 1,9 g de DMAP (15,45 mmol, 0,3 éq.) sont dissous dans 80 mL de pyridine. 24 g de chlorure de monométhoxytrityle (77,25 mmol, 1,5 éq.) sont ajoutés au mélange par fraction. La réaction est terminée après 1,5 heures, le milieu réactionnel est ensuite dilué dans de l'AcOEt et lavé successivement avec une solution de HCl à 2N, une solution de NaHCO₃ 5% et de l'eau. La phase organique est séchée sur du Na₂SO₄, filtrée et concentrée. Le produit est ensuite purifié par chromatographie flash sur gel de silice avec un gradient d'élution (CH₂Cl₂ jusqu'à CH₂Cl₂/MeOH 95/5 v/v) pour donner une poudre légèrement jaune (21,6 g, 90%).
**Rf :** 0,43 (CH₂Cl₂/MeOH 95/5 v/v).
**SM (ESI⁺/MeOH)** m/z : 489,45 [M+Na]⁺ ; 955,67 [2M+Na]⁺,
**(ESI⁺/MeOH)** m/z : 465,28 [M-H]⁻.
**RMN ¹H (400,13 MHz, CDCl₃)** δ (ppm) : 1,65-3,00 (3m, 3H, -OH) ; 3,30 (s, 3H, -OCH₃) ; 3,32 (m, 1H, H₆ₐ) ; 3,37 (m, 1H, H_{6b}) ; 3,55-3,80 (m, 4H, H₂, H₃, H₄ et H₅) ; 3,72 (s, 3H, H₁₃) ; 4,65 (d, 1H, J₁₋₂ = 1,4 Hz, H₁) ; 6,70-7,40 (m, 14H, H_{9,10,11}).
**RMN ¹³C (100,62 MHz, CDCl₃)** δ (ppm) : 54,90 (-OCH₃) ; 55,21 (C₁₃) ; 64,98 (C₆) ; 69,91, 69,99, 70,23 et 71,6 (C₂, C₃, C₄ et C₅) ; 87,15 (C₇) ; 100,64 (C₁) ; 113,38, 127,19, 127,85, 128,42, 130,42 (14C, C_{9,10,11}) ; 135,32, 144,17, 144,26 (C₈) ; 158,72 (C₁₂).

### 2) SYNTHESE DU 2,3,4-TRI-O-BENZYL-6-MONOMETHOXYTRITYL-α-D-MANNOPYRANOSIDE DE METHYLE (Composé 2e)

5 g de 6-monométhoxytrityl-α-D-mannopyranoside de méthyle (10,72 mmol, 1 éq.) sont dissous dans 19 mL de bromure de benzyle (160,8 mol, 15 éq.). 15 g de KOH (268 mmol, 20 éq.) sont ensuite ajoutés et le mélange est chauffé à une température de 80°C. Après 1 heure de réaction, le milieu réactionnel est dilué dans du CH₂Cl₂, puis la phase organique est lavée à l'eau distillée, séchée sur du Na₂SO₄, filtrée et concentrée. Le produit est ensuite purifié par chromatographie sur gel de silice avec un gradient d'élution (EP jusqu'à EP/Et₂O 5/5 v/v) pour donner une mousse écru (7,1 g, 90%).
**Rf :** 0,62 (EP/Et₂O 6/4 v/v).
**SM (ESI⁺/MeOH)** m/z : 759,45 [M+Na]⁺.
**RMN ¹H (400,13 MHz, CDCl₃)** δ (ppm) : 3,19 (dd, 1H, J₆ₐ₋₅ = 5,2 Hz, J_{6b-6a} = 9,8 Hz, H₆ₐ) ; 3,28 (s, 3H, -OCH₃) ; 3,43 (dd, 1H, H_{6b-5} =1,7 Hz, J_{6b-6a} = 9,8 Hz, H_{6b}) ; 3,64 (s, 3H, H₁₃) ; 3,69 (m, 1H, H₅) ; 3,73 (dd, 1H, J₂₋₁ = 1,8 Hz, J₂₋₃ = 3,1 Hz, H₂) ; 3,79 (dd, 1H, J₃₋₂ = 3,2 Hz, J₃₋₄ = 9,3 Hz, H₃) ; 3,95 (t, 1H, J₄₋₃ = J₄₋₅ = 9,6 Hz, H₄) ; 4,42-4,69 (m, 4H, Hₐ) ; 4,74 (s, 1H, H₁) ; 6,66-7,49 (m, 29H, H_{9,10,11,c,d,e}).
**RMN ¹³C (100,62 MHz, CDCl₃)** δ (ppm) : 55,00 (-OCH₃) ; 55,22 (C₁₃) ; 63,85 (C₆) ; 71,91 (C₅) ; 75,32 (C₄) ; 75,59 (C₂) ; 80,37 (C₃) ; 72,43, 72,86 et 75,20 (Cₐ) ; 86,01 (C₇) ; 98,87 (C₁) ; 113,18, 128,61, 127,45-127,90, 128,21-128,74, 130,64 (29C, C_{9,10,11,c,d,e}) ; 135,92, 138,48, 138,76, 138,81, 144,72 et 144,90 (6C, C_{b} et C₈) ; 158,52 (C₁₂).

### 3) SYNTHESE DU 2,3,4-TRI-O-BENZYL-α-D-MANNOPYRANOSIDE DE METHYLE (Composé 3e)

7,12 g de 2,3,4-tri-*O*-benzyl-6-monométhoxytrityl-α-D-mannopyranoside de méthyle (9,66 mmol, 1 éq.) sont dissous dans 80 mL d'un mélange CH₃CN/H₂O 95/5 v/v. 530 mg de CAN (0,97 mmol, 0,1 éq.) sont alors ajoutés et le mélange est chauffé à une température de 60°C pendant 30 minutes. Le milieu réactionnel est ensuite dilué avec du CH₂Cl₂, puis la phase organique est lavée avec de l'eau distillée, séchée sur du Na₂SO₄, filtrée et concentrée. Le produit est ensuite purifié par chromatographie flash sur gel de silice avec un gradient d'élution (EP/Et₂O 3/7 v/v) pour donner une huile incolore (4,26 g, 95 %).
**Rf :** 0,5 (Et₂O/EP 4/6 v/v).
**SM (ESI⁺/MeOH)** m/z : 487,12 [M+Na]⁺ ; 951,34 [2M+Na]⁺.
**RMN ¹H (400,13 MHz, CDCl₃)** δ (ppm) : 1,88 (s, 1H, -OH) ; 3,25 (s, 3H, -OCH₃) ; 3,57 (m, 1H, H₅) ; 3,73 (m, 3H, H₆ₐ et H₂) ; 3,80 (dd, 1H, J₆ₐ₋₅ = 3,0 Hz, J_{6a-6b} = 11,8 Hz, H_{6b}) ; 3,85 (dd, 1H, J₃₋₂ = 3,0 Hz, J₃₋₄ = 9,4 Hz, H₃) ; 3,92 (t, 1H, J₄₋₃ = J₄₋₅ = 9,6 Hz, H₄) ; 4,61 (m, 2H, Hₐ) ; 4,65 (m, 3H, H₁ et Hₐ) ; 4,73 (d, 2H, J = 12,4 Hz, Hₐ) ; 4,89 (d, 2H, J = 10,8 Hz, Hₐ) ; 7,21-7,305 (m, 15H, H_{c,d,e}).
**RMN ¹³C (100,62 MHz, CDCl₃)** δ (ppm) : 54,73 (-OCH₃) ; 62,37 (C₆) ; 71,99 (C₅) ; 74,64 (C₂) ; 74,83 (C₄) ; 72,17, 72,90, 75,16 (3Cₐ) ; 80,17 (C₃) ; 99,29 (C₁) ; 127,54-128,35 (C_{c,d,e}) ; 138,21, 138,37, 138,42 (C_{b}).

### 4) SYNTHESE DU 2,3,4-TRI-O-BENZYL-6-DEOXY-6-OXY-α-D-MANNOPYRANOSIDE DE METHYLE (Composé 4e)

500 mg de 2,3,4-tri-*O*-benzyl-α-D-mannopyranoside de méthyle (1,1 mmol, 1 éq.) sont dissous dans 12 mL de CH₂Cl₂ avant d'ajouter 2,5 g de tamis moléculaire 4Å et 464 mg de chlorochromate de pyridinium (2,15 mmol, 2 éq.). Après 1 heure d'agitation à température ambiante, le milieu réactionnel est filtré sur célite puis sur charbon actif, avant d'être purifié par chromatographie sur silice (AcOEt/EP 3/7 v/v) pour donner une huile transparente (250 mg, 50%).
**Rf :** 0,2 (AcOEt/EP 5/5 v/v).
**SM (ESI⁺/MeOH)** m/z : 485,46 [M+Na]⁺.
**RMN ¹H (400,13 MHz, CDCl₃)** δ (ppm) : 3,31 (s, 3H, -OCH₃) ; 3,70 (t, 1H, J₂₋₁ = J₂-₃ = 2,8 Hz, H₂) ; 3,88 (dd, 1H, J₃₋₂ = 3,0 Hz, J₃₋₄ = 7,8 Hz, H₃) ; 3,98 (t, 1H, J₄₋₅ = J₄₋₃ = 8,2 Hz, H₄) ; 4,01 (m, 1H, H₅) ; 4,54-4,76 (m, 6H, Hₐ) ; 4,78 (d, 1H, J₁₋₂ = 2,8 Hz, H₁) ; 7,19-7,29 (m, 15H, H_{c,d,e}) ; 9,66 (s, 1H, H₆).
**RMN ¹³C (100,62 MHz, CDCl₃)** δ (ppm) : 55,51 (-OCH₃) ; 72,27, 72,91 et 74,66 (3Cₐ) ; 74,17 (C₂) ; 74,36 (C₄) ; 76,00 (C₅) ; 79,15 (C₃) ; 99,42 (C₁) ; 127,61-128,41 (C_{c,d,e}) ; 137,73, 138,02 (3C_{b}) ; 197,86 (C₆).

### 5) SYNTHESE DU 2,3,4-TRI-O-BENZYL-6-DEOXY-6-ALLYL-α-D-MANNOPYRANOSIDE DE METHYLE (Composé 5e)

693 mg de bromure de méthyltriphosphonium (1,95 mmol, 1,2 éq.) sont dissous dans 12 mL de THF anhydre avant d'ajouter sous atmosphère d'argon à une température de -5°C, 2 mL de BuLi (4,88 mmol, 3 éq.). L'agitation est maintenue pendant 30 minutes à une température de-5°C. La solution devient jaune puis 751 mg de 2,3,4-tri-*O*-benzyl-6-déoxy-6-oxy-α-D-mannopyranoside de méthyle (1,63 mmol, 1 éq.) préalablement dissous dans 8 mL de THF anhydre sont ajoutés à la solution à une température de -78°C. Après 2 heures d'agitation à cette température et 16 heures d'agitation à température ambiante, le milieu réactionnel est dilué dans de l'Et₂O puis lavé avec une solution de NH₄Cl. La phase organique est ensuite séchée sur du Na₂SO₄, filtrée, concentrée et purifiée sur colonne de silice (AcOEt/EP 2/8 v/v) pour donner une huile beige (300 mg, 60%).
**Rf :** 0,55 (AcOEt/EP 3/7 v/v).
**SM (ESI⁺/MeOH)** m/z : 484,45 [M+Na]⁺.
**RMN ¹H (400,13 MHz, CD₃OD)** δ (ppm) : 3,27 (s, 3H, -OCH₃) ; 3,71 (t, 1H, J₄₋₃ = J₄₋₅ = 9,4 Hz, H₄) ; 3,75 (dd, 1H, J₂₋₁ = 1,8 Hz, J₂₋₃ = 3,0 Hz, H₂) ; 3,84 (dd, 1H, J₃₋₂ = 3,2 Hz, J₃₋₄ = 9,2 Hz, H₃) ; 3,98 (m, 1H, H₅) ; 4,57-4,81 (m, 6H, Hₐ) ; 4,68 (d, 1H, J₁₋₂ = 1,6 Hz, H₁) ; 5,24, 5,27 (2m, 1H, H₇ₐ) ; 5,40, 5,45 (2m, 1H, H_{7b}) ; 5,99 (m, 1H, H₆) ; 4,73 (d, 2H, J = 12,4 Hz, Hₐ) ; 7,21-7,35 (m, 15H, H_{c,d,e}).
**RMN ¹³C (100,62 MHz, CD₃OD)** δ (ppm) : 54,70 (-OCH₃) ; 72,40, 72,80 et 75,11 (3Cₐ) ; 72,83 (C₅) ; 74,80 (C₂) ; 78,73 (C₄) ; 79,82 (C₃) ; 99,12 (C₁) ; 118,12 (C₇) ; 127,49-128,31 (C_{c,d,e}) ; 135,49 (C₆) ; 138,31, 138,48, 138,59 (C_{b}).

### 6) SYNTHESE DU 6-DEOXY-6-ACIDE (2,3,4-TRI-O-BENZYL-α-D-MANNOPYRANOSIDE DE METHYLE) BORONIQUE (Composé 6e)

100 mg de 2,3,4-tri-*O*-benzyl-6-déoxy-6-allyl-α-D-mannopyranoside de méthyle (0,22 mmol, 1 éq.) sont dissous dans 2 mL de pentane avant d'ajouter sous argon à une température de -78°C, 36 µL de tribromure de bore (0,22 mmol, 1 éq.) et 34 µL de triéthylsilane (0,22 mmol, 1 éq.). L'agitation est maintenue pendant 3 heures à cette température, puis pendant 15 minutes à température ambiante. 17 mg de soude (0,44 mmol, 2 éq.) sont ensuite ajoutés au milieu réactionnel, qui est ensuite agité pendant 30 minutes. La solution est diluée dans de l'AcOEt, puis lavée à l'eau. La phase organique est séchée sur du Na₂SO₄, filtrée, concentrée puis purifiée sur colonne de silice (AcOEt/EP 2/8 v/v) pour donner une huile blanche (100 mg, 91%).
**Rf :** 0,34 (AcOEt/EP 5/5 v/v).
**SM (ESI⁺/MeOH)** m/z : 529,87 [M+Na]⁺.
**RMN ¹H (400,13 MHz, CD₃OD)** δ (ppm) : 3,33 (dd, 1H, J₆ₐ₋₅ = 7,6 Hz, J_{6a-6b} = 10,0 Hz, H₆ₐ) ; 3,38 (s, 3H, H_{OCH3}) ; 3,52 (m, 1H, H₅) ; 5,57 (dd, 1H, J_{6b-5} = 2,4 Hz, J_{6b-6a} = 10,0 Hz, H_{6b}) ; 3,78 (t, 1H, J₄₋₃ = J₄₋₅ = 9,2 Hz, H₄) ; 3,80 (m, 1H, H₂) ; 4,61-5,01 (m, 6H, Hₐ) ; 4,76 (d, 1 H, J₁₋₂ = 1,6 Hz, H₁) ; 7,26-7,41 (m, 15H, H_{c,d,e}).
**RMN ¹³C (100,62 MHz, CD₃OD)** δ (ppm) : 7,04 (C₆) ; 55,02 (C_{OCH3}) ; 71,41 (C₅) ; 72,07, 72,70 et 75,37 (3Cₐ) ; 74,58 (C₂) ; 78,58 (C₄) ; 79,90 (C₃) ; 99,03 (C₁) ; 127,60-128,43 (C_{c,d,e}) ; 138,17, 138,22 et 138,26 (3C_{b}).

### 7) SYNTHESE DU 6-DEOXY-6-ACIDE (α-D-MANNOPYRANOSIDE DE METHYLE) BORONIQUE (Composé 7e)

100 mg de boronate (0,42 mmol, 1 éq) sont mis en solution avec des résines Amberlyst 15-H⁺ dans 4 mL d'un mélange MeOH/THF (1/1 v/v) selon le même protocole que pour le {1-[acide(6,7-didésoxy-α-D-manno-heptopyranosyl)uronique]éthyl-1*H*-1,2,3-triazol-4-yl}méthyl-[*O*-(1-pent-5-yl)-*O*-hexa(éthylèneglycol)] (composé **13a**) (Exemple 1). On obtient une huile blanchâtre (0,40 mg, 85%).
**Rf :** 0,52 (IPrOH/NH₄Cl 5/5 v/v).
**SM (ESI⁺/MeOH)** m/z : 259,37 [M+Na]⁺.
**RMN ¹H (400,13 MHz, D₂O)** δ (ppm) : 1,57 (m, 1H, H₇ₐ) ; 1,88 (m, 1H, H_{7b}) ; 2,68 (m, 1H, H₆ₐ) ; 2,78 (m, 1H,H_{6b}) ; 3,25 (s, 3H, H_{-OCH3}) ; 3,36 (t, 1H, J₄₋₅ = J₄₋₃ = 9,6 Hz, H₄) ; 3,45 (td, 1 H, J₅₋₆ₐ = J₅₋₄ = 9,4 Hz, J_{5-6b} = 2,7 Hz, H₅) ; 3,57 (dd, 1 H, J₃₋₄ = 9,4 Hz, J₃₋₂ = 3,5 Hz, H₃) ; 3,79 (dd, 1H, J₂₋₃ = 3,4 Hz, J₂₋₁ = 1,7 Hz, H₂) ; 4,57 (s, 1H, H₁).
**RMN ¹³C (100,62 MHz, D₂O)** δ (ppm) : 32,90 (C₇) ; 37,72 (C₆) ; 55,16 (C_{-OCH3}) ; 70,26 (C₂) ; 70,58 (C₄) ; 70,88 (C₃) ; 71,04 (C₅) ; 101,23 (C₁).

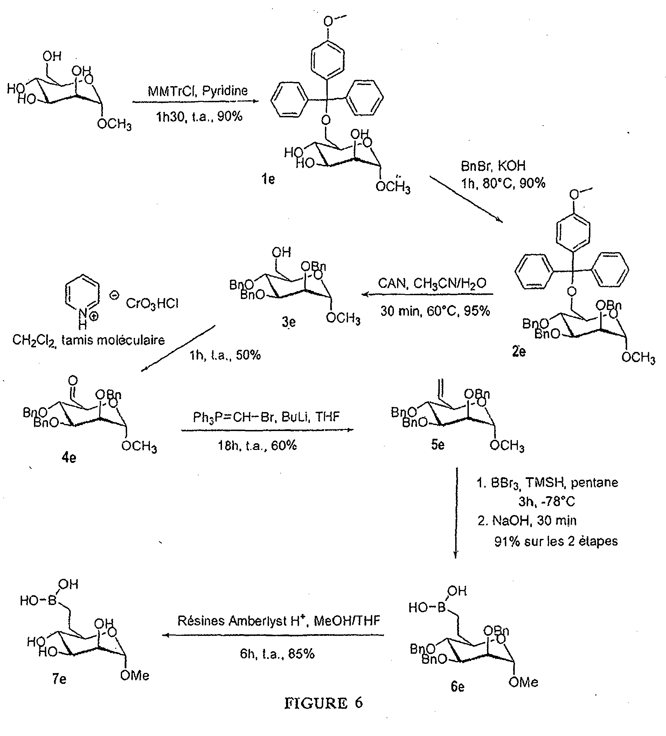

Le schéma de synthèse du composé de formule (III) (composé 7e) est présenté à la Figure 6 annexée.

### EXEMPLE 4 : PREPARATION D'UN COMPOSE DE FORMULE (III)

### 1) SYNTHESE DU 2,3,4-TRI-O-ACETYL-6-MONOMETHOXYTRITYL-α-D-MANNOPYRANOSIDE DE METHYLE (Composé 1f)

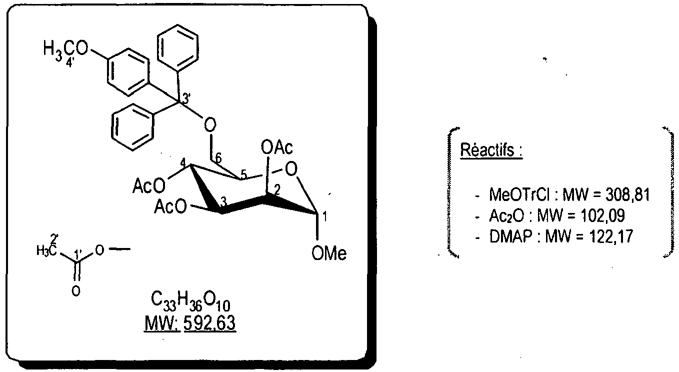

6 g (30,86 mmol - 1 éq.) d'α-D-mannopyranoside de méthyle et 1,13 g (9,26 mmol - 0,3 éq.) de DMAP sont dissous dans 60 mL de pyridine. 14,2 g (46,30 mmol - 1,5 éq.) de chlorure de monométhoxytrityle sont ajoutés au mélange par fraction. La réaction de tritylation dure 1,5 heures, et est suivie par CCM (CH₂Cl₂/MeOH 95/5 v/v). 13,15 mL (137,97 mmol - 4,5 éq.) d'anhydride acétique sont alors ajoutés au milieu réactionnel. L'acétylation est suivie par CCM (Et₂O/EP 7/3 v/v). Après 3 heures de réaction, les sels de pyridinium sont filtrés et le mélange réactionnel est dilué dans 250 mL d'AcOEt. La phase organique est lavée successivement avec une solution d'HCl à 2N (jusqu'à pH = 1), une solution de NaHCO₃ à 5%, et de l'eau distillée, puis séchée sur du Na₂SO₄, filtrée et concentrée. Le produit est ensuite purifié par chromatographie sur gel de silice avec un gradient d'élution (Et₂O/EP 3/7 v/v jusqu'à Et₂O/EP 5/5 v/v) pour donner une mousse blanche.
Aspect physique : Mousse blanche.
Rendement : 90%.
**Rf :** 0,62 (Et₂O/EP 7/3 v/v).
**SM : (ESI⁺/MeOH) m/z :** 615,2 [M+Na]⁺.
**RMN ¹H (400,13 MHz, CDCl₃)** δ **(ppm) :** 1,61, 1,81 et 2,02 (3s, 9H, H_{2'}) ; 3,07 (m, 2H, H₆ₐ et H_{6b}) ; 3,32 (s, 3H, -OC*H*₃) ; 3,62 (s, 3H, H_{4'}) ; 3,76 (m, 1H, H₅) ; 4,62 (d, 1H, ³J_{H1-H2} = 1,7 Hz, H₁) ; 5,09-5,17 (m, 3H, H₂, H₃ et H₄) ; 6,67-7,22 (m, 14H, 14H_{Ph}).

**RMN ¹³C (100,62 MHz, CDCl₃) δ (ppm) :** 21,0, 21,1 et 21,3 (3C, C_{2'}) ; 55,4 (1C,-OCH₃) ; 55,6 (1C, C_{4'}) ; 62,9 (1C, C₆) ; 67,1 (1C, C₄) ; 69,8 (1C, C₃) ; 70,2 (1C, C₂) ; 70,5 (1C, C₅) ; 86,8 (1C, C_{3'}) ; 98,7 (1C, C₁) ; 113,5, 127,3, 128,2, 128,3, 128,9 et 130,8 (14C, CH_{Ph}) ; 135,9, 144,7 et 144,8 (3C, C_{IVPh}) ; 155,0 (1C, C_{IVPh-}OCH₃) ; 169,8, 170,4 et 170,6 (3C, C_{1'}).

### 2) SYNTHESE DU 2,3,4-TRI-O-ACETYL-α-D-MANNOPYRANOSIDE DE METHYLE (Composé 2f)

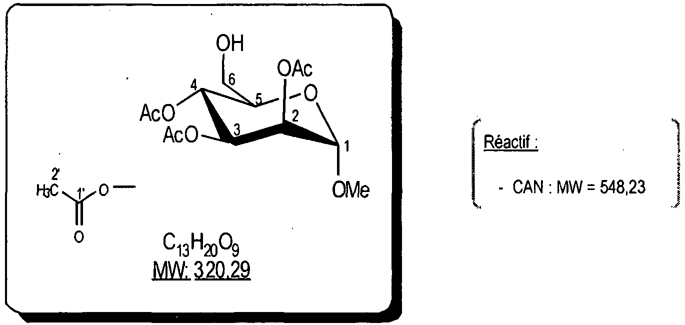

9,16 g (15,45 mmol - 1 éq.) de 2,3,4-tri-*O*-acétyl-6-monométhoxytrityl-α-D-mannopyranoside de méthyle sont dissous dans 150 mL d'un mélange CH₃CN/H₂O (95/5 v/v). 847 mg (1,55 mmol - 0,1 éq.) de CAN sont ajoutés, puis le mélange est chauffé à une température de 60°C. La réaction est suivie par CCM (Et₂O/EP 7/3 v/v) et dure 1 heure. Le milieu réactionnel est ensuite dilué avec du CH₂Cl₂. La phase organique est ensuite lavée 2 fois avec de l'eau distillée, puis séchée sur du Na₂SO₄, filtrée et concentrée. Le produit est ensuite purifié par chromatographie flash sur gel de silice avec un gradient d'élution (CH₂Cl₂ jusqu'à CH₂Cl₂/MeOH 96/4 v/v) pour donner une poudre blanche.
Aspect physique : Poudre blanche.
Rendement : 90%.
**Rf :** 0,67 (CH₂Cl₂/MeOH 95/5v/v).
**SM : (ESI⁺/MeOH)** m/z : 321,4 [M+H]⁺ ; 343,1 [M+Na]⁺; 663,5 [2M+Na]⁺,
**(ESI⁻/MeOH)** m/z : 639,2 [2M-H]⁻.
**RMN ¹H (400,13 MHz, CDCl₃) δ (ppm) :** 1,75, 1,83 et 1,90 (3s, 9H, H_{2'}) ; 2,48 (s, 1H, OH) ; 3,18 (s, 3H, -OC*H*₃) ; 3,41 (dd, 1H, ³J_{H6a-H5} = 4,5 Hz, ²J_{H6a-H6b} = -12,6 Hz, H₆ₐ) ; 3,48 (dd, 1H, ³J_{H6b-H5} = 2,0 Hz, ²J_{H6b-H6a} = -12,5 Hz, H_{6b}) ; 3,54 (ddd, 1H, ³J_{H5-H6a} = 4,5 Hz, ³J_{H5-H6b} = 2,3 Hz, ³J_{H5-H4} = 9,9 Hz, H₅) ; 4,50 (d, 1H, ³J_{H1-H2} = 1,6 Hz, H₁) ; 5,00 (t, 1H, ³J_{H4-H3} = ³J_{H4-H5} = 10,0 Hz, H₄) ; 5,01 (dd, 1H, ³J_{H2-H1} = 1,8 Hz, ³J_{H2-H3} = 3,5 Hz, H₃) ; 5,13 (dd, 1H, ³J_{H3-H2} = 3,4 Hz, ³J_{H3H4} = 10,2 Hz, H₃).
**RMN ¹³C (100,62 MHz, CDCl₃) δ (ppm) :** 20,9, 21,0 et 21,1 (3C, C_{2'}) ; 55,5 (1C,-OCH₃) ; 61,6 (1 C, C₆) ; 66,7 (1C, C₄) ; 69,3 (1 C, C₃) ; 69,2 (1C, C₂) ; 70,9 (1C, C₅) ; 98,1 (1C, C₁) ; 170,2, 170,4 et 171,0 (3C, C_{1'}).

### 3) SYNTHESE DU 6-O-HYDROGENOPHOSPHONATE-α-D-MANNOPYRANOSIDE DE METHYLE (Composé 4f)

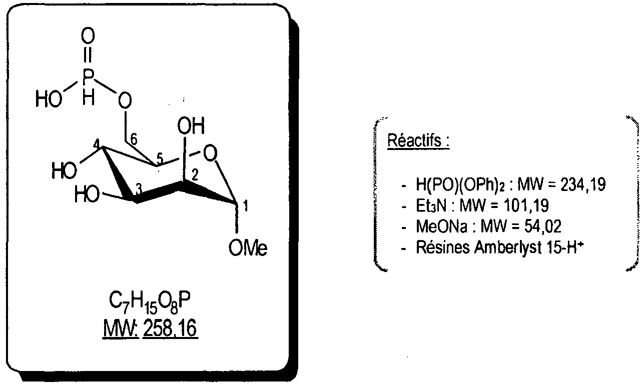

0,5 g (1,56 mmol - 1 éq.) de 2,3,4-tri-*O*-acétyl-α-D-mannopyranoside de méthyle (composé **3f**) sont dissous dans 10 mL de pyridine distillée. 2,1 mL (10,92 mmol - 7 éq.) de phosphite de diphényle sont ajoutés goutte à goutte au mélange à température ambiante. Après 30 minutes d'agitation, 4 mL d'un mélange Et₃N/H₂O (1/1 v/v) sont ajoutés au milieu réactionnel, puis le mélange est maintenu sous agitation pendant 30 minutes supplémentaires. La réaction est suivie par CCM (*i*PrOH/NH₄OH 95/5 v/v). Le mélange réactionnel est ensuite concentré, puis dilué dans 50 mL de CH₂Cl₂. La phase organique est lavée trois fois avec une solution saturée de NaHCO₃, lavée avec de l'eau distillée, puis séchée sur du Na₂SO₄, filtrée et concentrée. Lors de cette étape, la totalité du sucre de départ est consommée. 28 mg (0,46 mmol - 0,3 éq.) de MeONa sont ajoutés au milieu réactionnel, puis dilué à nouveau dans 10 mL de MeOH anhydre. La réaction est suivie par CCM (*i*PrOH/NH₄OH 9/1 v/v) et dure 30 minutes. Le milieu réactionnel est alors neutralisé avec des résines acides Amberlyst 15-H⁺. Les résines sont ensuite filtrées, puis rincées avec du MeOH. Le brut obtenu après concentration est purifié par chromatographie sur colonne de gel de silice avec un gradient d'élution (*i*PrOH jusqu'à *i*PrOH/NH₄OH 9/1 v/v).
Aspect physique : Poudre beige claire.
Rendement : 93%.
**Rf :** 0,24 (*i*PrOH/NH₄OH 9/1 v/v).
**SM (ESI⁺/MeOH) m/z :** 259,2 [M+H]⁺ ; 281,1 [M+Na]⁺ ; 539,2 [2M+Na]⁺,
**(ESI⁻/MeOH) m/z :** 257,4 [M-H]⁻.
**RMN ¹H (400,13 MHz, CD₃OD) δ (ppm) :** 3,23-3,26 (m, 1H, H₆ₐ) ; 3,32 (s, 3H, -OC*H*₃) ; 3,32-3,36 (m, 1H, H5) ; 3,45 (t, 1H, ³J_{H4-H3} = ³J_{H4-H5} = 9,4 Hz, H4) ; 3,5 (dd, 1H, ³J_{H6b-H5} = 2,2 Hz, ²J_{H6b-H6a} = -10,9 Hz, H_{6b}) ; 3,65 (dd, 1H, ³J_{H3-H2} = 3,4 Hz, ³J_{H3-H4} = 9,5 Hz, H3) ; 3,82 (dd, 1H, ³J_{H1-H2} = 1,7 Hz, ³J_{H2-H3} = 3,4 Hz, H2) ; 6,10 (d, 1H, ³J_{H1-H2} = 1,5 Hz, H1).
**RMN ¹³C (100,62 MHz, CD₃OD) δ (ppm) :** 6,3 (1C, C₆) ; 55,0 (1C, -OCH₃) ; 69,8 (1 C, C₂) ; 70,1 (1C, C₃) ; 70,6 (1C, C₄) ; 71,5 (1C, C₅) ; 101,1 (1C, C₁),
**RMN ³¹P (162 MHz, CD₃OD) δ (ppm) :** 9,23 (s, H(PO)OH).

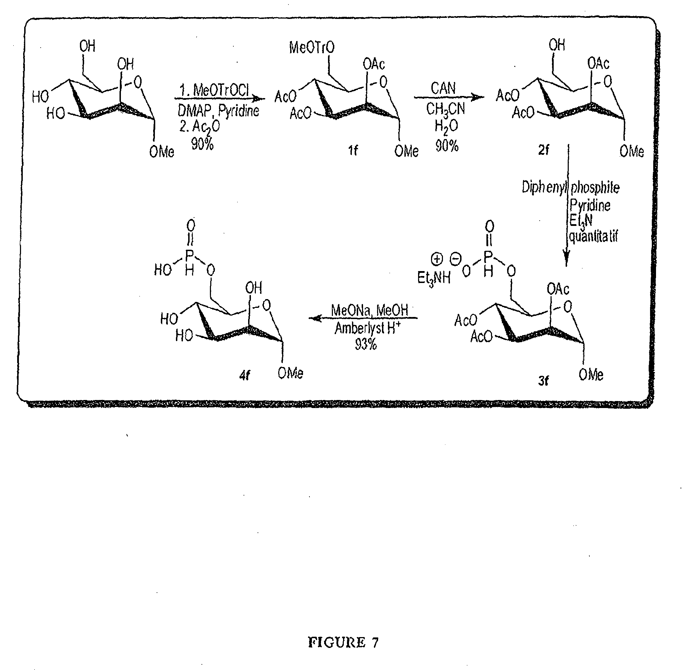

Le schéma de synthèse du composé de formule (III) (composé **4f)** est présenté à la Figure 7 annexée.

### EXEMPLE 5 : PREPARATION D'UN DERIVE PYROPHOSPHONATE DE FORMULE (III)

### 1) SYNTHESE DU 2,3-O-ISOPROPYLIDENE-4,6-O-(CYCLOSULFATE)-α-D-MANNOPYRANOSIDE DE METHYLE (Composé 1g)

3,79 g de 2,3-*O*-isopropylidène-α-D-mannopyranoside de méthyle (16,18 mmol, 1 éq.), 6,75 mL de triéthylamine (48,54 mmol, 3 éq.) et 1,3 mL de chlorure de thionyle (17,80 mmol, 1,1 éq.) sont mis à réagir dans 75 mL de CH₂Cl₂ selon le même protocole que pour le 2'-azidoéthyl-2,3-*O*-isopropylidène-4,6-*O*-(cyclosulfate)-α-D-mannopyranose (composé **5a)** (Exemple 1). Le sulfite brut (16,18 mmol, 1 éq.), 3,8 g de métapériodate de sodium (17,80 mmol, 1,1 éq.), 20 mL d'eau et 14 mg de chlorure de ruthénium (0,06 mmol, 0,004 éq.) sont ensuite mis à réagir dans 60 mL d'une solution CH₂Cl₂/CH₃CN (1/1 v/v) selon le même protocole.
**Rf :** 0,48 (AcOEt/EP 3/7 v/v).
**SM (ESI⁺/MeOH)** m/z : 297,65 [M+H]⁺ ; 319,23 [M+Na]⁺.

### 2) SYNTHESE DU 6,7-DIDEOXY-DIMETHOXYPHOSPHINYL-2,3-O-ISOPROPYLIDENE-4-SODIUMSULFATE-α-D-MANNOPYRANOSIDE DE METHYLE (Composé 2g)

Dans un bicol, 3,5 g de diméthylméthylphosphonate (28,37 mmol, 2 éq.), 3 gouttes de 1,1-diphényléthylène (indicateur coloré) et 10 mL de DMPU (40,53 mmol, 4 éq.) sont dissous dans 20 mL de THF anhydre, sous atmosphère d'argon. Le bicol est plongé dans un bain à une température de -80°C pendant 5 minutes. Un excès de BuLi est ensuite ajouté goutte à goutte jusqu'à obtention d'une coloration rouge persistante. 6 g de cyclosulfate (20,27 mmol, 1 éq.) préalablement dissous dans 40 mL de THF anhydre sont ajoutés goutte à goutte. La solution devient jaune après ajout de quelques gouttes. Le bain est ensuite maintenu à une température de -70/-80 °C pendant 3 heures, puis pendant 14 heures à température ambiante. Le mélange est ensuite dilué dans du CH₂Cl₂, puis le produit est extrait par lavage à l'eau. La phase aqueuse est lavée avec du CH₂Cl₂ avant d'être lyophilisée. L'huile marron obtenue est purifiée par chromatographie sur gel de silice (CH₂Cl₂/MeOH 9/1 v/v) pour donner une huile jaune (1,84 g, 20%).
**Rf :** 0,28 (CH₂Cl₂/MeOH 9/1 v/v).
**SM (ESI⁺/MeOH)** m/z : 465,15 [M+Na]⁺
**(ESI⁻/MeOH)** m/z : 419,18 [M-Na]⁻.
**RMN ¹H (400,13 MHz, CD₃OD)** δ (ppm) : 1,33, 1,51 (2s, 6H, H₁₀) ; 1,89 et 2,17 (2m, 4H, H₆ et H₇) ; 3,35 (s, 3H, -OCH₃) ; 3,62 (m, 1H, H₅) ; 3,74 et 3,77 (2s, 6H, H₈) ; 4,10 (d, 1H, J₂₁ = J₂₋₃ = 5,2 Hz, H₂) ; 4,23 (m, 2H, H₃ et H₄) ; 4,83 (s, 1H, H₁).
**RMN ¹³C (100,62 MHz, CD₃OD)** δ (ppm) : 21,00 et 25,28 (C₆ et C₇) ; 26,30 et 27,90 (C₁₀) ; 53,09 et 53,16 (C₈) ; 55,43 (-OCH₃) ; 68,60 (C₅) ; 76,79 (C₂) ; 77,93 et 78,56 (C₃ et C₄) ; 99,54 (C₁) ; 110,41 (C₉).
**RMN ³¹P (81,02 MHz, CD₃OD)** δ (ppm) : 36,31.

### 3) SYNTHESE DU 6,7-DIDEOXY-PHOSPHINYL-α-D-MANNOPYRANOSIDE DE METHYLE (Composé 3g)

1,8 g de 6,7-didéoxy-diméthoxyphosphinyl-2,3-*O*-isopropylidène-4-sodiumsulfate-α-D-mannopyranoside de méthyle (4,07 mmol, 1 éq.) sont tout d'abord mis à réagir dans 15 mL d'un mélange CH₃CN/H₂O (8/2 v/v) selon le protocole décrit pour le 2'-azidoéthyl-2,3-*O-*isopropylidène-α-D-mannopyranose (composé **4a)** (Exemple 1). 1,1 g du produit obtenu, une huile jaune (3,67 mmol, 1 éq.), sont mis à réagir avec 2,9 mL de pyridine (36,77 mmol, 10 éq.) et 2,42 mL de bromure de triméthylsilane (18,33 mmol, 5 éq.) dans 12 mL de dichlorométhane, sous atmosphère d'argon. Après 8 heures d'agitation à température ambiante, le mélange est concentré et 10 mL de soude à 0,1N sont alors ajoutés. L'agitation est maintenue pendant 30 minutes. Les traces de pyridine sont éliminées par trois extractions à l'Et₂O, puis la phase aqueuse est acidifiée avec une solution de HCl à 1N avant extraction trois fois à l'Et₂O. Les phases organiques sont rassemblées, séchées sur du Na₂SO₄, filtrées et concentrées pour donner une huile incolore (708 mg, 71%).
**Rf :** 0,18 (CH₂Cl₂/MeOH 8/2 v/v).
**SM (ESI⁺/MeOH)** m/z : 295,12 [M+Na]⁺.
**RMN ¹H (400,13 MHz, CD₃OD)** δ (ppm) : 1,94 et 2,17 (2m, 4H, H₆ et H₇) ; 3,36 (s, 3H, -OCH₃) ; 7,20 (m, 1H, H₅) ; 3,87 (m, 2H, J₃₋₂ et H₃) ; 4,32 (t, 1H, J₄₋₃ = J₄₋₅ = 9,2 Hz, H₄) ; 4,64 (s, 1H, H₁).
**RMN ¹³C (100,62 MHz, CD₃OD)** δ (ppm) : 20,93 et 25,03 (C₆ et C₇) ; 55,52 (-OCH₃) ; 70,33 (H₅) ; 71,58 et 71,85 (C₂ et C₃) ; 78,48 (C₄) ; 102,23 (C₁).
**RMN ³¹P (81,02, CD₃OD)** δ (ppm) : 36,66.

### 4) SYNTHESE DU 6,7-DIDEOXY-PHOSPHINYL-2,3,4-O-ACETYL-α-D-MANNOPYRANOSIDE DE METHYLE (Composé 4g)

1,1 g de 6,7-didéoxy-phosphinyl-α-D-mannopyranoside de méthyle (2,74 mmol, 1 éq.) et 2 mL d'anhydride acétique (10,9 mmol, 4 éq.) sont mis à réagir dans 20 mL de pyridine. Après 1 heure d'agitation à température ambiante, la solution est diluée dans du CH₂Cl₂, puis lavée avec une solution de HCl à 1N et de l'eau. La phase organique est séchée sur du Na₂SO₄, filtrée et concentrée pour donner une huile marron (850 mg, 64%).
**Rf :** 0,15 (CH₂Cl₂/MeOH 8/2 v/v).
**SM (ESI⁺/MeOH)** m/z : 421,87 [M+Na]⁺.
**RMN ¹H (400,13 MHz, CD₃OD)** δ (ppm) : 1,90 et 2,21 (2m, 4H, H₆ et H₇) ; 2,01 et 2,11 (2s, 6H, H_{b}) ; 3,41 (s, 3H, -OCH₃) ; 3,62 (m, 1H, H₅) ; 4,41 (t, 1 H, J₄₋₃ = J₄₋₅ = 9,8 Hz, H₄) ; 4,67 (s, 1H, H₁) ; 5,12 (m, 1H, H₂) ; 5,26 (dd, 1H, J₃₋₂ = 3,4 Hz, J₃₋₄ = 9,8 Hz, H₃).
**RMN ¹³C (100,62 MHz, CD₃OD)** δ (ppm) : 19,62 et 25,18 (C₆ et C₇) ; 20,73 et 20,85 (C_{b}) ; 55,66 (-OCH₃) ; 70,63, 71,56, 72,87 (H₂, C₃ et C₅) ; 75,73 (C₄) ; 99,68 (C₁) ; 171,75, 172,26 (Cₐ).
**RMN ³¹P (81,02, CD₃OD)** δ (ppm) : 36,31.

### 5) SYNTHESE DU 6,7-DIDEOXY-PYROPHOSPHINYL-2,3,4-O-ACETYL-α-D-MANNOPYRANOSIDE DE METHYLE (Composé 5g)

700 mg de 6,7-didéoxy-phosphinyl-2,3,4-*O*-acétyl-α-D-mannopyranoside de méthyle (0,15 mmol, 1 éq.) sont dissous dans 8 mL de méthanol avant d'ajouter de la dibutylamine (0,15 mmol, 1 éq.). Le mélange est laissé sous agitation à température ambiante pendant 30 minutes. Le solvant est ensuite évaporé, puis coévaporé avec de la pyridine anhydre pour éliminer toute trace d'eau. Le monosel phosphonique de dibutylamine obtenu est dissous dans 7 mL de THF anhydre, puis du chlorophosphate de diphényle (0,15 mmol, 1 éq.) et de la dibutylamine (4,39 mmol, 3 éq.) sont successivement ajoutés. Le mélange est maintenu sous agitation à température ambiante sous atmosphère d'argon pendant 2 heures.

De la même manière, le monosel de dibutylamine d'orthophosphate est préparé : l'acide orthophosphorique (4,39 mmol, 3 éq.) est dissous dans 8 mL de méthanol, puis de la dibutylamine (4,39 mmol, 3 éq.) est ensuite ajoutée. Après 20 minutes d'agitation à température ambiante, les traces de pyridine sont éliminées par coévaporation à la pyridine anhydre. Le monosel de dibutylammonium d'orthophosphate (4,39 mmol, 3 éq.) est dissous dans 8 mL de pyridine anhydre, puis l'anhydride phosphonique activé est additionné lentement. La solution est maintenue sous agitation à température ambiante sous atmosphère d'argon pendant 15 heures. Les solvants sont ensuite évaporés, l'huile obtenue est purifiée par chromatographie sur gel de silice (IPrOH/NH₄Cl 9/1 v/v) pour donner une huile transparente (470 mg, 50%).
**Rf :** 0,12 (CH₂Cl₂/MeOH 8/2 v/v).
**SM (ESI⁺/MeOH)** m/z : 421,87 [M+Na]⁺.
**RMN ¹H (400,13 MHz, CD₃OD)** δ (ppm) : 1,74 et 2,00 (2m, 4H, H₆ et H₇) ; 2,02 et 2,10 (2s, 6H, H_{b}) ; 3,40 (s, 3H, -OCH₃) ; 3,58 (m, 1H, H₅) ; 4,43 (t, 1H, J₄₋₃ = J₄₋₅ = 9,8 Hz, H₄) ; 4,69 (s, 1H, H₁) ; 5,13 (m, 1H, H₂) ; 5,28 (dd, 1H, J₃₋₂ = 3,4 Hz, J₃₋₄ = 9,8 Hz, H₃).
**RMN ¹³C (100,62 MHz, CD₃OD)** δ (ppm) : 19,56 et 25,01 (C₆ et C₇) ; 20,73 et 20,85 (C_{b}) ; 55,69 (-OCH₃) ; 70,63, 71,58, 72,87 (H₂, C₃ et C₅) ; 75,71 (C₄) ; 99,65 (C₁) ; 171,75, 172,26 (Cₐ).
**RMN ³¹P (81,02, CD₃OD)** δ (ppm) : -9,9 et 8,4.

### 6) SYNTHESE DU 6,7-DIDEOXY-PYROPHOSPHINYL-α-D-MANNOPYRANOSIDE DE METHYLE (Composé 6g)

470 mg de 6,7-didéoxy-phosphinyl-α-D-mannopyranoside de méthyle (0,89 mmol, 1 éq.) sont déprotégés selon le protocole décrit pour le 2'-azidoéthyl-α-D-mannopyranose (composé 3a) (Exemple 1) dans 8 mL de méthanol et en présence de 190 mg de méthanolate de sodium (3,55 mmol, 4 éq.), pour donner une huile blanche (297 mg, 80%).
**Rf :** 0,20 (IPrOH/NH₄Cl 5/5 v/v).
**SM (ESI⁺/MeOH)** m/z : 441,57 [M+Na]⁺.
**RMN ¹H (400,13 MHz, D₂O)** δ (ppm) : 1,97 (m, 1H, H₇ₐ) ; 2,36 (m, 1H, H_{7b}) ; 2,99 (m, 1H, H₆ₐ) ; 3,13 (m, 1H, H_{6b}) ; 3,37 (s, 3H, -OCH₃) ; 3,78 (m, 1H, H₅) ; 3,89-3,96 (m, 2H, H₂ et H₃) ; 4,45 (t, 1H, J₄₋₅ = J₄₋₃ = 9,4 Hz, H₄) ; 4,70 (s, 1H, H₁).
**RMN ¹³C (100,62 MHz, D₂O)** δ (ppm) : 26,81 (C₇) ; 47,60 (C₆) ; 55,43 (-OCH₃) ; 69,16 (C₅) ; 69,94 (C₃) ; 70,44 (C₂) ; 79,03 (C₄) ; 100,99 (C₁).
**RMN ³¹P (81,02, CD₃OD)** δ (ppm) : -9,8 et 8,5.

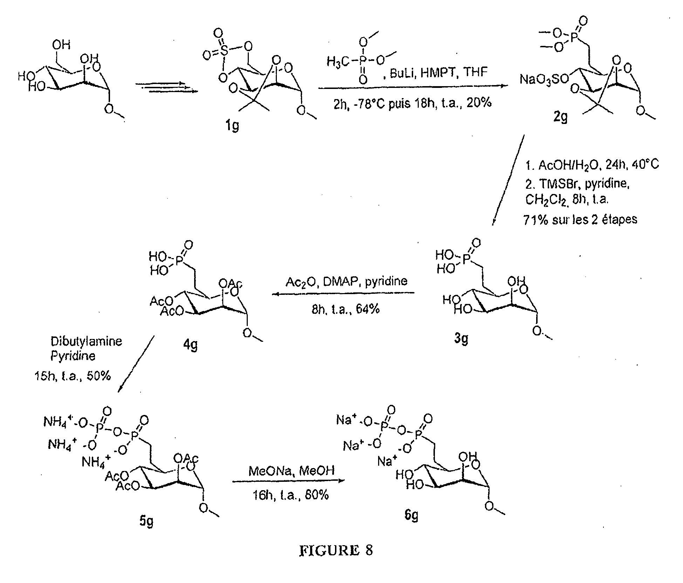

Le schéma de synthèse du dérivé pyrophosphonate de formule (III) (composé 6g) est présenté à la Figure 8 annexée.

### EXEMPLE 6 : PREPARATION D'UN DERIVE PYROPHOSPHATE DE FORMULE (III)

Les composés **1h, 2h** et **3h** sont préparés selon le mode opératoire décrit ci-dessus (cf. synthèse des composés 1e, 2e et 3e de l'exemple 3).

### 1) SYNTHESE DE L'ETHYL (6-DEOXY-6-PHOSPHATE-2,3,4-TRI-O-BENZYL-α-D-MANNOPYRANOSIDE DE METHYLE (Composé 4h)

100 mg de sucre (0,22 mmol, 1 éq.) et 750 mg de diéthyldiéthylphosphoramidite (0,22 mmol, 1 éq.) sont dissous dans 2 mL de THF avant d'ajouter sous atmosphère d'argon 290 µL de 1*H*-tétrazole goutte à goutte. Après 4 heures d'agitation à température ambiante, 600 mg d'acide méta-chloroperbenzoïque (mCPBA) (3,47 mmol, 1,6 éq.) dissous au préalable dans 6 mL de CH₂Cl₂ sont ajoutés au mélange à une température de -78°C. L'agitation est maintenue à cette température pendant 10 minutes, puis à température ambiante pendant 10 minutes. Le mélange réactionnel est dilué avec de l'AcOEt, puis lavé avec une solution de NaHCO₃ saturée. La phase organique est séchée sur du Na₂SO₄, filtrée, concentrée puis purifiée sur colonne de silice (AcOEt/EP 2/8 v/v) pour donner une huile transparente.
**Rf :** 0,45 (CH₂Cl₂/MeOH 9/1 v/v).
**SM (ESI⁺/MeOH)** m/z : 623,13 [M+Na]⁺.
**RMN ¹H (400,13 MHz, CDCl₃)** δ (ppm) : 1,21 (t, 6H, J₈₋₇ = 7,1 Hz, H₈) ; 2,80 (m, 1H, H₆ₐ) ; 2,93 (m, 1H, H_{6b}) ; 3,29 (s, 3H, -OCH₃) ; 3,78 (td, 1H, J₅₋₄ = J₅₋₆ₐ = 9,3 Hz, J_{5-6b} = 2,6 Hz, H₅) ; 4,03 (m, 4H, H₇) ; 4,53 (s, 1H, H₁) ; 4,55-4,64 (m, 6H, Hₐ) ; 5,00 (t, 1H, J₄₋₅ = J₄₋₃ = 9,9 Hz, H₄) ; 5,07 (dd, 1H, J₂₋₃ = 3,3 Hz, J₂₋₁ = 1,7 Hz, H₂) ; 5,15 (dd, 1H, J₃₋₂ = 3,4 Hz, J₃₋₄ = 10,0 Hz, H₃) ; 7,17-7,31 (m, 15H, H_{c,d,e}).
**RMN ¹³C (100,62 MHz, CDCl₃)** δ (ppm) : 16,42 et 16,58 (C₈) ; 32,63 (C₆) ; 55,76 (-OCH₃) ; 64,03 et 64,10 (H₇) ; 69,31 (C₃ et C₄) ; 69,97 (C₂) ; 70,58 (C₅) ; 98,84 (C₁) ; 72,33, 73,01 et 75,34 (Cₐ) ; 127,64-128,56 (C_{c,d,e}) ; 138,30, 138,41 et 138,55 (C_{b}).
**RMN ³¹P (81,02 CDCl₃)** δ (ppm) : 28,0.

### 2) SYNTHESE DU 6-DEOXY-6-PHOSPHATE-2,3,4-TRI-O-BENZYL-α-D-MANNOPYRANOSIDE DE METHYLE (Composé 5h)

Le composé 5h est préparé selon le mode opératoire décrit ci-dessus (cf. Exemple 5, synthèse du 6,7-didéoxy-phosphinyl-2,3,4-*O*-acétyl-α-D-mannopyranoside de méthyle).
**Rf :** 0,23 (CH₂Cl₂/MeOH 9/1 v/v).
**SM (ESI⁺/MeOH)** m/z : 567,34 [M+Na]⁺.
**RMN ¹H (400,13 MHz, CDCl₃)** δ (ppm) : 2,96 (m, 1H, H₆ₐ) ; 3,23 (m, 1H, H_{6b}) ; 3,28 (s, 3H, -OCH₃) ; 3,65 (td, 1H, J₅₋₄ = J₅₋₆ₐ = 9,3 Hz, J_{5-6b} = 2,6 Hz, H₅) ; 4,51 (s, 1H, H₁) ; 4,54-4,64 (m, 6H, Hₐ) ; 5,04 (t, 1H, J₄₋₅ = J₄₋₃ = 9,9 Hz, H₄) ; 5,04 (dd, 1H, J₂₋₃ = 3,3 Hz, J₂₋₁ = 1,7 Hz, H₂) ; 5,17 (dd, 1 H, J₃₋₂ = 3,4 Hz, J₃₋₄ = 10,0 Hz, H₃) ; 7,18-7,32 (m, 15H, H_{c,d,e}).
**RMN ¹³C (100,62 MHz, CDCl₃)** δ (ppm) : 32,61 (C₆) ; 55,78 (-OCH₃) ; 69,33 (C₃ et C₄) ; 69,89 (C₂) ; 70,56 (C₅) ; 98,84 (C₁) ; 72,36, 73,05 et 75,34 (Cₐ) ; 127,64-128,53 (C_{c,d,e}) ; 138,33, 138,48 et 138,65 (C_{b}).
**RMN ³¹P (81,02 CDCl₃)** δ (ppm) : 22,5.

### 3) SYNTHESE DU 6-DEOXY-6-PYROPHOSPHATE-2,3,4-TRI-O-BENZYL-α-D-MANNOPYRANOSIDE DE METHYLE (Composé 6h)

100 mg de 6-déoxy-6-phosphate-2,3,4-tri-*O*-benzyl-α-D-mannopyranoside de méthyle (0,52 mmol, 1 éq.) sont dissous dans 3 mL de THF anhydre, puis 85 µL de pyridine (1,03 mmol, 2 éq.) et 52 µL de POCl₃ (0,57 mmol, 1,1 éq.) sont ajoutés à une température de 0°C sous atmosphère d'argon. L'agitation est maintenue à une température de 0°C pendant 4 heures, quelques mL d'une solution de NaHCO₃ saturée sont ajoutés toujours à une température de 0°C, et le mélange est agité pendant encore 15 minutes. Le milieu réactionnel est ensuite lyophilisé, puis purifié sur colonne de silice (CH₂Cl₂/MeOH 9/1 v/v) pour donner une huile blanchâtre (35 mg, 30%).
**Rf :** 0,12 (CH₂Cl₂/MeOH 8/2 v/v).
**SM (ESI⁺/MeOH)** m/z : 647,78 [M+Na]⁺.
**RMN ¹H (400,13 MHz, CDCl₃)** δ (ppm) : 2,96 (m, 1H, H₆ₐ) ; 3,23 (m, 1H, H_{6b}) ; 3,28 (s, 3H, -OCH₃) ; 3,65 (td, 1H, J₅₋₄ = J₅₋₆ₐ = 9,3 Hz, J_{5-6b} = 2,6 Hz, H₅) ; 4,51 (s, 1H, H₁) ; 4,54-4,64 (m, 6H, Hₐ) ; 5,04 (t, 1H, J₄₋₅ = J₄₋₃ = 9,9 Hz, H₄) ; 5,04 (dd, 1H, J₂₋₃ = 3,3 Hz, J₂₋₁ = 1,7 Hz, H₂) ; 5,17 (dd, 1H, J₃₋₂ = 3,4 Hz, J₃₋₄ = 10,0 Hz, H₃) ; 7,18-7,32 (m, 15H, H_{c,d,e}).
**RMN ¹³C (100,62 MHz, CDCl₃)** δ (ppm) : 32,61 (C₆) ; 55,78 (-OCH₃) ; 69,33 (C₃ et C₄) ; 69,89 (C₂) ; 70,56 (C₅) ; 98,84 (C₁) ; 72,36, 73,05 et 75,34 (Cₐ) ; 127,64-128,53 (C_{c,d,e}) ; 138,33, 138,48 et 138,65 (C_{b}).
**RMN ³¹P (81,02 CDCl₃)** δ (ppm) : -6,78 et 7,34.

### 4) SYNTHESE DU 6-DEOXY-6-PYROPHOSPHATE-α-D-MANNOPYRANOSIDE DE METHYLE (Composé 7h)

Le composé **7h** est préparé selon le mode opératoire décrit ci-dessus (cf. Exemple 5, synthèse du 6,7-didéoxy-pyrophosphinyl-2,3,4-*O*-acétyl-α-D-mannopyranoside de méthyle).
**Rf :** 0,12 (IPrOH/NH₄Cl 7/3 v/v).
**SM (ESI⁺/MeOH)** m/z : 377,91 [M+Na]⁺.
**RMN ¹H (400,13 MHz, CD₃OD)** δ (ppm) : 3,44 (s, 3H, H_{-OCH3}) ; 3,64 (m, 1H, H₅) ; 3,67 (t, 1H, J₄₋₃ = J₄₋₅ = 10,0 Hz, H₄) ; 3,77 (m, 1H, H₆ₐ) ; 3,80 (d, 1H, J₂₋₁ = J₂₋₃ = 5,6 Hz, H₂) ; 3,91 (d, 1H, J₁₋₂ = 1,6 Hz, H₁) ; 3,96 (dd, 1H, J_{6b-5} = 1,8 Hz, J_{6b-6a} = 9,8 Hz, H_{6b}) ; 3,97 (dd, 1H, J₃₋₂ = 1,6 Hz, J₃₋₄ = 3,2 Hz, H₃).
**RMN ¹³C (100,62 MHz, CD₃OD)** δ (ppm) : 58,59 (C₆) ; 64,42 (C₄) ; 67,57 (C₃) ; 68,19 (C₂); 70,19 (C₅) ; 98,51 (C₁).
**RMN ³¹P (81,02 CDCl₃)** δ (ppm) : -6,53 et 8,62.

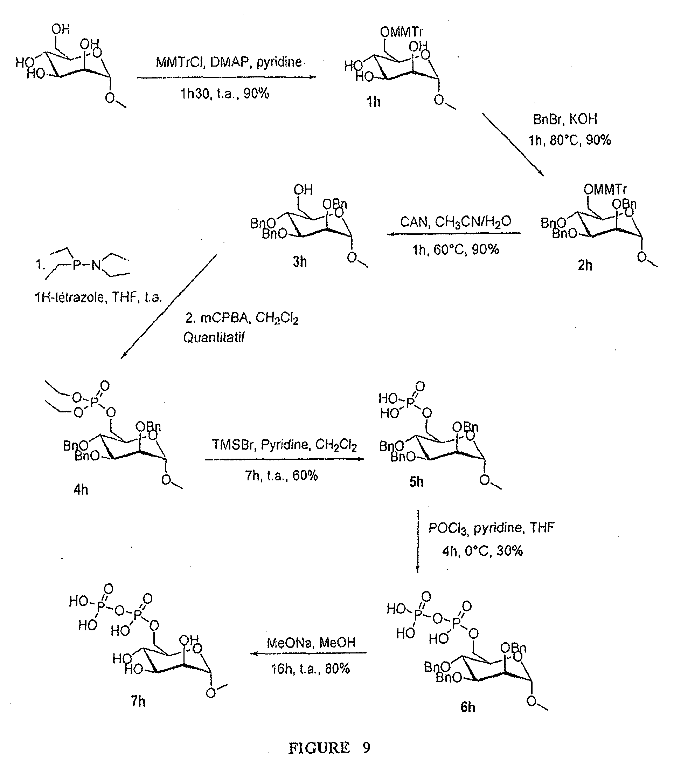

Le schéma de synthèse du dérivé pyrophosphate de formule (III) est présenté à la Figure 9 annexée.

### EXEMPLE 7 : PREPARATION D'UN COMPOSE DE FORMULE (II)

### 1) SYNTHESE DU 6-DESOXY-6-AZIDO-D-MANNOPYRANOSYL-(1,6)-6-DESOXY-6-AZIDO-D-MANNOPYRANOSYL-(1,4)-D-MANNOPYRANOSIDE DE METHYLE

A 2,35 g de mono-2,3-isopropylidène-mannopyranoside de méthyle (0,01 mole, 1 éq.) dissous dans 40 mL de THF anhydre sont ajoutés 12 g de trichloroacétimidate (0,03 mole, 3 éq.). La solution est ensuite refroidie à une température de 0°C, avant que 30 mL (0,28 mole) de BF₃Et₂O soient ajoutés goutte-à-goutte, sous atmosphère d'argon. Le mélange réactionnel est ensuite maintenu à température ambiante et laissé sous agitation pendant toute une nuit. Une fois la réaction terminée, le mélange réactionnel est lavé successivement avec 2 fois 80 mL d'une solution aqueuse saturée de NaHCO₃, puis avec 2 fois 80 mL d'eau distillée. La phase organique est séchée avec du Na₂SO₄, filtrée et évaporée sous pression réduite. Le résidu obtenu est chromatographié sur gel de silice en utilisant un mélange de CH₂Cl₂/MeOH comme éluant. Le produit obtenu est une poudre blanche (5 g, rendement 80%).
**Rf** : 0,5 (CH₂Cl₂/MeOH : 8/2 v/v).
**SM (ESI⁺/MeOH)** m/z : 661,7 [M+Na]⁺ ; 661,7 [M+H]⁺.

3,3 g de trisaccharide (5 mmol, 1 éq.) et 2,1 mL de triéthylamine (15 mmol, 3 éq.) sont dissous dans 25 mL de CH₂Cl₂. Le ballon est placé dans un bain de glace et 3,5 mL de chlorure de thionyle (5,5 mmol, 1,1 éq.) sont ajoutés lentement. Un précipité blanc de chlorure de triéthylammonium apparaît rapidement et le mélange réactionnel devient progressivement jaune. Après 5 minutes d'agitation à une température de 0°C, le produit de départ a disparu, le sulfite désiré est obtenu. Le mélange est filtré, la phase organique est lavée avec de l'eau distillée, une solution de HCl à 1N, puis à nouveau de l'eau. Elle est ensuite séchée sur du Na₂SO₄, filtrée et concentrée pour donner un solide brun directement remis en réaction.

### Formation du sulfate :

Le sulfite brut (5 mmol, 1 éq.,) est dissous dans 20 mL d'un mélange CH₂Cl₂/CH₃CN (1/1 v/v). 1,17 g de métapériodate de sodium (5,5 mmol, 1,1 éq.), 5 mL d'eau et trois grain de chlorure de ruthénium sont ajoutés successivement. La réaction est exothermique, un précipité de NaIO₃ se forme très rapidement. Après 1 heure d'agitation à température ambiante, le sulfite a été consommé, le mélange réactionnel est filtré et dilué dans 200 mL de CH₂Cl₂. La phase organique est lavée avec une solution de NaHCO₃ à 5%, de l'eau distillée, puis séchée, filtrée et concentrée. Le solide obtenu est dissous dans un minimum de CH₂Cl₂ et filtré sur silice. La silice est rincée plusieurs fois avec du CH₂Cl₂. Un solide blanc est obtenu (2,8 g, 70%).

### Formation du di-azido protégé :

823 mg de sulfate cyclique (0,15 mmol, 1 éq.) et 200 mg d'azide de sodium (0,31 mmol, 2 éq.) sont dissous dans 10 mL de DMF. Après 4 heures d'agitation à température ambiante, le mélange réactionnel est dilué dans 50 mL de NaHCO₃ à 5% et repris avec 100 mL de CH₂Cl₂, lavé à l'eau, séché sur du Na₂SO₄, puis évaporé. La poudre obtenue est dissoute dans 10 mL de méthanol, puis traitée avec 2 mL de résine Amberlite H⁺. Après évaporation du solvant, 800 mg de poudre jaune sont isolés et chromatographiés sur gel de silice avec un gradient d'élution (CH₂Cl₂ jusqu'à CH₂Cl₂/MeOH 6/4 v/v) pour donner une poudre blanche (rendement de 80%).
Rf : 0,67 (CH₂Cl₂/MeOH 6/4 v/v).
SM **(ESI⁺/MeOH) m/z :** 688,7 [M+H]⁺ ; 710,7 [M+Na]⁺.
**(ESI⁻/MeOH) m/z :** 687,7 [M-H]⁻.

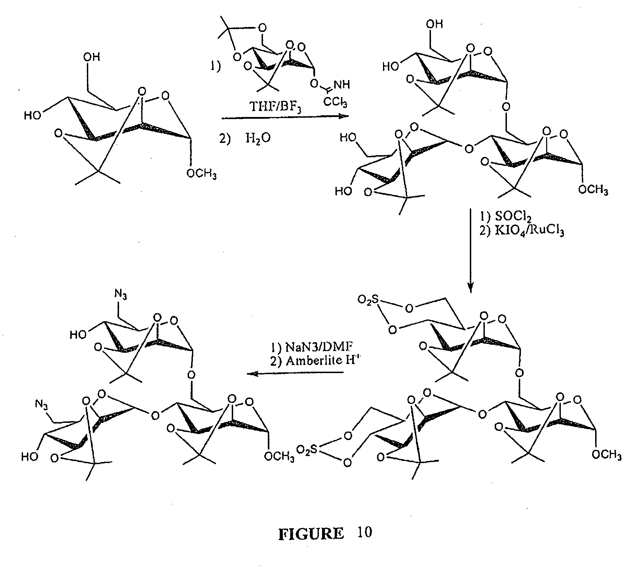

Le schéma de synthèse du composé de formule (II) exemplifié est présenté à la Figure 10 annexée.

### EXEMPLE 8 : PREPARATION D'UN COMPOSE DE FORMULE (III)

### 1) SYNTHESE DU 6-DEOXY-6-IODO-α-D-MANNOPYRONOSIDE DE METHYLE (Composé 1i)

Une solution de 25 mL de THF anhydre contenant 4,90 g (19,3 mmol, 1,5 éq.) de diiode est ajoutée, goutte à goutte, sous azote, à une solution de THF anhydre à reflux contenant 100 mL de THF anhydre contenant 2,5 g (12,8 mmol - 1éq.) de α-D-mannopyranoside de méthyle, 5 g (19,3 mmol - 1,5 éq.) de P_{ϕ3} et 1,75 g (25,7 mmol - 2 éq.) d'imidazole. Après 3 heures de réaction à reflux, le mélange est refroidi à température ambiante, les sels d'imidazole sont filtrés et après concentration du filtrat, le brut réactionnel est directement purifié par chromatographie sur colonne de gel de silice (CH₂Cl₂/MeOH 9/1 v/v). Le produit est ensuite recristallisé dans Et₂O pour donner des cristaux blancs (85%).
**Rf:** 0,25 (CH₂Cl₂/MeOH 9/1 v/v).
**Pf :** 118-120°C.
**SM (ESI⁺/CH₃CN-H₂O-CF₃CO₃H)** m/z : 305,0 [M+H]⁺, 327,0 [M+Na]⁺, 609,0 [2M+H]⁺,
**(ESI⁻/CH₃CN-H₂O-CF₃CO₃H)** m/z : 339,2 [M+Cl]⁺.
**RMN ¹H (400,13 MHz, CDCl₃)** δ (ppm) : 3,26 (dd, 1H, J₆ₐ₋₅ = 7,0 Hz, J_{6a-6b} = 10,9 Hz, H₆ₐ) ; 3,32 (s, 3H, -OCH₃) ; 3,32-3,36 (m, 1H, H₅) ; 3,45 (t, 1H, J₄₋₃ = J₄₋₅ = 9,4 Hz, H₄) ; 3,5 (dd, 1H, J_{6b-5} = 2,2 Hz, J_{6b-6a} = 10,9 Hz, H_{6b}); 3,65 (dd, 1H, J₃₋₂ = 3,4 Hz, J₃₋₄ = 9,5 Hz, H₃) ; 3,82 (dd, 1H, J₁₋₂ = 1,7 Hz, J₂₋₃ = 3,4 Hz, H₂); 6,10 (d, 1H, J₁₋₂ = 1,5 Hz, H₁).
**RMN ¹³C (100,62 MHz, CDCl₃)** δ (ppm) : 6,31 (C₆) ; 55,08 (-OCH₃) ; 69,86 (C₂) ; 70,14 (C₃) ; 70,66 (C₄) ; 71,59 (C₅); 101,10 (C₁).
**[α]_{D} :** + 67,5 (c = 1,00 g/100 mL, MeOH).

### 2) SYNTHESE DU 6-DESOXY-6-IODO-2,3,4-TRI-O-BENZYL-α-D-MANNOPYRANOSIDE DE METHYLE (Composé 2i)

4,37 g de 6-déoxy-6-iodo-α-D-mannopyranoside de méthyle (14,34 mmol, 1 éq.) sont dissous dans 100 mL de DMF anhydre avant d'ajouter 8,5 mL de bromure de benzyle (71,72 mmol, 5 éq.). 1,7 g de NaH (71,72 mmol, 5 éq.) sont ensuite ajoutés par petites fractions pendant 1 heure. Après 4 heures de réaction, 5 mL de MeOH sont ajoutés et le mélange est dilué dans de l'éther Et₂O avant lavage à l'eau. La phase organique est relavée plusieurs fois à l'eau, séchée puis concentrée. Une purification par chromatographie sur colonne de silice (AcOEt/EP 4/6 v/v) permet d'obtenir le produit sous forme d'une huile jaune (3,57 g, 44%).
**Rf :** 0,42 (EP/AcOEt 8/2 v/v).
**SM (ESI ESI⁺/CH₃CN-H₂O-CF₃CO₃H)** m/z: 543,1 [M-OCH3]⁺, 592,2 [M+NH₄]⁺, 597,1 [M+Na]⁺.
**RMN ¹H (400,13 MHz, CDCl₃)** δ (ppm) : 3,31-3,43 (m, 1H, H₆ₐ) ; 3,38 (s, 3H, -OCH₃) ; 3,47-3,59 (m, 2H, H₅ et N_{6b}) ; 3,76-3,80 (m, 2H, H₂ et H₄) ; 3,90 (dd, 1H, J₃₋₂ = 2,9 Hz, J₃₋₄ = 9,2 Hz, H₃) ; 4,61 (s, 2H, Hₐ) ; 4,74 (d, 2H, J = 12,2 Hz, Hₐ) ; 4,76 (d, 1H, J₁₋₂ = 1,5 Hz, H₁) ; 4,84 (d, 2H, J = 11,0 Hz, Hₐ) ; 7,28-7,41 (m, 15H, 15H_{c,d,e}).
**RMN ¹³C (100,62 MHz, CDCl₃)** δ (ppm) : 7,01 (C₆) ; 55,05 (-OCH3) ; 71,48 (C₅) ; 72,09, 72,73 (2 Cₐ) ; 74,56 (C₂) ; 75,44 (Cₐ) ; 78,52 (C₄) ; 79,87 (C₃) ; 99,05 (C₁) ; 127,66, 127,82, 128,06, 128,33, 128,40 (15C, C_{c,d,e}) ; 138,11 (C_{b}) ; 138,24 (2C_{b}).
**[α]_{D} :** + 28,0 (c = 1,00 g/100 mL, CHCl₃).

### 3) SYNTHESE DU (3R,4R,5S,6R)-3,4,5-TRIS(BENZYLOXY)-TETRAHYDRO-6-METHOXYPYR-2-ENE (Composé 3i)

1 g de 6-désoxy-6-iodo-2,3,4-tri-*O*-benzyl-α-D-mannopyranoside de méthyle (1,74 mmol, 1 éq.) et 2,6 mL de DBU (17,4 mmol, 10 éq.) sont mis en réaction sous argon dans 20 mL de DMF. Après 3 heures et demi de réaction à 80°C, la solution est refroidie à température ambiante, diluée dans de l'AcOEt et lavée avec une solution de NaHCO₃ saturée. La phase organique est ensuite lavée à l'eau, séchée, concentrée et purifiée par chromatographie sur colonne de silice (AcOEt/EP 2/8 v/v) pour donner une huile marron (430 mg, 56%).
**Rf :** 0,34 (AcOEt/EP 5/5 v/v).
**SM (ESI⁺/MeOH) :** m/z 469,34 [M+Na]⁺.
**RMN ¹H (400,13 MHz, CDCl₃)** δ (ppm) : 3,62 (s, 3H, -OCH₃) ; 3,99 (dd, 1H, J₂₋₁ = 2,6 Hz, J₂₋₃ = 8,2 Hz, H₂) ; 4,02 (m, 2H, H₄ et 1Hₐ) ; 4,17 (d, 2H, J = 10,2 Hz, Hₐ) ; 4,34-4,62 (m, 4H, Hₐ), 4,94 (dd, 1H, J₃₋₂ = 1,2 Hz, J₃₋₄ = 5,1 Hz, H₃) ; 4,73 (d, 1H, J₁₋₂ = 2,2 Hz, H₁) ; 4,82, 4,90 (2m, 2H, H₆) ; 7,18-7,32 (m, 15H, H_{c,d,e}).
**RMN ¹³C (100,62 MHz, CDCl₃)** δ (ppm) : 55,52 (-OCH₃) ; 72,27, 72,91 et 74,66 (3Cₐ) ; 74,34 (C₃) ; 76,08 (C₂) ; 79,18 (C₄) ; 82,34 (C₆) ; 99,43 (C₁) ; 127,59-129,11 (C_{c,d,e}) ; 137,73, 138,02, 140,12 (3C_{b} et C₅).

### 4) SYNTHESE DU (3R,4R,5S,6R)-3,4,5-TRIS(BENZYLOXY)-TETRAHYDRO-6-METHOXYPYRAN-2-ONE (Composé 4i)

1,13 g de (3R,4R,5S,6R)-3,4,5-tris(benzyloxy)-tetrahydro-6-methoxypyr-2-ène (2,53 mmol, 1 éq.) sont mis en solution dans 500 mL de tBuOH avant d'ajouter 1,3 g de K₂CO₃ (8,36 mmol, 3,3 éq.) dissous au préalable dans 100 mL d'eau. 0,12 g de KMnO₄ (0,76 mmol, 0,3 éq.) en solution dans 100 mL d'eau et 406 mg de NaIO₄ (1,90 mmol, 0,75 éq.) en solution dans 100 mL d'eau sont ensuite ajoutés. Après 1h d'agitation à température ambiante, la solution est extraite au CH₂Cl₂, la phase organique est séchée, concentrée et purifiée sur colonne de silice (AcOEt/EP 5/5 v/v). Le produit est obtenu sous forme d'une huile jaune pâle (1,07 g, 94%).
**Rf :** 0,55 (AcOEt/EP 6/4 v/v).
**SM (ESI⁺/MeOH) :** m/z 471,18 [M+Na]⁺.
**RMN ¹H (400,13 MHz, CDCl₃)** δ (ppm) : 3,54 (s, 3H, -OCH₃) ; 3,73 (dd, 1H, J₂₋₁ = 2,7 Hz, J₂₋₃ = 8,1 Hz, H₂) ; 4,01 (m, 2H, H₄ et 1Hₐ) ; 4,15 (d, 2H, J = 10,4 Hz, Hₐ) ; 4,96 (dd, 1H, J₃₋₂ = 1,0 Hz, J₃₋₂ = 5,0 Hz, H₃) ; 4,76 (d, 1H, J₁₋₂ = 2,6 Hz, H₁) ; 7,20-7,29 (m, 15H, H_{c,d,e}).
**RMN ¹³C (100,62 MHz, CDCl₃)** δ (ppm) : 55,51 (-OCH₃) ; 72,27, 72,91 et 74,66 (3Cₐ) ; 74,17 (C₅) ; 74,36 (C₃) ; 76,00 (C₂); 79,15 (C₄) ; 99,42 (C₁) ; 127,61-128,41 (C_{c,d,e}) ; 137,73, 138,02 (3C_{b}).

### 5) SYNTHESE DU (3R,4R,5S,6R)-3,4,5-TRIS(BENZYLOXY)-TETRAHYDRO-6-METHOXYPYRAN-2-OL (Composé 5i)

1,07 g de (3R,4R,5S,6R)-3,4,5-tris(benzyloxy)-tétrahydro-6-méthoxypyran-2-one (2,39 mmol, 1 éq.) sont mis à réagir avec 180 mg de NaBH₄ (4,78 mmol, 2 éq.) dans 25 mL de MeOH. Après 16 heures d'agitation à température ambiante, 2 mL d'eau sont ajoutés et la solution est ensuite concentrée. Une chromatographie sur colonne de gel de silice (AcOEt/EP 5/5 v/v) permet d'obtenir le produit sous forme d'une huile jaune (1 g, 94%).
**Rf** : 0,55 (AcOEt/EP 5/5 v/v).
**SM (ESI⁺/MeOH) :** m/z 473,67 [M+Na]⁺.
**RMN ¹H (400,13 MHz, CDCl₃)** δ (ppm) : 3,49 (s, 3H, -OCH₃) ; 3,60 (dd, 1H, J₂₋₁ = 1,2 Hz, J₂₋₃ = 6,8 Hz, H₂) ; 3,82 (m, 2H, H₄ et 1Hₐ) ; 4,00 (d, 1H, J = 10,4 Hz, 1Hₐ) ; 4,10 (dd, 1H, J₃₋₂ = 1,0 Hz, J₃₋₄ = 5,0 Hz, H₃); 4,31 (t, 1H, J₅₋₄ = J₅₋₆ = 6,2 Hz, H₅); 4,38-4,78 (m, 4H, Hₐ) ; 4,77 (d, 1H, J₁₋₂ = 2,6 Hz, H₁) ; 7,18-7,31 (m, 15H, H_{c,d,e}).
**RMN ¹³C (100;62 MHz, CDCl₃)** δ (ppm) : 57,40 (-OCH₃) ; 73,60, 74,23, 74,86 (3Cₐ) ; 76,65 (C₃) ; 78,61 (C₂); 79,12 (C₄) ; 100,56 (C₁) ; 127,97-128,64 (C_{c,d,e}); 137,14, 137,89 (3C_{b}).

### 6) SYNTHESE DU (3R,4R,5S,6R)-3,4,5-TRIS(BENZYLOXY)-TETRAHYDRO-6-OXYDIISOPROPYLPHOSHONYLMETHOXYPYRANE (Composé 6i)

640 mg de (3R,4R,5S,6R)-3,4,5-tris(benzyloxy)-tétrahydro-6-méthoxypyran-2-ol (1,43 mmol, 1 éq.) sont dissous dans 6,4 mL de DMF pour former une solution très visqueuse. 14 mg de LiI (0,10 mmol, 0,07 éq.) et 241 mg de tBuOK (2,14 mmol, 1,5 éq.) sont ensuite ajoutés, la solution devient liquide. Sous argon, 482 mg de phosphonate (1,86 mmol, 1,3 éq.) dissous au préalable dans 3,2 mL de DMF sont ajoutés au mélange goutte à goutte. La réaction est laissée 10 minutes sous agitation à température ambiante puis chauffée pendant 3 heures à 60°C. Après refroidissement, le mélange est dilué dans l'AcOEt et lavé avec une solution de NaCl saturée. La phase organique est séchée sur Na₂SO₄, filtrée, concentrée et purifiée sur colonne de silice (AcOEt/EP 8/2 v/v) pour donner une huile incolore (698 mg, 78%).
**Rf :** 0,45 (AcOEt/EP 8/2 v/v).
**SM (ESI⁺/MeOH) :** m/z 651,90 [M+Na]⁺.
**RMN ¹H (400,13 MHz, CDCl₃)** δ (ppm) : 1,35 (d, 8H, J₈₋₇ = 6,0 Hz, H₈) ; 3,22 (s, 2H, H₆) ; 3,55 (s, 3H, -OCH₃) ; 3,66 (dd, 1H, J₂₋₁ = 1,2 Hz, J₂₋₃ = 6,4 Hz, H₂) ; 3,89 (m, 2H, H₄ et 1Hₐ) ; 4,06 (d, 2H, J = 10,4 Hz, Hₐ) ; 4,16 (dd, 1H, J₃₋₂ = 1,2 Hz, J₃₋₄ = 6,0 Hz, H₃) ; 4,37 (t, 1H, J₅₋₄ = J₅₋₆ = 6,2 Hz, H₅) ; 4,44-4,84 (m, 6H, Hₐ, H₁ et H₇) ; 7,26-7,36 (m, 15H, H_{c,d,e}).
**RMN ¹³C (100,62 MHz, CDCl₃)** δ (ppm) : 17,99 (C₆) ; 23,78, 23,83, 23,97, 24,01 (C₈) ; 56,94 (-OCH₃) ; 69,47, 71,95 et 72,67 (3Cₐ) ; 72,10 et 72,17 (C₇) ; 73,12 (C₅) ; 75,27 (C₃) ; 75,92 (C₂); 77,17 (C₄) ; 102,86 (C₁) ; 127,53-128,46 (C_{c,d,e}) ; 137,23, 138,35 (3C_{b}).

### 7) SYNTHESE DU (3R,4R,5S,6R)-3,4,5-TRIS(BENZYLOXY)-TETRAHYDRO-6-OXYPHOSHONYLMETHOXYPYRANE (Composé 7i)

700 mg de (3R,4R,5S,6R)-3,4,5-tris(benzyloxy)-tétrahydro-6-oxydiisopropylphosphonyl-méthoxypyrane (1,12 mmol, 1 éq.) sont mis à réagir dans 20 mL de CH₂Cl₂ avec 900 µL de pyridine (11,2 mmol, 10 éq.) et 813 µL de TMSBr (5,59 mmol, 5 éq.). Après 16 heures d'agitation à température ambiante, le mélange est dilué dans de l'AcOEt, puis lavé à l'eau. La phase organique est séchée et concentrée. L'huile transparente obtenue est assez pure pour être remise directement en réaction. Le phosphonate protégé dissous dans 30 mL d'un mélange MeOH/THF 1/1 v/v, dégazé au préalable sous argon, est mis à réagir avec du Pd/C sous atmosphère de H₂. Après 18 heures de réaction, la solution est filtrée puis concentrée. Le produit est obtenu sous forme d'une huile blanche (43% sur les 2 étapes).
**Rf :** 0,15 (Isopropanol, NH₄OH 5/5 v/v).
**SM (ESI⁺/MeOH)** : m/z 277,64 [M+Na]⁺.
**RMN ¹H (400,13 MHz, D₂O)** δ (ppm) : 3,25 (s, 3H, -OCH₃) ; 3,48 (m, 2H, H₄ et H₅) ; 3,60 (m, 2H, H₃ et H₆ₐ) ; 3,76 (m, 1H, H_{6b}) ; 3,77 (dd, 1H, J₂₋₁ = 1,8 Hz, J₂₋₃ = 3,4 Hz, H₂) ; 4,61 (d, 1H, J₁₋₂ = 2,0 Hz, H₁).
**RMN ¹³C (100,62 MHz, D₂O)** δ (ppm) : 54,61 (-OCH₃) ; 60,86 (C₆) ; 66,67 (C₄) ; 69,82 (C₂) ; 70,44 (C₃) ; 72,45 (C₅) ; 100,76 (C₁).

### RESULTATS BIOLOGIQUES :

### A- Sur anneaux d'aorte de rat

Les effets biologiques des composés de l'invention sur l'angiogenèse ont été testés selon une technique bien connue de l'homme du métier : la technique des anneaux d'aorte de rat (Nicosia et al., M. Am. J. Pathol., 1994 Nov ; 145(5) : 1023-9). Cette approche est rapide et consiste à placer un anneau d'aorte de rat d'un millimètre d'épaisseur dans un système de culture en trois dimensions formé à partir d'un réseau de collagène de type 1. Les aortes sont obtenues chez des rats Sprague-Dawley et cultivées pendant 9 à 11 jours en présence ou non des composés à tester. L'angiogenèse est ensuite appréciée en évaluant le nombre, la taille et l'organisation des bourgeons vasculaires progressant dans le lattis de collagène.

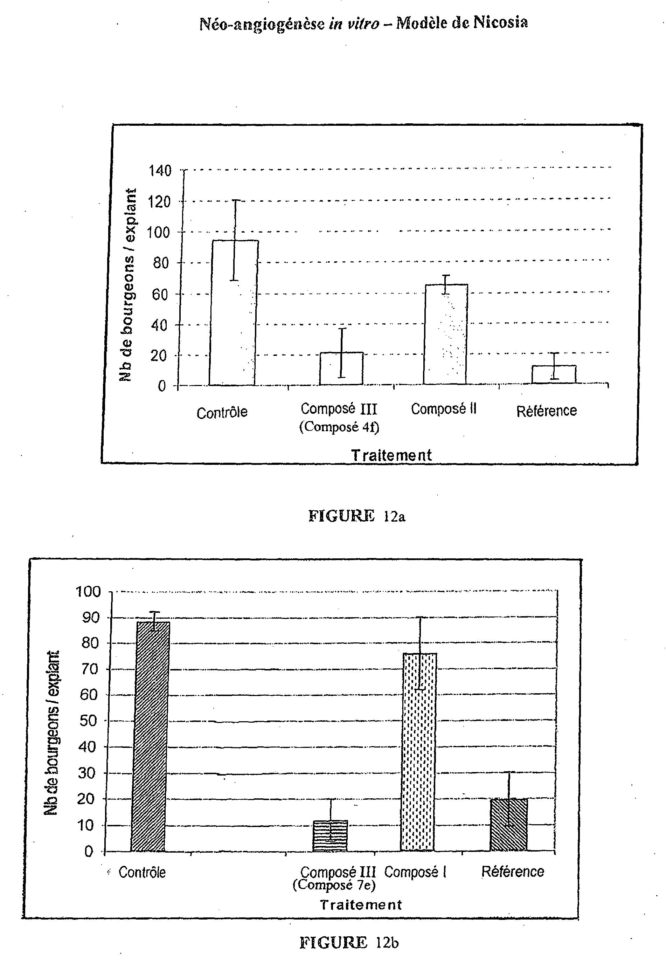

Les histogrammes des Figures 12a et 12b représentent l'effet néo-angiogénique *in vitro* (modèle de Nicosia) de différents composés de l'invention :
- Contrôle : aorte non traitée,
- Composé III (composé **4f) :** aorte traitée avec un composé de formule (III) dans lequel Z est -X-HP(O)OH, X est un atome d'oxygène et R₂ est un groupement méthyle (synthétisé selon l'exemple 4),
- Composé II : aorte traitée avec un composé de formule (II) dans lequel 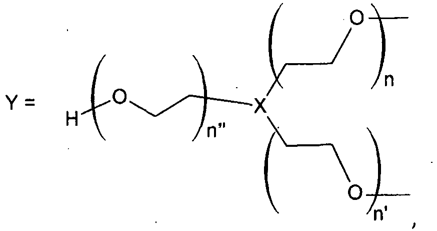 X est un atome d'oxygène, n = 1, n' = 2 et n" = 0, et R₁ = R'₁ = -N₃,
- Composé III (composé 7e) : aorte traitée avec un composé de formule (III) dans lequel Z est -CH₂-B(OH)₂ et R₂ est un groupement méthyle (synthétisé selon l'exemple 3),
- Composé 1 : aorte traitée avec un composé de formule (I) dans lequel A est une nanoparticule d'or, R₁ = -COOH et m = 5, et
- Référence : aorte traitée avec du sunitinib : le Sutent^{®} (commercialisé par Pfizer) répondant à la formule :

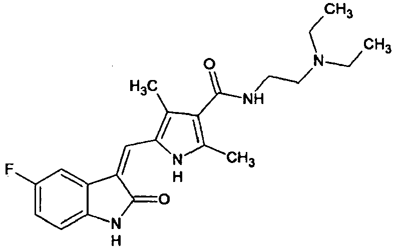

### B- Sur cellules endothéliales

Des cellules endothéliales dermiques humaines (15 000/puits) sont traitées avec différents composés répondant à la définition de l'invention pendant 48 heures.

Au terme de ce traitement, le nombre de cellule est estimé grâce à l'activité des succinates déshydrogénases (test au MTT, Mosmann T. J., Immunol. Methods, 1983 Dec. 16 ; 65(1-2) : 55-63). Les résultats montrent que le traitement des cellules avec les composés de l'invention à trois doses différentes (10⁻² mol.L⁻¹, 10⁻⁴ mol.L⁻¹ et 10⁻⁶ mol.L⁻¹) n'a pas d'effet cytotoxique sur les cellules endothéliales.

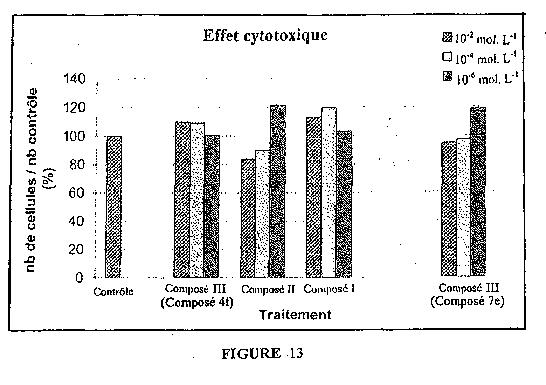

L'histogramme de la Figure 13 représente l'effet cytotoxique de différents composés :
- Contrôle : cellules non traitées,
- Composé III (composé **4f**) : cellules traitées avec un composé de formule (III) dans lequel Z est -X-HP(O)OH, X est un atome d'oxygène et R₂ est un groupement méthyle (synthétisé selon l'Exemple 4),
- Composé II : cellules traitées avec un composé de formule (II) dans lequel : 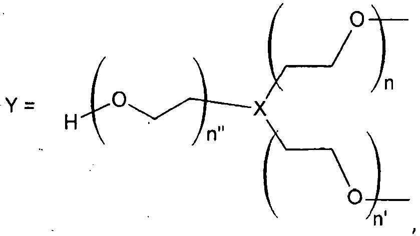 X est un atome d'oxygène, n = 1, n' = 2, n" = 0, et R₁ = R'₁ = -N₃,
- Composé 1 : cellules traitées avec un composé de formule (I) dans lequel A est une nanoparticule d'or, R₁ = -COOH et m = 5, et
- Composé III (composé **7e) :** cellules traitées avec un composé de formule (III) dans lequel Z est -CH₂-B(OH)₂ et R₂ est un groupement méthyle (synthétisé selon l'Exemple 3).

### C- Sur souris présentant un mélanome B16

Un mélanome est induit sur une souris par injection sous-cutanée de cellules tumorales (cellules B16). La tumeur se développe dans les 10 jours en position sous-cutanée (face ventrale de la cuisse). La taille de la tumeur est mesurée au pied à coulisse et son volume estimée selon la formule V = L x 1 x 1, dans laquelle L représente la longueur et 1 représente la largeur. Différents composés de l'invention ont été administrés à des souris ayant reçu des cellules de mélanomes syngéniques (B16) à une dose de 300 mg/kg.

Les différents composés testés sont les suivants :
- Contrôle : souris non traitées,
- M6P : souris traitées avec du mannose-6-phosphate (M6P),
- Composé III (composé **4f**) : souris traitées avec un composé de formule (III) dans lequel Z est -X-HP(O)OH, X est un atome d'oxygène et R₂ est un groupement méthyle (synthétisé selon l'Exemple 4),
- Composé III (composé **7e) :** souris traitées avec un composé de formule (III) dans lequel Z est -CH₂-B(OH)₂ et R₂ est un groupement méthyle (synthétisé selon l'Exemple 3),
- Composé II : cellules traitées avec un composé de formule (II) dans lequel 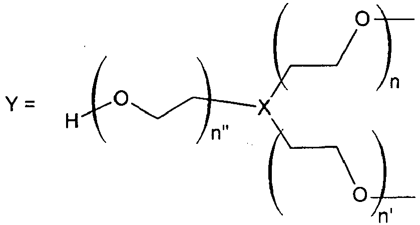 X est un atome d'oxygène, n = 1, n' = 2, n" = 0, et R₂ = R'₂ = -N₃,
- Composé I : cellules traitées avec un composé de formule (I) dans lequel A est une nanoparticule d'or, R₁ = -COOH et m = 3.

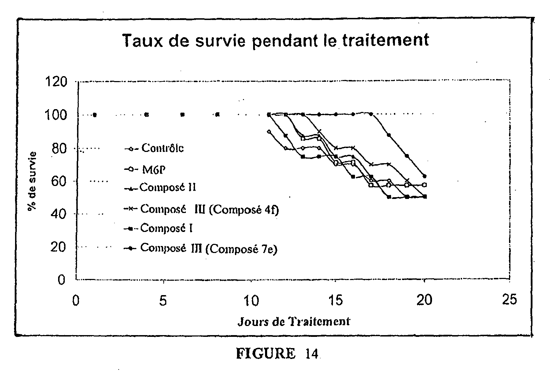

Le taux de survie des souris en fonction du nombre de jours de traitement est représenté sur le graphe de la Figure 14 annexée.

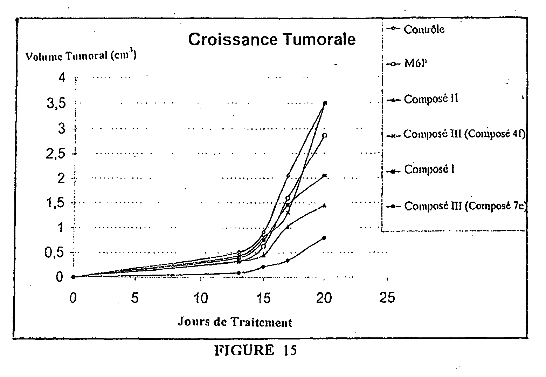

L'évolution de la croissance tumorale en fonction du nombre de jours de traitement est représentée sur le graphe de la Figure 15 annexée.

Nous avons observé une forte inhibition (65%) de la croissance tumorale dans le groupe de souris traité avec le composé **7f** de formule (III) de l'invention, une inhibition de 40% dans le groupe de souris traité avec le composé **4e** de formule (III) de l'invention, et une inhibition de 30% dans le groupe de souris traité avec le composé de formule (I) de l'invention. Parallèlement, aucune mortalité n'est observée dans le groupe de souris traité avec le composé 7f de formule (III) de l'invention après 18 jours de traitement, en comparaison au groupe de souris non traité (40% de mortalité).

### D- Activité angiogénique sur membrane chorioallantoïdienne (CAM)

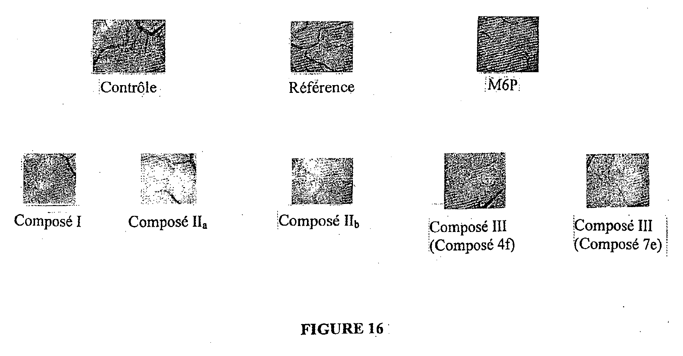

Sur les photographies de la Figure 16 annexée, on peut observer les résultats obtenus lors d'un test classique d'étude de l'angiogénèse *in vivo* réalisé sur la membrane chorioallantoïdienne (CAM) d'embryon de poulet.

Les différents composés testés sont les suivants :
- Contrôle : souris non traitées,
- Référence : souris traitées avec le Sutent^{®},
- M6P : souris traitées avec du mannose-6-phosphate (M6P),
- Composé 1 : cellules traitées avec un composé de formule (1) dans lequel A est une nanoparticule d'or, R₁ = -N₃ et m = 5,
- Composé IIₐ : cellules traitées avec un composé de formule (II) dans lequel 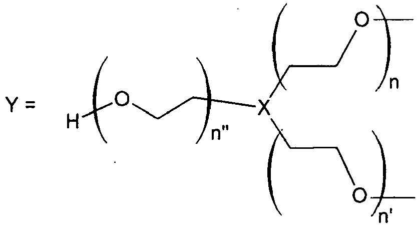 X est un atome d'oxygène, n = 1, n' = 2, n" = 0, et R₁ = R'₁ = -N₃,
- Composé II_{b} : cellules traitées avec un composé de formule (II) (composé de l'exemple 7) dans lequel 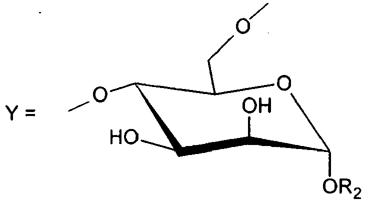 R₁ = R'₁ = -N₃, et R₂ est un groupement méthyle,
- Composé III (composé **4f**) : souris traitées avec un composé de formule (III) dans lequel Z est -X-HP(O)OH, X est un atome d'oxygène et R₂ est un groupement méthyle (synthétisé selon l'Exemple 4),
- Composé III (composé 7e) : souris traitées avec un composé de formule (III) dans lequel Z est -CH₂-B(OH)₂ et R₂ est un groupement méthyle (synthétisé selon l'Exemple 3).

Les composés de l'invention présentent un fort effet inhibiteur, ainsi que des effets secondaires considérablement amoindris par rapport à ceux de la référence. Les composés de l'invention présentent également une activité anti-angiogénique supérieure à celle du M6P.

## Revendications

1. Composé dérivé de mannopyranoside ou ses sels pharmaceutiquement acceptables, **caractérisé en ce qu'**il répond à l'une des formules suivantes :
➢ Formule (I) : 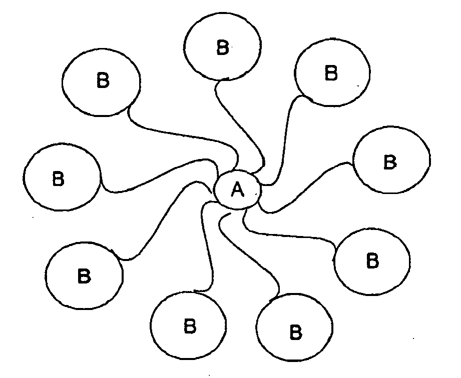 dans laquelle :
■ A est une nanoparticule de silice ou une nanoparticule métallique choisie parmi les éléments des colonnes (IB), (IIB), (IIIB), (IVB), (VB), (VIB), (VIIB) ou (VIIIB) de la classification de Mendeleïev, et
■ B est un groupement porteur d'une fonction mannopyranoside répondant à la structure suivante : 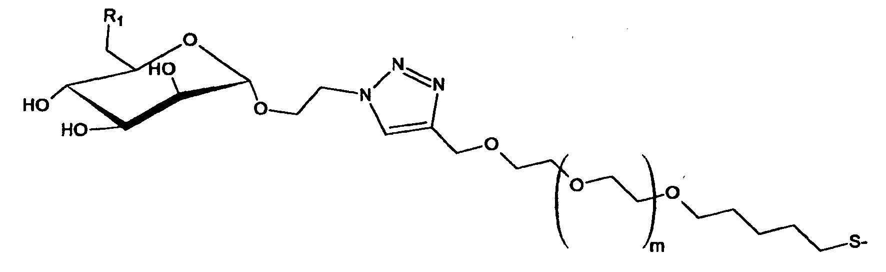 dans lequel :
- le radical R₁ représente un radical sélectionné parmi -O-PO₃H₂, -N₃, -CH₂-PO₃H₂,-CH₂-COOH, -SO₃H, -OPHO₂H -CH₂-B(OH)₂, -X-PHO₂H -X'-PO₂H-X-PO₃H₂, et de préférence -CH₂-COOH et -N₃, et
- m est un entier compris entre 0 et 10, et de préférence m = 3, 4, 5 ou 6,
les groupements B étant liés à la nanoparticule A via l'atome de soufre, et le nombre de groupements B liés à la nanoparticule A étant compris entre 100 et 1000, et de préférence entre 400 et 600,
➢ Formule (II) : 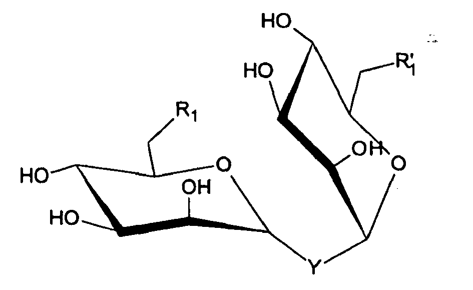 dans laquelle :
- les radicaux R₁ et R'₁, identiques ou différents, représentent des radicaux sélectionnés parmi -O-PO₃H₂, -N₃, -CH₂-PO₃H₂, -CH₂-COOH, -SO₃H, -OPHO₂H -CH₂-B(OH)₂, -X-PHO₂H, -X'-PO₂H-X-PO₃H₂, et de préférence -CH₂-COOH et -N₃, et
- Y représente l'un des groupements suivants : 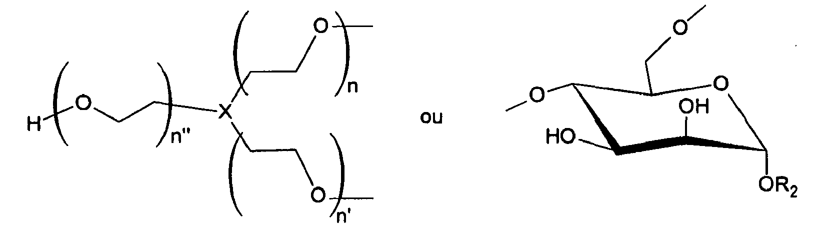
avec :
○ n, n' et n" étant des entiers compris entre 1 et 12, et de préférence entre 1 et 6,
○ n" étant égal à 0 lorsque X représente un atome d'oxygène, le groupement X étant choisi parmi: N, O, S, une chaîne alkyle en C₁-C₄, le groupement X étant de préférence un atome d'oxygène, et
○ le radical R₂ représente une chaîne alkyle linéaire ou ramifiée en C₁-C₁₂, et de préférence en C₁-C₄ ; une chaîne alkyle linéaire ou ramifiée en C₁-C₁₂, et de préférence en C₁-C₄, porteur d'au moins un groupement -OH, -NH₂, -SH, -COOH, -N₃, -NO₂ ; un cycle hydrocarboné, saturé ou insaturé, en C₃-C₆ ; un cycle hydrocarboné en C₃-C₁₀, saturé ou insaturé, porteur d'au moins un groupement -OH, -NH₂, -SH, -COOH, -N₃,-NO₂, alkyle en C₁-C₄ ; un hétérocycle saturé ou insaturé comportant au moins un hétéroatome choisi parmi les atomes d'oxygène, d'azote ou de soufre ; un radical - (CH₂-CH₂-O)_{y}-H, dans lequel y est compris entre 1 et 12, et de préférence entre 1 et 6,
➢ Formule (III) : 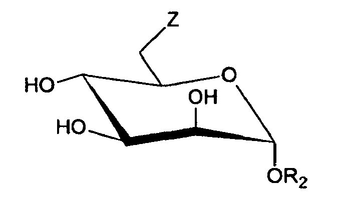
dans laquelle :
- Z représente l'un des groupements suivants : 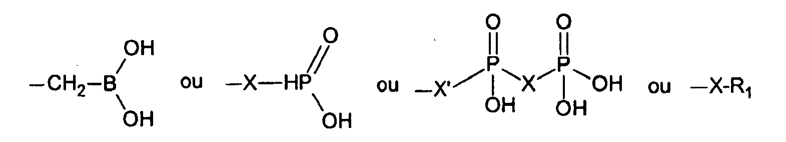
- le radical R₁ est sélectionné parmi -O-PO₃H₂, -N₃, -CH₂-PO₃H₂, -CH₂-COOH,-OPHO₂H, -CH₂-B(OH)₂, -X-PHO₂H -X'-PO₂H-X-PO₃H₂, et de préférence -CH₂-COOH et-N₃,
- le radical R₂ représente une chaîne alkyle linéaire ou ramifiée en C₁-C₁₂, et de préférence en C₁-C₄ ; une chaîne alkyle linéaire ou ramifiée en C₁-C₁₂, et de préférence en C₁-C₄, porteur d'au moins un groupement -OH, -NH₂, -SH, -COOH, -N₃, -NO₂; un cycle hydrocarboné, saturé ou insaturé, en C₃-C₆ ; un cycle hydrocarboné en C₃-C₁₀, saturé ou insaturé, porteur d'au moins un groupement -OH, -NH₂, -SH, -COOH, -N₃, -NO₂, alkyle en C₁-C₄ ; un hétérocycle saturé ou insaturé comportant au moins un hétéroatome choisi parmi les atomes d'oxygène, d'azote ou de soufre ; un radical -(CH₂-CH₂-O)_{y}-H, dans lequel y est compris entre 1 et 12, et de préférence entre 1 et 6, et
- les groupements X et X', identiques ou différents, sont choisis parmi : N, O, S, une chaîne alkyle en C₁-C₄, les groupements X et X' étant de préférence des atomes d'oxygène.

2. Composé selon la revendication 1, **caractérisé en ce que** R₂ représente une chaîne alkyle en C₁-C₄ choisie parmi les radicaux méthyle, éthyle, *n*-propyle, isopropyle, *n-*butyle, *tert*-butyle, isobutyle et *n*-hexyle, et de préférence le radical méthyle.

3. Composé selon la revendication 1, **caractérisé en ce que** R₂ représente un cycle hydrocarboné saturé choisi parmi le cyclopropane, le cyclobutane, le cyclopentane et le cyclohexane.

4. Composé selon la revendication 1, **caractérisé en ce que** R₂ représente un cycle hydrocarboné insaturé ou un hétérocycle saturé choisi parmi les cycles phényle, oxadiazole, triazole, oxazole, isoxazole, imidazole, thiadiazole, pyrrole, tétrazole, furane, thiophène, pyrazole, pyrazoline, pyrazolidine, thiazole, isothiazole, pyridine, pyrimidine, pipéridine, pyranne, pyrazine, pyridazine, indole, indazole, benzoxazole, naphtalène, quinoline, quinoxaline, quinazoline, anthracène et acridine, et de préférence les cycles phényles.

5. Composé selon l'une des revendications 1 à 4, **caractérisé en ce que** les nanoparticules A du composé de formule (I) sont choisies parmi les nanoparticules d'or, de fer et de cobalt.

6. Composé selon l'une des revendications 1 à 5, **caractérisé en ce que** les nanoparticules A ont un diamètre compris entre 2 et 10 nm, et de préférence entre 4 et 8 nm.

7. Procédé de préparation d'un composé dérivé de mannopyranoside selon l'une des revendications 1 à 6, **caractérisé en ce qu'**il comprend au moins les étapes suivantes :
(i) une étape d'halogénation entre un composé de formule (I'), (II') ou (III') porteur d'au moins une fonction alcool primaire, par réaction avec un mélange dihalogène/phosphine ou N-halogénosuccinimide/phosphine, lesdits composés (I'), (II') ou (III') répondant aux formules suivantes :
➢ Formule (I') : 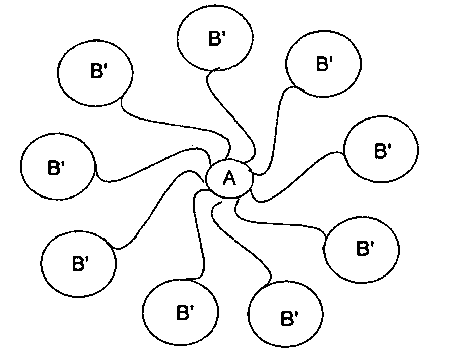 dans laquelle A est telle que définie aux revendications 1 à 6, et B' est un groupement répondant à la structure suivante : 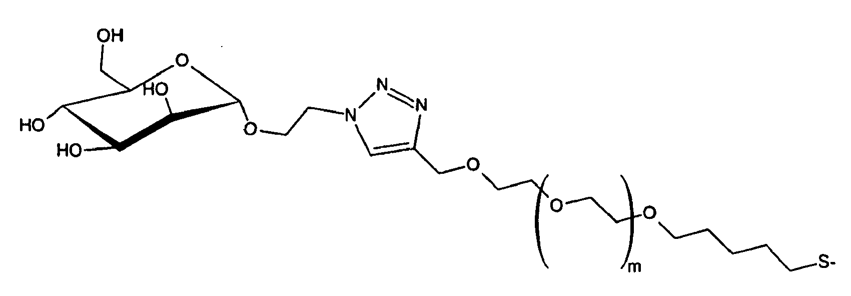 dans lequel m est tel que défini aux revendications 1 à 6,
les groupements B' étant liés à la nanoparticule A via l'atome de soufre, et le nombre de groupements B' liés à la nanoparticule A étant compris entre 100 et 1000, et de préférence entre 400 et 600,
➢ Formule (II') : 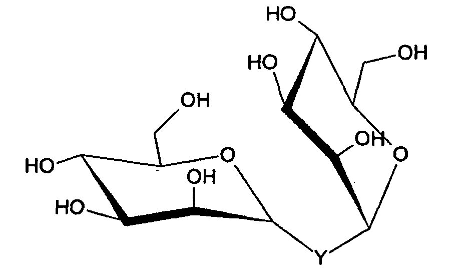 dans laquelle Y, n, n' et n" sont tels que définis aux revendications 1 à 6,
➢ Formule (III') : 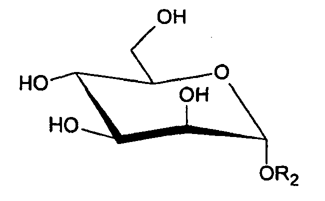 le radical R₂ et les groupements X et X' étant tels que définis aux revendications 1 à 6,
(ii) une étape de substitution nucléophile des composés halogénés obtenus lors de l'étape (i) par réaction avec un réactif nucléophile porteur d'un radical R₁ et/ou R'₁, pour obtenir les composés de formule (I), (II) ou (III) tels que définis selon les revendications 1 à 6.

8. Procédé selon la revendication 7, **caractérisé en ce que** l'étape (i) est réalisée avec un mélange diiode/triphénylphoshine, en présence d'imidazole.

9. Composé dérivé de mannopyranoside selon l'une des revendications 1 à 6, pour une utilisation à titre de médicament.

10. Composé selon la revendication 9, pour une utilisation à titre de médicament destiné à la prévention et/ou au traitement de maladies dépendantes d'une inhibition de l'angiogénèse.

11. Composé selon la revendication 10, pour une utilisation à titre de médicament destiné au traitement de maladies cancéreuses, de la cécité diabétique, de la dégénérescence maculaire, de la polyarthrite rhumatoïde et du psoriasis.

12. Composé selon la revendication 11, pour une utilisation à titre de médicament destiné au traitement de maladies cancéreuses.

13. Composition pharmaceutique **caractérisé en ce qu'**elle comprend, à titre de principe actif, au moins un composé dérivé de mannopyranoside tel que défini selon l'une des revendications 1 à 6, et au moins un excipient pharmaceutiquement acceptable.

14. Composition pharmaceutique selon la revendication 13 **caractérisée en ce qu'**elle comprend un ou plusieurs principes actifs antitumoraux choisis parmi la doxorubicine, l'étoposide, le fluorouracile, le melphalan, la cyclophosphamide, la béomycine, la vinblastine, la mitomycine, la lomustine (CCNU), le taxotère, le taxol, le métotrexate et le cisplatinum.

15. Dispositif médical implantable **caractérisé en ce qu'**il est traité en surface par au moins un composé dérivé de mannopyranoside tel que défini selon l'une des revendications 1 à 6, ledit dispositif pouvant être choisi parmi les prothèses, et plus particulièrement les stents vasculaires, urétrals et biliaires.

## Patentansprüche

1. Mannopyranosidderivat-Verbindung oder pharmazeutisch verträgliche Salze davon, **dadurch gekennzeichnet, dass** sie eine der folgenden Formeln hat:
○ Formel (I) 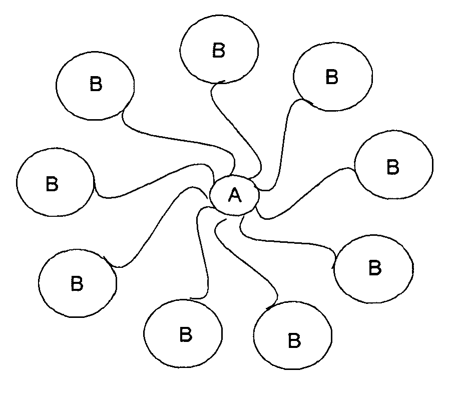 worin:
- A ein Siliciumdioxidnanoprtikel oder ein metallisches Nanopartikel ist, das ausgewählt ist aus der Elementen der Gruppen (IB), (IIB), (IIIB), (IVB), (VB), (VIB), (VIIB) oder (VIIIB) des Periodensystems nach Mendeleïev, und
- B eine Gruppe ist, die eine Mannopyranosidfunktion der folgenden Struktur trägt: 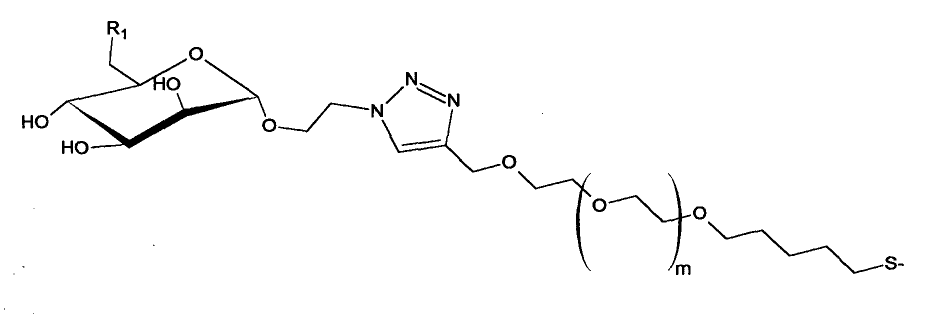
worin
- der Rest R₁ einen Rest bedeutet, der ausgewählt ist aus -O-PO₃H₂, -N₃, -CH₂-PO₃H₂, -CH₂-COOH, -SO₃H, -OPHO₂H -CH₂-B(OH)₂, -X-PHO₂H -X'-PO₂H-X-PO₃H₂ und vorzugsweise -CH₂-COOH und -N₃, und
- m eine ganze Zahl zwischen 0 und 10 ist und vorzugsweise m = 3, 4, 5 oder 6,
wobei die Gruppen B über das Schwefelatom an das Nanopartikel A gebunden sind und die Anzahl der an das Nanopartikel A gebundenen Gruppen B zwischen 100 und 1000 und vorzugsweise zwischen 400 und 600 liegt,
○ Formel (II) 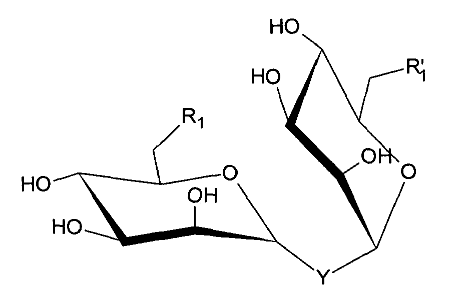 worin
- die Reste R₁ und R'₁, die gleich oder verschieden sein können, Reste bedeuten, die ausgewählt sind aus -O-PO₃H₂, -N₃, -CH₂-PO₃H₂, -CH₂-COOH, -SO₃H, -OPHO₂H -CH₂-B(OH)₂, -X-PHO₂H -X'-PO₂H-X-PO₃H₂ und vorzugsweise -CH₂-COOH und -N₃, und
- Y eine der folgenden Gruppen bedeutet: 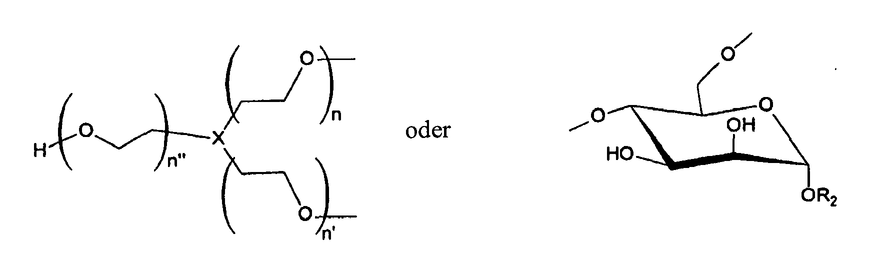
wobei:
- n, n' und n" ganze Zahlen zwischen 1 und 12 und vorzugsweise zwischen 1 und 6 sind,
- n" 0 ist, wenn X ein Sauerstoffatom bedeutet, die Gruppe X ausgewählt ist aus: N, O, S und einer C₁-C₄-Alkylkette, wobei die Gruppe X vorzugsweise ein Sauerstoffatom ist, und
- der Rest R₂ eine lineare oder verzweigte C₁-C₁₂- und vorzugsweise C₁-C₄-Alkylkette; eine lineare oder verzweigte C₁-C₁₂- und vorzugsweise C₁-C₄-Alkylkette, die wenigstens eine -OH-, -NH₂-, -SH-, -COOH-, -N₃- oder -NO₂-Gruppe trägt; einen gesättigten oder ungesättigten C₃-C₆-Kohlenwasserstoffring; einen gesättigten oder ungesättigten C₃-C₁₀-Kohlenwasserstoffring, der wenigstens eine -OH-, -NH₂-, -SH-, -COOH-, -N₃-, -NO₂- oder C₁-C₄-Gruppe trägt; einen gesättigten oder ungesättigten heterozyklischen Ring, der wenigstens ein Heteroatom enthält, das ausgewählt ist aus Sauerstoff-, Stickstoff- oder Schwefelatomen; einen -(CH₂-CH₂-O)_{y}-H-Rest, worin y zwischen 1 und 12 und vorzugsweise zwischen 1 und 6 ist, bedeutet
○ Formel (III) 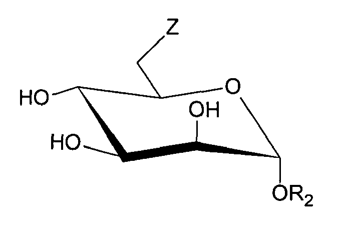 worin:
- Z eine der folgenden Gruppen bedeutet: 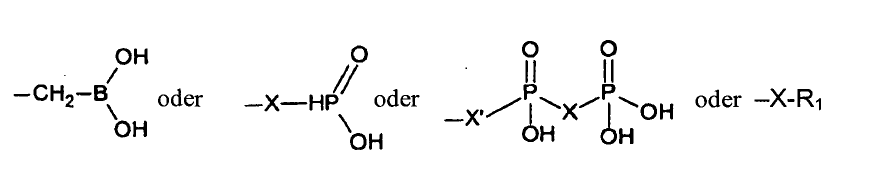
- der Rest R₁ ausgewählt ist aus -O-PO₃H₂, -N₃, -CH₂-PO₃H₂, -CH₂-COOH, -OPHO₂H -CH₂-B(OH)₂, -X-PHO₂H -X'-PO₂H-X-PO₃H₂ und vorzugsweise -CH₂-COOH und -N₃,
- der Rest R₂ eine lineare oder verzweigte C₁-C₁₂- und vorzugsweise C₁-C₄-Alkylkette; eine lineare oder verzweigte C₁-C₁₂- und vorzugsweise C₁-C₄-Alkylkette, die wenigstens eine -OH-, -NH₂-, -SH-, -COOH-, -N₃- oder -NO₂-Gruppe trägt; einen gesättigten oder ungesättigten C₃-C₆-Kohlenwasserstoffring; einen gesättigten oder ungesättigten C₃-C₁₀-Kohlenwasserstoffring, der wenigstens eine -OH-, -NH₂-, -SH-, -COOH-, -N₃-, -NO₂- oder C₁-C₄-Gruppe trägt; einen gesättigten oder ungesättigten heterozyklischen Ring, der wenigstens ein Heteroatom enthält, das ausgewählt ist aus Sauerstoff-, Stickstoff- oder Schwefelatomen; einen -(CH₂-CH₂-O)_{y}-H-Rest, worin y zwischen 1 und 12 und vorzugsweise zwischen 1 und 6 ist, bedeutet, und
- die Gruppen X und X', die gleich oder verschieden sein können, ausgewählt sind aus: N, O, S und einer C₁-C₄-Alkylkette, wobei die Gruppen X und X' vorzugsweise Sauerstoffatome sind.

2. Verbindung nach Anspruch 1, **dadurch gekennzeichnet, dass** R₂ eine C₁-C₄-Alkylkette bedeutet, ausgewählt aus den Resten Methyl, Ethyl, n-Propyl, Isopropyl, n-Butyl, *tert*.-Butyl, Isobutyl und n-Hexyl, und vorzugsweise einen Methylrest.

3. Verbindung nach Anspruch 1, **dadurch gekennzeichnet, dass** R₂ einen gesättigten Kohlenwasserstoffring bedeutet, ausgewählt aus Cyclopropan, Cyclobutan, Cyclopentan und Cyclohexan.

4. Verbindung nach Anspruch 1, **dadurch gekennzeichnet, dass** R₂ einen ungesättigten Kohlenwasserstoffring oder einen gesättigten heterocyclischen Ring bedeutet, ausgewählt aus Phenyl-, Oxadiazol-, Triazol-, Oxazol-, Isoxazol-, Imidazol-, Thiadiazol-, Pyrrol-, Tetrazol-, Furan-, Thiophen-, Pyrazol-, Pyrazolin-, Pyrazolidin-, Thiazol-, Isothiazol-, Pyridin-, Pyrimidin-, Piperidin-, Pyran-, Pyrazin-, Pyridazin-, Indol-, Indazol-, Benzoxazol-, Naphthalin-, Chinolin-, Chinoxalin-, Chinazolin-, Anthracen- und Acridin-Ringen und vorzugsweise Phenylringen.

5. Verbindung nach einem der Ansprüche 1 bis 4, **dadurch gekennzeichnet, dass** die Nanopartikel A der Verbindung der Formel (I) ausgewählt sind aus Nanopartikeln aus Gold, aus Eisen und aus Cobalt.

6. Verbindung nach einem der Ansprüche 1 bis 5, **dadurch gekennzeichnet, dass** die Nanopartikel A einen Durchmesser zwischen 2 und 10 nm und vorzugsweise zwischen 4 und 8 nm haben.

7. Verfahren zur Herstellung einer Mannopyranosidderivat-Verbindung nach einem der Ansprüche 1 bis 6, **dadurch gekennzeichnet, dass** es wenigstens die folgenden Schritte umfasst:
(i) einen Halogenierungsschritt einer Verbindung der Formel (I'), (II') oder (III'), die wenigstens eine primäre Alkoholfunktion trägt, durch Umsetzung mit einer Dihalogen/Phosphin- oder N-Halogensuccinimid/Phosphin-Mischung, wobei die Verbindungen (I'), (II') oder (III') die folgenden Formeln haben:
○ Formel (I') 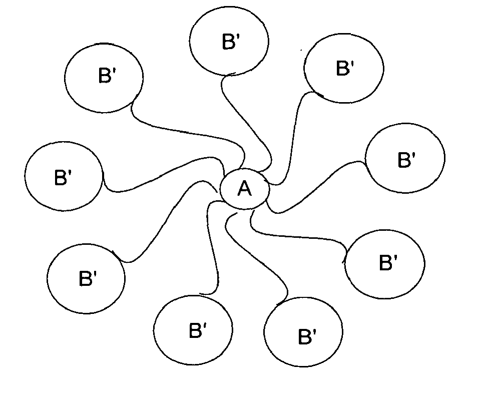 worin A wie in den Ansprüchen 1 bis 6 definiert ist und B' eine Gruppe der folgenden Struktur ist: 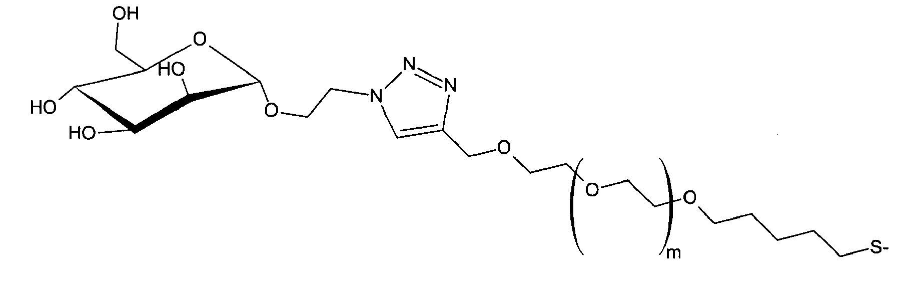 worin m wie in den Ansprüchen 1 bis 6 definiert ist,
wobei die Gruppen B' über das Schwefelatom an das Nanopartikel A gebunden sind und die Anzahl der an das Nanopartikel A gebunden Gruppen B' zwischen 100 und 1000 und vorzugsweise zwischen 400 und 600 liegt,
○ Formel (II') 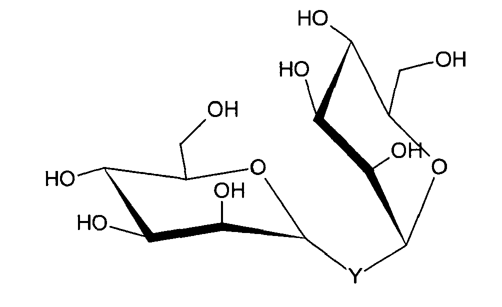 worin Y, n, n' und n" wie in den Ansprüchen 1 bis 6 definiert sind,
○ Formel (III') 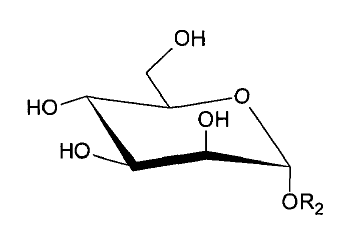 wobei der Rest R₂ und die Gruppen X und X' wie in den Ansprüchen 1 bis 6 definiert sind,
(ii) einen Schritt der nukleophilen Substitution der in Schritt (i) erhaltenen halogenierten Verbindungen durch Umsetzung mit einem nukleophilen Reaktionspartner, der einen Rest R₁ und/oder R'₁ trägt, zu den Verbindungen der Formeln (I), (II) oder (III), wie sie in den Ansprüchen 1 bis 6 definiert sind.

8. Verfahren nach Anspruch 7, **dadurch gekennzeichnet, dass** Schritt (i) mit einer Diiodid/Triphenylphosphin-Mischung in Gegenwart von Imidazol durchgeführt wird.

9. Mannopyranosidderivat-Verbindung nach einem der Ansprüche 1 bis 6 zur Verwendung als Medikament.

10. Verbindung nach Anspruch 9 zur Verwendung als Medikament zur Prävention und/oder Behandlung von Krankheiten, die von einer Angiogenesehemmung abhängen.

11. Verbindung nach Anspruch 10 zur Verwendung als Medikament zur Behandlung von Krebserkrankungen, diabetischer Erblindung, Makuladegeneration, rheumatoider Polyarthritis und Psoriasis.

12. Verbindung nach Anspruch 11 zur Verwendung als Medikament zur Behandlung von Krebserkrankungen.

13. Pharmazeutische Zusammensetzung, **dadurch gekennzeichnet, dass** sie als Wirkstoff wenigstens eine Mannopyranosidderivat-Verbindung gemäß einem der Ansprüche 1 bis 6 und wenigstens einen pharmazeutisch verträglichen Hilfsstoff umfasst.

14. Pharmazeutische Zusammensetzung nach Anspruch 13, **dadurch gekennzeichnet, dass** sie ein oder mehrere Antitumor-Wirkstoffe umfasst, ausgewählt aus Doxorubicin, Etoposid, Fluoruracil, Melphalan, Cyclophosphamid, Bleomycin, Vinblastin, Mitomycin, Lomustin (CCNU), Taxoter, Taxol, Metotrexat und Cisplatin.

15. Implantierbare medizinische Vorrichtung, **dadurch gekennzeichnet, dass** sie mit wenigstens einer Mannopyranosidderivat-Verbindung gemäß einem der Ansprüche 1 bis 6 oberflächenbehandelt ist, wobei die Vorrichtung ausgewählt sein kann aus Prothesen und insbesondere Gefäß-, Harnröhren- und Gallenprothesen.

## Claims

1. Compound derived from mannopyranoside or its pharmaceutically acceptable salts, **characterised in that** it satisfies one of the following formulas:
➢ Formula (I): 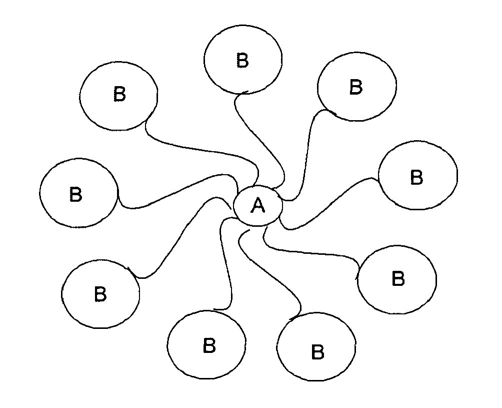 in which:
○ A is a silica nanoparticle or a metal nanoparticle selected from the elements of columns (IB), (IIB), (IIIB), (IVB), (VB), (VIB), (VIIB) or (VIIIB) of Mendeleev's periodic table, and
○ B is a group carrying a mannopyranoside function conforming to the following structure: 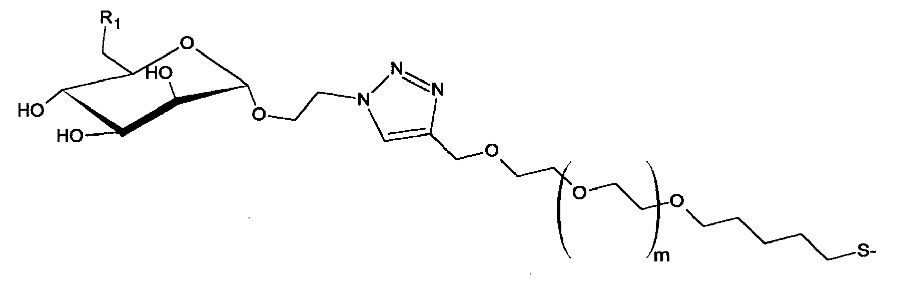
in which:
- the radical R₁ represents a radical selected from -O-PO₃H₂, -N₃, -CH₂-PO₃H₂,
- CH₂-COOH, -SO₃H, -OPHO₂H -CH₂-B(OH)₂, -X-PHO₂H -X'-PO₂H-X-PO₃H₂, and preferably -CH₂-COOH and -N₃, and
- m is an integer between 0 and 10, and preferably m = 3, 4, 5 or 6,
the groups B being bonded to the nanoparticle A via the sulphur atom, and the number of groups B bonded to the nanoparticle A being between 100 and 1000, and preferably between 400 and 600,
➢ Formula (II): 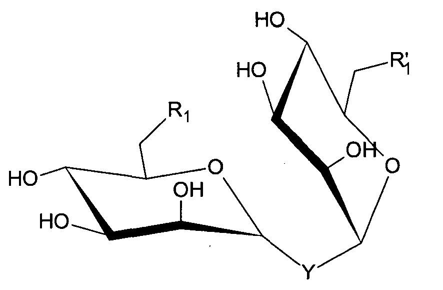 in which:
- the radicals R₁ and R'₁, identical or different, represent radicals selected from -O-PO₃H₂, -N₃, -CH₂-PO₃H₂, -CH₂-COOH, -SO₃H, -OPHO₂H -CH₂-B(OH)₂, -X-PHO₂H -X'-PO₂H-X-PO₃H₂, and preferably -CH₂-COOH and -N₃, and
- Y represents one of the following groups: 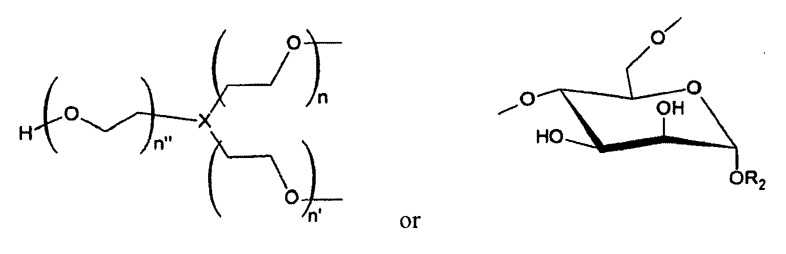 where:
○ n, n' and n" are integers between 1 and 12, and preferably between 1 and 6,
○ n" being equal to 0 if X represents an oxygen atom, the group X being selected from: N, O, S, a C₁-C₄ alkyl chain, the group X preferably being an oxygen atom, and
○ the radical R₂ represents a linear or branched C₁-C₁₂, and preferably C₁-C₄ alkyl chain; a linear or branched C₁-C₁₂, and preferably C₁-C₄, alkyl chain carrying at least one -OH, -NH₂, -SH, -COOH, -N₃, -NO₂ group; a saturated or unsaturated C₃-C₆ hydrocarbon ring; a saturated or unsaturated C₃-C₁₀ hydrocarbon ring carrying at least one -OH, -NH₂, -SH, -COOH, -N₃, -NO₂, C₁-C₄ alkyl chain; a saturated or unsaturated heterocycle comprising at least one heteroatom selected from oxygen, nitrogen or sulphur atoms; a -(CH₂-CH₂-O)_{y}-H radical, in which y is between 1 and 12, and preferably between 1 and 6,
➢ Formula (III): 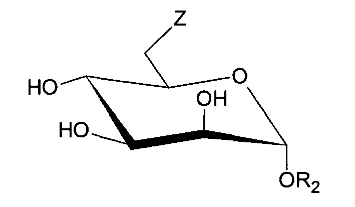 in which:
- Z represents one of the following groups 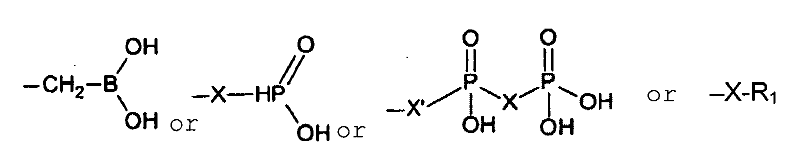
- the radical R₁ is selected from -O-PO₃H₂, -N₃, -CH₂-PO₃H₂, -CH₂-COOH, -OPHO₂H -CH₂-B(OH)₂, -X-PHO₂H -X'-PO₂H-X-PO₃H₂, and preferably -CH₂-COOH and -N₃,
- the radical R₂ represents a linear or branched C₁-C₁₂, and preferably C₁-C₄, alkyl chain; a linear or branched C₁-C₁₂, and preferably C₁-C₄, alkyl chain carrying at least one -OH, -NH₂, -SH, -COOH, -N₃, -NO₂ group; a saturated or unsaturated C₃-C₆ hydrocarbon ring; a saturated or unsaturated C₃-C₁₀ hydrocarbon ring carrying at least one -OH, -NH₂, -SH, -COOH, -N₃, -NO₂, alkyl C₁-C₄ group; a saturated or unsaturated heterocycle comprising at least one heteroatom selected from oxygen, nitrogen or sulphur atoms; a -(CH₂-CH₂-O)_{y}-H radical, in which y is between 1 and 12, and preferably between 1 and 6, and
- the groups X and X', identical or different, are selected from: N, O, S, a C₁-C₄ alkyl chain, the groups X and X' being preferably oxygen atoms.

2. Compound as claimed in claim 1, **characterised in that** R₂ represents a C₁-C₄ alkyl chain selected from methyl, ethyl, *n*-propyl, isopropyl, *n*-butyl, *tert*-butyl, isobutyl and *n*-hexyl radicals, and preferably the methyl radical.

3. Compound as claimed in claim 1, **characterised in that** R₂ represents a saturated hydrocarbon ring selected from cyclopropane, cyclobutane, cyclopentane and cyclohexane.

4. Compound as claimed in claim 1, **characterised in that** R₂ represents an unsaturated hydrocarbon ring or a saturated heterocycle selected from phenyl, oxadiazole, triazole, oxazole, isoxazole, imidazole, thiadiazole, pyrrole, tetrazole, furane, thiophene, pyrazole, pyrazoline, pyrazolidine, thiazole, isothiazole, pyridine, pyrimidine, piperidine, pyran, pyrazine, pyridazine, indole, indazole, benzooxazole, naphthalene, quinoline, quinoxaline, quinazoline, anthracene, acridine rings, and preferably phenyl rings.

5. Compound as claimed in one of claims 1 to 4, **characterised in that** the nanoparticles A of the compound based on formula (I) are selected from gold, iron and cobalt nanoparticles.

6. Compound as claimed in one of claims 1 to 5, **characterised in that** the nanoparticles A have a diameter of between 2 and 10 nm, and preferably between 4 and 8 nm.

7. Method of preparing a compound derived from mannopyranoside as claimed in one of claims 1 to 6, **characterised in that** it comprises at least the following steps:
(i) a halogenation step between a compound based on formula (I'), (II') or (III') carrying at least one primary alcohol function, by a reaction with a dihalogen/phosphine or N-halosuccinimide/phosphine mixture, said compounds (I'), (II') or (III') satisfying the following formulas:
➢ Formula (I'): 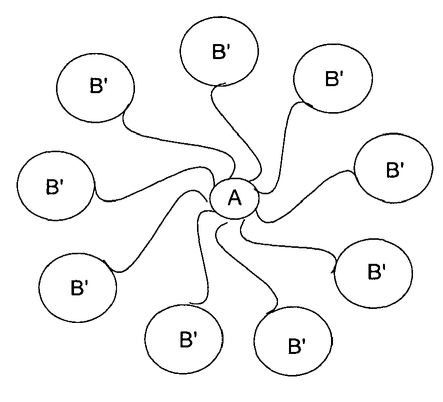 in which A is as defined in claims 1 to 6, and B' is a group conforming to the following structure: 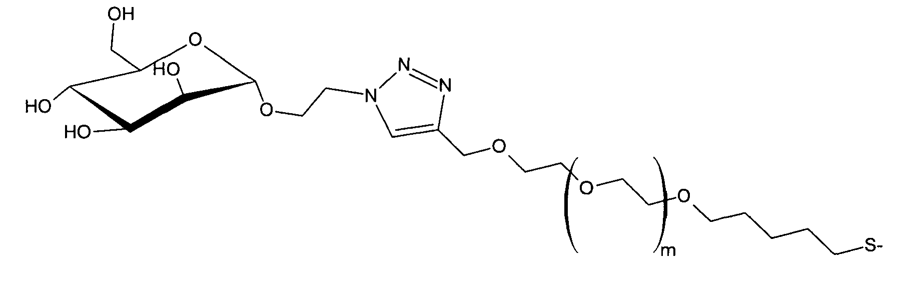 in which m is as defined in claims 1 to 6,
the groups B' being bonded to the nanoparticle A via the sulphur atom, and the number of groups B' bonded to the nanoparticle A being between 100 and 1000, and preferably between 400 and 600,
➢ Formula (II'): 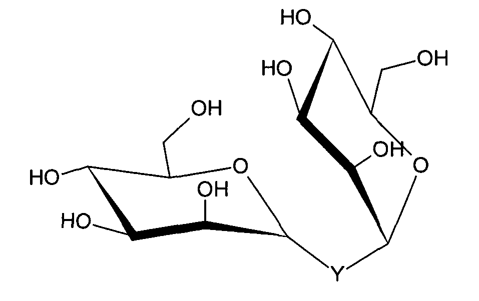 in which Y, n, n' and n" are as defined in claims 1 to 6,
➢ Formula (III'): 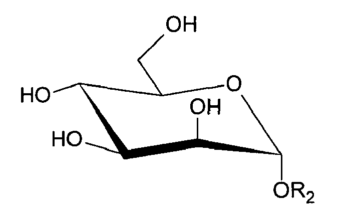 the radical R₂ and the groups X and X' being as defined in claims 1 to 6,
(ii) a step of nucleophilic substitution of the halogenated compounds obtained in step (i) by a reaction with a nucleophilic reagent carrying a radical R₁ and/or R'₁ to obtain the compounds of formula (I), (II) or (III) as defined in claims 1 to 6.

8. Method as claimed in claim 7, **characterised in that** step (i) is conducted with a diiodine/triphenylphosphine mixture in the presence of imidazole.

9. Compound derived from mannopyranoside as claimed in one of claims 1 to 6, for use as a medicament.

10. Compound as claimed in claim 9, for use as a medicament intended for the prevention and/or treatment of diseases dependent on an inhibition of angiogenesis.

11. Compound as claimed in claim 10, for use as a medicament intended for the treatment of cancer-related diseases, diabetic blindness, macular degeneration, rheumatoid arthritis and psoriasis.

12. Compound as claimed in claim 11, for use as a medicament intended for the treatment of cancer-related diseases.

13. Pharmaceutical composition **characterised in that** it contains as the main active ingredient at least one compound derived from mannopyranoside as defined in one of claims 1 to 6, and at least one pharmaceutically acceptable excipient.

14. Pharmaceutical composition as claimed in claim 13, **characterised in that** it contains one or more active ingredients to combat tumours selected from doxorubicin, etoposide, fluorouracil, melphalan, cyclophosphamide, beomycin, vinblastine, mytomycin, lomustine (CCNU), taxotere, taxol, methotrexate and cisplatinum.

15. Implantable medical device, **characterised in that** it is surface-treated with at least one compound derived from mannopyranoside as defined in one of claims 1 to 6, said device being selected from prostheses, and more particularly vascular, urethral and biliary stents.
